# EUROPEAN PATENT APPLICATION

(11) **EP 3 984 548 A1**
(43) Date of publication of application: **20.04.2022**
(21) Application number: 20306235.1
(22) Date of filing: 16.10.2020
(51) Int. Cl.: A61K 39/00, A61K 39/04

(54) **GENERATION OF LENTIVIRAL VECTORS ENABLING ROUTING ANTIGENS TO MHC-II PATHWAY AND INDUCING CD4+ AND CD8+ T-CELL RESPONSES IMMUNE RESPONSE IN A HOST**

(71) Applicant: INSTITUT PASTEUR, 75724 Paris Cedex 15 (FR); Theravectys, 75724 Paris Cedex 15 (FR)
(72) Inventor: CHARNEAU, Pierre, 75724 PARIS CEDEX 15 (FR); MAJLESSI, Laleh, 75724 PARIS CEDEX 15 (FR); LOPEZ, Jodie, 75724 PARIS CEDEX 15 (FR); ANNA, François, 75724 PARIS CEDEX 15 (FR); BLANC, Catherine, 75724 PARIS CEDEX 15 (FR); MONCOQ, Fanny, 75724 PARIS CEDEX 15 (FR)
(74) Representative: Ernest Gutmann - Yves Plasseraud S.A.S.

(57) **Abstract**

The invention relates to a recombinant lentiviral vector genome comprising a polynucleotide encoding a fusion polypeptide, wherein said fusion protein comprises arranged from N-terminal to C-terminal ends: (i) a first polypeptide comprising a multimerization scaffold which comprises at least one collectin or a fragment thereof suitable to enable self-assembly of multimers of the first polypeptide, fused with at least one antigenic polypeptide ; (ii) a second polypeptide comprising a CD40L ectodomain or a receptor binding fragment thereof, in particular the CD40L ectodomain of the human CD40L. The invention also relates to a lentiviral vector and pharmaceutical compositions comprising it.

SEQUENCE LISTING

<110>  INSTITUT PASTEUR
       THERAVECTYS

<120>  New generation of lentiviral vectors enabling routing antigens to
        MHC-II pathway and inducing CD4+ and CD8+ T-cell responses
       immune response in a host

<130>  B14346-AD/PPT

<160>  24

<170>  PatentIn version 3.5

<210>  1
<211>  414
<212>  PRT
<213>  Artificial Sequence

<220>
<223>  M40-H

<400>  1

Met Ser Ile Phe Thr Ser Phe Leu Leu Leu Cys Val Val Thr Val Val
1               5                   10                  15

Tyr Ala Glu Thr Leu Thr Glu Gly Val Gln Asn Ser Cys Pro Val Val
            20                  25                  30

Thr Cys Ser Ser Pro Gly Leu Asn Gly Phe Pro Gly Lys Asp Gly Arg
        35                  40                  45

Asp Gly Ala Lys Gly Glu Lys Gly Glu Pro Gly Gln Gly Leu Arg Gly
    50                  55                  60

Leu Gln Gly Pro Pro Gly Ala Val Gly Pro Thr Gly Pro Pro Gly Asn
65                  70                  75                  80

Pro Gly Leu Lys Gly Ala Val Gly Pro Lys Gly Asp Arg Gly Asp Arg
                85                  90                  95

Gly Gly Gly Ser Gln Ile Met Tyr Asn Tyr Pro Ala Met Leu Gly His
            100                 105                 110

Ala Gly Asp Met Ala Gly Tyr Ala Gly Thr Leu Gln Ser Leu Gly Ala
        115                 120                 125

Glu Ile Ala Val Glu Gln Ala Ala Leu Gln Ser Ala Trp Gln Gly Asp
    130                 135                 140

Thr Gly Ile Thr Tyr Gln Ala Trp Gln Ala Gln Trp Asn Gln Ala Met
145                 150                 155                 160

Glu Asp Leu Val Arg Ala Tyr His Ala Met Ser Ser Thr His Glu Ala
                165                 170                 175

Asn Thr Met Ala Met Met Ala Arg Asp Thr Ala Glu Ala Ala Lys Trp
            180                 185                 190

Gly Gly Gly Ser Gly Leu Arg Gly Leu Gln Gly Pro Pro Gly Ala Leu
        195                 200                 205

Gly Pro Pro Gly Ser Val Gly Ser Pro Gly Ser Pro Gly Pro Lys Gly
    210                 215                 220

Gln Lys Gly Asp His Gly Asp Asn Arg Ala Ile Glu Glu Lys Leu Ala
225                 230                 235                 240

Asn Met Glu Ala Glu Ile Arg Ile Leu Lys Ser Lys Leu Gln Leu Thr
                245                 250                 255

Asn Lys Leu His Ala Phe Ser Met Gly Gly Gly Ser Gly Asp Glu Asp
            260                 265                 270

Pro Gln Ile Ala Ala His Val Val Ser Glu Ala Asn Ser Asn Ala Ala
        275                 280                 285

Ser Val Leu Gln Trp Ala Lys Lys Gly Tyr Tyr Thr Met Lys Ser Asn
    290                 295                 300

Leu Val Met Leu Glu Asn Gly Lys Gln Leu Thr Val Lys Arg Glu Gly
305                 310                 315                 320

Leu Tyr Tyr Val Tyr Thr Gln Val Thr Phe Cys Ser Asn Arg Glu Pro
                325                 330                 335

Ser Ser Gln Arg Pro Phe Ile Val Gly Leu Trp Leu Lys Pro Ser Ser
            340                 345                 350

Gly Ser Glu Arg Ile Leu Leu Lys Ala Ala Asn Thr His Ser Ser Ser
        355                 360                 365

Gln Leu Cys Glu Gln Gln Ser Val His Leu Gly Gly Val Phe Glu Leu
    370                 375                 380

Gln Ala Gly Ala Ser Val Phe Val Asn Val Thr Glu Ala Ser Gln Val
385                 390                 395                 400

Ile His Arg Val Gly Phe Ser Ser Phe Gly Leu Leu Lys Leu

405                    410

<210>  2
<211>  1245
<212>  DNA
<213>  Artificial Sequence

<220>
<223>  M40-H

<400>  2
atgtcaattt tcacctcatt cctgctgctg tgcgtcgtca ctgtcgtgta tgctgagacc      60

ctgaccgaag gcgtccagaa ctcctgtcca gtggtgacct gcagctcccc aggcctgaat     120

ggcttccctg gcaaggacgg aagggatgga gcaaagggag agaagggaga gcctggacag     180

ggcctgaggg gcctgcaggg accacctggc gccgtgggcc caacaggccc acccggcaac     240

cccggcctga agggagcagt gggacctaag ggcgaccggg gcgatagagg aggaggctcc     300

cagatcatgt acaattatcc agccatgctg ggacacgcag gcgacatggc aggatacgca     360

ggcaccctgc agtccctggg agcagagatc gcagtggagc aggccgccct gcagtctgcc     420

tggcagggcg acaccggcat cacataccag gcatggcagg cacagtggaa ccaggccatg     480

gaggatctgg tgcgggccta tcacgccatg tctagcaccc acgaggccaa tacaatggcc     540

atgatggccc gggatacagc cgaggccgcc aagtggggcg gcggcagcgg cctgagaggc     600

ctgcagggac ctccaggcgc cctgggacca cctggctctg tgggcagccc aggctcccca     660

ggccccaagg gccagaaggg cgaccacggc gataaccggg ccatcgagga gaagctggcc     720

aatatggagg ccgagatcag aatcctgaag tccaagctgc agctgaccaa caagctgcac     780

gccttttcta tgggaggagg cagcggcgac gaggaccccc agatcgcagc acacgtggtg     840

tctgaggcca acagcaatgc cgcctccgtg ctgcagtggg ccaagaaggg ctactatacc     900

atgaagtcta acctggtcat gctggagaat ggcaagcagc tgacagtgaa gagggagggc     960

ctgtactacg tgtacaccca ggtgacattc tgctctaaca gggagccttc ctctcagcgg    1020

cccttcatcg tgggcctgtg gctgaagcct agctccggca gcgagcgcat cctgctgaag    1080

gcagccaata cacactctag ctcccagctg tgcgagcagc agagcgtgca cctgggaggc    1140

gtgttcgagc tgcaggcagg agcctccgtg tttgtgaacg tgacagaggc atcacaggtc    1200

atccatcggg tggggttctc atcattcggg ctgctgaaac tgtga                    1245

<210>  3
<211>  556
<212>  PRT
<213>  Artificial Sequence

<220>
<223>  M40-HA

<400> 3

Met Ser Ile Phe Thr Ser Phe Leu Leu Leu Cys Val Val Thr Val Val
1               5                   10                  15

Tyr Ala Glu Thr Leu Thr Glu Gly Val Gln Asn Ser Cys Pro Val Val
            20                  25                  30

Thr Cys Ser Ser Pro Gly Leu Asn Gly Phe Pro Gly Lys Asp Gly Arg
        35                  40                  45

Asp Gly Ala Lys Gly Glu Lys Gly Glu Pro Gly Gln Gly Leu Arg Gly
    50                  55                  60

Leu Gln Gly Pro Pro Gly Ala Val Gly Pro Thr Gly Pro Pro Gly Asn
65                  70                  75                  80

Pro Gly Leu Lys Gly Ala Val Gly Pro Lys Gly Asp Arg Gly Asp Arg
                85                  90                  95

Gly Gly Gly Ser Gln Ile Met Tyr Asn Tyr Pro Ala Met Leu Gly His
            100                 105                 110

Ala Gly Asp Met Ala Gly Tyr Ala Gly Thr Leu Gln Ser Leu Gly Ala
        115                 120                 125

Glu Ile Ala Val Glu Gln Ala Ala Leu Gln Ser Ala Trp Gln Gly Asp
    130                 135                 140

Thr Gly Ile Thr Tyr Gln Ala Trp Gln Ala Gln Trp Asn Gln Ala Met
145                 150                 155                 160

Glu Asp Leu Val Arg Ala Tyr His Ala Met Ser Ser Thr His Glu Ala
                165                 170                 175

Asn Thr Met Ala Met Met Ala Arg Asp Thr Ala Glu Ala Ala Lys Trp
            180                 185                 190

Gly Gly Gly Ser Gly Phe Pro Gly Pro Pro Gly Pro Lys Gly Glu Pro
        195                 200                 205

Gly Ser Pro Ala Gly Arg Gly Glu Arg Gly Phe Gln Gly Ser Pro Gly
    210                 215                 220

Lys Met Gly Pro Ala Gly Ser Lys Gly Glu Pro Gly Gly Gly Ser Gly
225                 230                 235                 240

Thr Glu Gln Gln Trp Asn Phe Ala Gly Ile Glu Ala Ala Ala Ser Ala

245                 250                 255

Ile Gln Gly Asn Val Thr Ser Ile His Ser Leu Leu Asp Glu Gly Lys
            260                 265                 270

Gln Ser Leu Thr Lys Leu Ala Ala Ala Trp Gly Gly Ser Gly Ser Glu
        275                 280                 285

Ala Tyr Gln Gly Val Gln Gln Lys Trp Asp Ala Thr Ala Thr Glu Leu
    290                 295                 300

Asn Asn Ala Leu Gln Asn Leu Ala Arg Thr Ile Ser Glu Ala Gly Gln
305                 310                 315                 320

Ala Met Ala Ser Thr Glu Gly Asn Val Thr Gly Met Phe Ala Gly Gly
                325                 330                 335

Gly Ser Gly Leu Arg Gly Leu Gln Gly Pro Pro Gly Ala Leu Gly Pro
            340                 345                 350

Pro Gly Ser Val Gly Ser Pro Gly Ser Pro Gly Pro Lys Gly Gln Lys
        355                 360                 365

Gly Asp His Gly Asp Asn Arg Ala Ile Glu Glu Lys Leu Ala Asn Met
    370                 375                 380

Glu Ala Glu Ile Arg Ile Leu Lys Ser Lys Leu Gln Leu Thr Asn Lys
385                 390                 395                 400

Leu His Ala Phe Ser Met Gly Gly Gly Ser Gly Asp Glu Asp Pro Gln
                405                 410                 415

Ile Ala Ala His Val Val Ser Glu Ala Asn Ser Asn Ala Ala Ser Val
            420                 425                 430

Leu Gln Trp Ala Lys Lys Gly Tyr Tyr Thr Met Lys Ser Asn Leu Val
        435                 440                 445

Met Leu Glu Asn Gly Lys Gln Leu Thr Val Lys Arg Glu Gly Leu Tyr
    450                 455                 460

Tyr Val Tyr Thr Gln Val Thr Phe Cys Ser Asn Arg Glu Pro Ser Ser
465                 470                 475                 480

Gln Arg Pro Phe Ile Val Gly Leu Trp Leu Lys Pro Ser Ser Gly Ser
                485                 490                 495

Glu Arg Ile Leu Leu Lys Ala Ala Asn Thr His Ser Ser Ser Gln Leu
            500                 505                 510

Cys Glu Gln Gln Ser Val His Leu Gly Gly Val Phe Glu Leu Gln Ala
        515                 520                 525

Gly Ala Ser Val Phe Val Asn Val Thr Glu Ala Ser Gln Val Ile His
    530                 535                 540

Arg Val Gly Phe Ser Ser Phe Gly Leu Leu Lys Leu
545                 550                 555

<210>  4
<211>  1671
<212>  DNA
<213>  Artificial Sequence

<220>
<223>  M40-HA

<400>  4
atgtctatct tcaccagctt tctgctgctg tgcgtggtga cagtggtgta cgccgagacc      60

ctgacagagg gcgtgcagaa ctcctgccca gtggtgacct gtagctcccc aggcctgaat     120

ggcttccccg gcaaggacgg ccgggatggc gccaagggcg agaagggcga gcccggccag     180

ggcctgagag gactgcaggg accacctgga gcagtgggac ctacaggccc acccggcaac     240

ccaggcctga agggagcagt gggaccaaag ggcgacaggg gcgatagagg cggcggcagc     300

cagatcatgt acaattatcc tgcaatgctg ggacacgcag gcgacatggc aggctatgca     360

ggcaccctgc agtctctggg agcagagatc gcagtggagc aggccgccct gcagagcgcc     420

tggcagggcg acaccggaat cacataccag gcatggcagg cacagtggaa ccaggccatg     480

gaggatctgg tgagggccta tcacgccatg tctagcaccc acgaggccaa tacaatggcc     540

atgatggccc gcgataccgc cgaggccgcc aagtggggcg gcggctccgg cttcccaggc     600

cctccaggcc ccaagggaga gccaggcagc cctgcaggaa ggggcgagcg cggctttcag     660

ggctcccctg gcaagatggg accagcaggc agcaagggag agccaggagg aggctccggc     720

acagagcagc agtggaactt tgcaggaatc gaggcagcag catccgccat ccagggcaat     780

gtgaccagca tccactccct gctggacgag ggcaagcagt ctctgacaaa gctggcagca     840

gcatggggag gctccggctc tgaggcatac cagggagtgc agcagaagtg ggatgccacc     900

gccacagagc tgaacaatgc cctgcagaac ctggcaagga ccatctctga ggcaggacag     960

gcaatggcaa gcaccgaggg caatgtgaca ggcatgttcg ccggcggcgg ctccggcctg    1020

agaggcctgc agggaccacc tggcgccctg ggaccaccag gctctgtggg cagccctggc    1080

tcccctggac caaagggaca gaagggcgac cacggcgata acagggccat cgaggagaag    1140

ctggccaata tggaggccga gatccgcatc ctgaagagca agctgcagct gaccaataag 1200

ctgcacgcct tttccatggg aggaggctct ggcgacgagg acccccagat cgcagcacac 1260

gtggtgtccg aggcaaactc taatgccgcc agcgtgctgc agtgggccaa gaagggctac 1320

tataccatga agtctaacct ggtcatgctg gagaatggca agcagctgac agtgaagagg 1380

gagggcctgt actacgtgta cacccaggtg acattctgca gcaaccggga gccctcctct 1440

cagagacctt ttatcgtggg cctgtggctg aagcctagct ccggcagcga gcgcatcctg 1500

ctgaaggcag ccaataccca ctctagctcc cagctgtgcg agcagcagtc cgtgcacctg 1560

ggaggcgtgt tcgagctgca ggcaggagcc tccgtgtttg tgaacgtgac agaggcctct 1620

caggtcatcc accgggtggg cttctctagc tttggcctgc tgaagctgtg a 1671

<210> 5
<211> 721
<212> PRT
<213> Artificial Sequence

<220>
<223> M40-HAP

<400> 5

Met Ser Ile Phe Thr Ser Phe Leu Leu Leu Cys Val Val Thr Val Val
1               5                   10                  15

Tyr Ala Glu Thr Leu Thr Glu Gly Val Gln Asn Ser Cys Pro Val Val
            20                  25                  30

Thr Cys Ser Ser Pro Gly Leu Asn Gly Phe Pro Gly Lys Asp Gly Arg
        35                  40                  45

Asp Gly Ala Lys Gly Glu Lys Gly Glu Pro Gly Gln Gly Leu Arg Gly
    50                  55                  60

Leu Gln Gly Pro Pro Gly Ala Val Gly Pro Thr Gly Pro Pro Gly Asn
65                  70                  75                  80

Pro Gly Leu Lys Gly Ala Val Gly Pro Lys Gly Asp Arg Gly Asp Arg
                85                  90                  95

Gly Gly Gly Ser Gln Ile Met Tyr Asn Tyr Pro Ala Met Leu Gly His
            100                 105                 110

Ala Gly Asp Met Ala Gly Tyr Ala Gly Thr Leu Gln Ser Leu Gly Ala
        115                 120                 125

Glu Ile Ala Val Glu Gln Ala Ala Leu Gln Ser Ala Trp Gln Gly Asp
    130                 135                 140

Thr Gly Ile Thr Tyr Gln Ala Trp Gln Ala Gln Trp Asn Gln Ala Met
145                 150                 155                 160

Glu Asp Leu Val Arg Ala Tyr His Ala Met Ser Ser Thr His Glu Ala
                165                 170                 175

Asn Thr Met Ala Met Met Ala Arg Asp Thr Ala Glu Ala Ala Lys Trp
            180                 185                 190

Gly Gly Gly Ser Gly Phe Pro Gly Pro Pro Gly Pro Lys Gly Glu Pro
        195                 200                 205

Gly Ser Pro Ala Gly Arg Gly Glu Arg Gly Phe Gln Gly Ser Pro Gly
    210                 215                 220

Lys Met Gly Pro Ala Gly Ser Lys Gly Glu Pro Gly Gly Gly Ser Gly
225                 230                 235                 240

Thr Glu Gln Gln Trp Asn Phe Ala Gly Ile Glu Ala Ala Ala Ser Ala
                245                 250                 255

Ile Gln Gly Asn Val Thr Ser Ile His Ser Leu Leu Asp Glu Gly Lys
            260                 265                 270

Gln Ser Leu Thr Lys Leu Ala Ala Ala Trp Gly Gly Ser Gly Ser Glu
        275                 280                 285

Ala Tyr Gln Gly Val Gln Gln Lys Trp Asp Ala Thr Ala Thr Glu Leu
    290                 295                 300

Asn Asn Ala Leu Gln Asn Leu Ala Arg Thr Ile Ser Glu Ala Gly Gln
305                 310                 315                 320

Ala Met Ala Ser Thr Glu Gly Asn Val Thr Gly Met Phe Ala Gly Gly
                325                 330                 335

Gly Ser Gly Leu Pro Gly Arg Asp Gly Arg Asp Gly Arg Glu Gly Pro
            340                 345                 350

Arg Gly Glu Lys Gly Asp Pro Gly Leu Pro Gly Pro Met Gly Leu Ser
        355                 360                 365

Gly Leu Gln Gly Pro Thr Gly Pro Val Gly Pro Lys Gly Glu Asn Gly
    370                 375                 380

Ser Ala Gly Glu Pro Gly Pro Lys Gly Glu Arg Gly Leu Ser Gly Gly

385                 390                 395                 400

Gly Gly Ser Phe Val Thr Thr Gln Pro Glu Ala Leu Ala Ala Ala Ala
                405                 410                 415

Ala Asn Leu Gln Gly Ile Gly Thr Thr Met Asn Ala Gln Asn Ala Ala
            420                 425                 430

Ala Ala Ala Pro Thr Thr Gly Val Val Pro Ala Ala Ala Asp Glu Val
        435                 440                 445

Ser Ala Leu Thr Ala Ala Gln Phe Ala Ala His Ala Gln Met Tyr Gln
    450                 455                 460

Thr Val Ser Ala Gln Ala Ala Ala Ile His Glu Met Phe Val Asn Thr
465                 470                 475                 480

Leu Val Ala Ser Ser Gly Ser Tyr Ala Ala Thr Glu Ala Ala Asn Ala
                485                 490                 495

Ala Ala Ala Gly Gly Gly Ser Gly Leu Arg Gly Leu Gln Gly Pro Pro
            500                 505                 510

Gly Ala Leu Gly Pro Pro Gly Ser Val Gly Ser Pro Gly Ser Pro Gly
        515                 520                 525

Pro Lys Gly Gln Lys Gly Asp His Gly Asp Asn Arg Ala Ile Glu Glu
    530                 535                 540

Lys Leu Ala Asn Met Glu Ala Glu Ile Arg Ile Leu Lys Ser Lys Leu
545                 550                 555                 560

Gln Leu Thr Asn Lys Leu His Ala Phe Ser Met Gly Gly Gly Ser Gly
                565                 570                 575

Asp Glu Asp Pro Gln Ile Ala Ala His Val Val Ser Glu Ala Asn Ser
            580                 585                 590

Asn Ala Ala Ser Val Leu Gln Trp Ala Lys Lys Gly Tyr Tyr Thr Met
        595                 600                 605

Lys Ser Asn Leu Val Met Leu Glu Asn Gly Lys Gln Leu Thr Val Lys
    610                 615                 620

Arg Glu Gly Leu Tyr Tyr Val Tyr Thr Gln Val Thr Phe Cys Ser Asn
625                 630                 635                 640

Arg Glu Pro Ser Ser Gln Arg Pro Phe Ile Val Gly Leu Trp Leu Lys
                645                 650                 655

Pro Ser Ser Gly Ser Glu Arg Ile Leu Leu Lys Ala Ala Asn Thr His
            660                 665                 670

Ser Ser Ser Gln Leu Cys Glu Gln Gln Ser Val His Leu Gly Gly Val
        675                 680                 685

Phe Glu Leu Gln Ala Gly Ala Ser Val Phe Val Asn Val Thr Glu Ala
    690                 695                 700

Ser Gln Val Ile His Arg Val Gly Phe Ser Ser Phe Gly Leu Leu Lys
705                 710                 715                 720

Leu

<210>  6
<211>  2166
<212>  DNA
<213>  Artificial Sequence

<220>
<223>  M40-HAP

<400>  6
atgtccatct tcacctcttt tctgctgctg tgcgtggtga cagtggtgta cgccgagacc      60

ctgacagagg gcgtgcagaa ctcctgccct gtggtgacct gtagctcccc aggcctgaat     120

ggcttccctg gcaaggacgg aagggatgga gcaaagggag agaagggcga gccaggacag     180

ggactgaggg gactgcaggg accacctggc gccgtgggcc ctacaggccc acccggcaac     240

ccaggcctga agggagcagt gggaccaaag ggcgacaggg gcgatagagg aggaggcagc     300

cagatcatgt acaattatcc agcaatgctg ggacacgcag gcgacatggc cggctatgca     360

ggcaccctgc agagcctggg agcagagatc gcagtggagc aggccgccct gcagtccgcc     420

tggcagggcg acaccggaat cacataccag gcatggcagg cacagtggaa ccaggccatg     480

gaggatctgg tgagagccta tcacgccatg tctagcaccc acgaggccaa tacaatggcc     540

atgatggcca gggataccgc cgaggccgcc aagtggggag gaggctccgg attcccagga     600

cctccaggcc ctaagggcga gcctggctcc ccagccggaa ggggagagag gggatttcag     660

ggctctccag gcaagatggg accagcaggc tccaagggag agcctggagg aggctctggc     720

acagagcagc agtggaactt tgcaggaatc gaggcagcag catctgccat ccagggcaat     780

gtgacctcta tccacagcct gctggacgag ggcaagcaga gcctgacaaa gctggcagca     840

gcatggggag gcagcggctc cgaggcctac cagggcgtgc agcagaagtg ggatgccacc     900

gccacagagc tgaacaatgc cctgcagaac ctggcaagaa ccatcagcga ggcaggacag 960

gcaatggcat ccaccgaggg caatgtgaca ggcatgttcg caggaggagg cagcggactg 1020

ccaggacggg acggcagaga tggaagggag ggacccaggg gcgagaaggg cgaccctgga 1080

ctgcctggac caatgggact gtccggactg cagggaccaa ccggccctgt gggcccaaag 1140

ggagagaatg gctctgccgg agagccaggc cctaagggag agaggggact gagcggagga 1200

ggaggctcct ttgtgaccac acagccagag gccctggcag cagcagcagc aaacctgcag 1260

ggcatcggca ccacaatgaa cgcccagaat gcagcagcag cagcaccaac cacaggagtg 1320

gtgcccgccg ccgccgatga ggtgagcgcc ctgaccgcag cacagttcgc agcacacgcc 1380

cagatgtacc agacagtgtc cgcccaggca gcagcaatcc acgagatgtt tgtgaacacc 1440

ctggtggcct cctctggctc ttatgccgcc acagaggccg ccaatgcagc agcagccggc 1500

ggaggcagcg gactgcgggg cctgcagggc ccccctggcg ccctgggccc acccggctcc 1560

gtgggctctc ccggcagccc aggccccaag ggccagaagg gcgaccacgg cgataacaga 1620

gccatcgagg agaagctggc caatatggag gccgagatca gaatcctgaa gagcaagctg 1680

cagctgacca acaagctgca cgccttctct atgggaggag gcagcggcga cgaggacccc 1740

cagatcgcag cacacgtggt gagcgaggca aactccaatg ccgcctctgt gctgcagtgg 1800

gccaagaagg gctactatac catgaagtcc aacctggtca tgctggagaa tggcaagcag 1860

ctgacagtga agcgcgaggg cctgtactac gtgtacaccc aggtgacatt ctgcagcaac 1920

agggagccta gctcccagag gcccttcatc gtgggactgt ggctgaagcc ctctagcggc 1980

tccgagagaa tcctgctgaa ggccgccaat acccactcct ctagccagct gtgcgagcag 2040

cagtctgtgc acctgggagg cgtgttcgag ctgcaggcag gagcaagcgt gttcgtgaac 2100

gtgacagagg caagccaggt catccacagg gtgggcttct cctcttttgg cctgctgaag 2160

ctgtga 2166

<210> 7
<211> 885
<212> PRT
<213> Artificial Sequence

<220>
<223> M40-HAPE

<400> 7

Met Ser Ile Phe Thr Ser Phe Leu Leu Leu Cys Val Val Thr Val Val
1               5                   10                  15

Tyr Ala Glu Thr Leu Thr Glu Gly Val Gln Asn Ser Cys Pro Val Val
            20                  25                  30

Thr Cys Ser Ser Pro Gly Leu Asn Gly Phe Pro Gly Lys Asp Gly Arg
        35                  40                  45

Asp Gly Ala Lys Gly Glu Lys Gly Glu Pro Gly Gln Gly Leu Arg Gly
    50                  55                  60

Leu Gln Gly Pro Pro Gly Ala Val Gly Pro Thr Gly Pro Pro Gly Asn
65                  70                  75                  80

Pro Gly Leu Lys Gly Ala Val Gly Pro Lys Gly Asp Arg Gly Asp Arg
                85                  90                  95

Gly Gly Gly Ser Gln Ile Met Tyr Asn Tyr Pro Ala Met Leu Gly His
            100                 105                 110

Ala Gly Asp Met Ala Gly Tyr Ala Gly Thr Leu Gln Ser Leu Gly Ala
        115                 120                 125

Glu Ile Ala Val Glu Gln Ala Ala Leu Gln Ser Ala Trp Gln Gly Asp
    130                 135                 140

Thr Gly Ile Thr Tyr Gln Ala Trp Gln Ala Gln Trp Asn Gln Ala Met
145                 150                 155                 160

Glu Asp Leu Val Arg Ala Tyr His Ala Met Ser Ser Thr His Glu Ala
                165                 170                 175

Asn Thr Met Ala Met Met Ala Arg Asp Thr Ala Glu Ala Ala Lys Trp
            180                 185                 190

Gly Gly Gly Ser Gly Phe Pro Gly Pro Pro Gly Pro Lys Gly Glu Pro
        195                 200                 205

Gly Ser Pro Ala Gly Arg Gly Glu Arg Gly Phe Gln Gly Ser Pro Gly
    210                 215                 220

Lys Met Gly Pro Ala Gly Ser Lys Gly Glu Pro Gly Gly Gly Ser Gly
225                 230                 235                 240

Thr Glu Gln Gln Trp Asn Phe Ala Gly Ile Glu Ala Ala Ala Ser Ala
                245                 250                 255

Ile Gln Gly Asn Val Thr Ser Ile His Ser Leu Leu Asp Glu Gly Lys
            260                 265                 270

Gln Ser Leu Thr Lys Leu Ala Ala Ala Trp Gly Gly Ser Gly Ser Glu
        275                 280                 285

Ala Tyr Gln Gly Val Gln Gln Lys Trp Asp Ala Thr Ala Thr Glu Leu
    290                 295                 300

Asn Asn Ala Leu Gln Asn Leu Ala Arg Thr Ile Ser Glu Ala Gly Gln
305                 310                 315                 320

Ala Met Ala Ser Thr Glu Gly Asn Val Thr Gly Met Phe Ala Gly Gly
                325                 330                 335

Gly Ser Gly Leu Pro Gly Arg Asp Gly Arg Asp Gly Arg Glu Gly Pro
            340                 345                 350

Arg Gly Glu Lys Gly Asp Pro Gly Leu Pro Gly Pro Met Gly Leu Ser
        355                 360                 365

Gly Leu Gln Gly Pro Thr Gly Pro Val Gly Pro Lys Gly Glu Asn Gly
    370                 375                 380

Ser Ala Gly Glu Pro Gly Pro Lys Gly Glu Arg Gly Leu Ser Gly Gly
385                 390                 395                 400

Gly Gly Ser Phe Val Thr Thr Gln Pro Glu Ala Leu Ala Ala Ala Ala
                405                 410                 415

Ala Asn Leu Gln Gly Ile Gly Thr Thr Met Asn Ala Gln Asn Ala Ala
            420                 425                 430

Ala Ala Ala Pro Thr Thr Gly Val Val Pro Ala Ala Ala Asp Glu Val
        435                 440                 445

Ser Ala Leu Thr Ala Ala Gln Phe Ala Ala His Ala Gln Met Tyr Gln
    450                 455                 460

Thr Val Ser Ala Gln Ala Ala Ala Ile His Glu Met Phe Val Asn Thr
465                 470                 475                 480

Leu Val Ala Ser Ser Gly Ser Tyr Ala Ala Thr Glu Ala Ala Asn Ala
                485                 490                 495

Ala Ala Ala Gly Gly Cys Pro Gly Leu Pro Gly Ala Ala Gly Pro Lys
            500                 505                 510

Gly Glu Ala Gly Ala Lys Gly Asp Arg Gly Glu Ser Gly Leu Pro Gly
        515                 520                 525

Ile Pro Gly Lys Glu Gly Pro Thr Gly Pro Lys Gly Asn Gln Gly Glu
    530                 535                 540

Lys Gly Ile Arg Gly Glu Lys Gly Asp Ser Gly Pro Ser Gly Gly Gly
545                 550                 555                 560

Ser Thr Glu Asn Leu Thr Val Gln Pro Glu Arg Leu Gly Val Leu Ala
                565                 570                 575

Ser His His Asp Asn Ala Ala Val Asp Ala Ser Ser Gly Val Glu Ala
            580                 585                 590

Ala Ala Gly Leu Gly Glu Ser Val Ala Ile Thr His Gly Pro Tyr Cys
        595                 600                 605

Ser Gln Phe Asn Asp Thr Leu Asn Val Tyr Leu Thr Ala His Asn Ala
    610                 615                 620

Leu Gly Ser Ser Leu His Thr Ala Gly Val Asp Leu Ala Lys Ser Leu
625                 630                 635                 640

Arg Ile Ala Ala Lys Ile Tyr Ser Glu Ala Asp Glu Ala Trp Arg Lys
                645                 650                 655

Ala Ile Asp Gly Leu Phe Thr Gly Gly Gly Ser Gly Leu Arg Gly Leu
            660                 665                 670

Gln Gly Pro Pro Gly Ala Leu Gly Pro Pro Gly Ser Val Gly Ser Pro
        675                 680                 685

Gly Ser Pro Gly Pro Lys Gly Gln Lys Gly Asp His Gly Asp Asn Arg
    690                 695                 700

Ala Ile Glu Glu Lys Leu Ala Asn Met Glu Ala Glu Ile Arg Ile Leu
705                 710                 715                 720

Lys Ser Lys Leu Gln Leu Thr Asn Lys Leu His Ala Phe Ser Met Gly
                725                 730                 735

Gly Gly Ser Gly Asp Glu Asp Pro Gln Ile Ala Ala His Val Val Ser
            740                 745                 750

Glu Ala Asn Ser Asn Ala Ala Ser Val Leu Gln Trp Ala Lys Lys Gly
        755                 760                 765

Tyr Tyr Thr Met Lys Ser Asn Leu Val Met Leu Glu Asn Gly Lys Gln
    770                 775                 780

Leu Thr Val Lys Arg Glu Gly Leu Tyr Tyr Val Tyr Thr Gln Val Thr

785                 790                 795                 800

Phe Cys Ser Asn Arg Glu Pro Ser Ser Gln Arg Pro Phe Ile Val Gly
                805                 810                 815

Leu Trp Leu Lys Pro Ser Ser Gly Ser Glu Arg Ile Leu Leu Lys Ala
            820                 825                 830

Ala Asn Thr His Ser Ser Ser Gln Leu Cys Glu Gln Gln Ser Val His
        835                 840                 845

Leu Gly Gly Val Phe Glu Leu Gln Ala Gly Ala Ser Val Phe Val Asn
    850                 855                 860

Val Thr Glu Ala Ser Gln Val Ile His Arg Val Gly Phe Ser Ser Phe
865                 870                 875                 880

Gly Leu Leu Lys Leu
                885

<210>  8
<211>  2658
<212>  DNA
<213>  Artificial Sequence

<220>
<223>  M40-HAPE

<400>  8
atgtccatct tcacctcttt tctgctgctg tgcgtggtga cagtggtgta cgccgagacc      60

ctgacagagg gcgtgcagaa ctcctgccca gtggtgacct gtagctcccc aggcctgaat     120

ggcttccctg gcaaggacgg cagagatggc gccaagggcg agaagggcga gcctggccag     180

ggcctgaggg gactgcaggg accacctgga gcagtgggcc ctacaggccc acccggcaac     240

ccaggcctga agggagcagt gggacctaag ggcgacaggg gcgatagagg aggaggcagc     300

cagatcatgt acaattatcc agcaatgctg ggacacgcag gcgacatggc aggctatgca     360

ggcaccctgc agtccctggg agcagagatc gcagtggagc aggccgccct gcagtctgcc     420

tggcagggcg acaccggcat cacataccag gcatggcagg cacagtggaa ccaggccatg     480

gaggatctgg tgcgcgccta tcacgccatg tctagcaccc acgaggccaa tacaatggcc     540

atgatggccc gggataccgc cgaggccgcc aagtggggag gaggctccgg attcccagga     600

cctccaggac ctaagggaga gcctggctct ccagcaggaa gaggagagag gggatttcag     660

ggcagcccag gcaagatggg accagcaggc tccaagggag agccaggcgg cggctctggc     720

acagagcagc agtggaactt tgcaggaatc gaggcagcag caagcgccat ccagggcaat     780

gtgaccagca tccactccct gctggacgag ggcaagcagt ccctgacaaa gctggcagca     840

gcatggggag gctccggctc tgaggcatac cagggagtgc agcagaagtg ggatgccacc 900

gccacagagc tgaacaatgc cctgcagaac ctggcaagaa ccatcagcga ggcaggacag 960

gcaatggcat ccaccgaggg caatgtgaca ggcatgttcg caggaggagg cagcggactg 1020

cctggacggg acggcagaga tggaagggag ggacccaggg gcgagaaggg cgaccctgga 1080

ctgcctggac caatgggact gtctggactg cagggaccaa caggacctgt gggaccaaag 1140

ggagagaatg gcagcgccgg cgagcccggc cctaagggag agaggggact gagcggagga 1200

ggaggctcct ttgtgaccac acagccagag gccctggcag cagcagcagc aaacctgcag 1260

ggcatcggca ccacaatgaa cgcccagaat gcagcagcag cagcaccaac cacaggagtg 1320

gtgcccgccg ccgccgatga ggtgtccgcc ctgaccgcag cacagttcgc agcacacgcc 1380

cagatgtacc agacagtgtc tgcccaggca gcagcaatcc acgagatgtt tgtgaacacc 1440

ctggtggcct cctctggctc ctatgccgcc acagaggccg ccaatgcagc agcagcagga 1500

ggatgcccag gactgcctgg cgccgccggc cccaagggcg aggccggcgc caagggcgac 1560

cggggcgaga gcggcctgcc aggcatcccc ggcaaggagg gacctaccgg accaaagggc 1620

aatcagggcg agaagggcat cagaggcgag aagggcgatt ctggaccaag cggaggaggc 1680

agcaccgaga acctgacagt gcagccagag aggctgggcg tgctggcatc ccaccacgac 1740

aatgccgccg tggatgccag ctccggagtg gaggcagcag caggactggg agagagcgtg 1800

gccatcaccc acggccctta ctgttcccag ttcaacgaca ccctgaacgt gtacctgaca 1860

gcccacaacg cactgggctc tagcctgcac acagcaggcg tggatctggc caagtctctg 1920

agaatcgccg ccaagatcta cagcgaggca gacgaggcat ggaggaaggc catcgatgga 1980

ctgtttaccg gaggaggctc cggactgaga ggcctgcagg gaccacctgg cgccctggga 2040

ccaccaggct ccgtgggctc tccaggcagc ccaggaccaa agggacagaa gggcgaccac 2100

ggcgataacc gcgccatcga ggagaagctg gccaatatgg aggccgagat cagaatcctg 2160

aagagcaagc tgcagctgac caacaagctg cacgccttct ctatgggagg aggcagcggc 2220

gacgaggacc cccagatcgc agcacacgtg gtgagcgagg caaactccaa tgccgcctct 2280

gtgctgcagt gggccaagaa gggctactat accatgaagt ccaacctggt catgctggag 2340

aatggcaagc agctgacagt gaagcgcgag ggcctgtact acgtgtacac ccaggtgaca 2400

ttctgctcta accgcgagcc ttcctctcag cggcccttca tcgtgggact gtggctgaag 2460

cccagctccg gctctgagag aatcctgctg aaggccgcca atacccactc tagctcccag 2520

ctgtgcgagc agcagagcgt gcacctggga ggcgtgttcg agctgcaggc aggagcctcc 2580

gtgtttgtga acgtgacaga ggcctctcag gtcatccaca gggtgggctt ctctagcttt 2640

ggcctgctga agctgtga 2658

<210>  9
<211>  506
<212>  PRT
<213>  Artificial Sequence

<220>
<223>  S40-H

<400>  9

Met Leu Pro Phe Leu Ser Met Leu Val Leu Leu Val Gln Pro Leu Gly
1               5                   10                  15

Asn Leu Gly Ala Glu Met Lys Ser Leu Ser Gln Arg Ser Val Pro Asn
            20                  25                  30

Thr Cys Thr Leu Val Met Cys Ser Pro Thr Glu Asn Gly Leu Pro Gly
        35                  40                  45

Arg Asp Gly Arg Asp Gly Arg Glu Gly Pro Arg Gly Glu Lys Gly Asp
    50                  55                  60

Pro Gly Leu Pro Gly Pro Met Gly Leu Ser Gly Leu Gln Gly Pro Thr
65                  70                  75                  80

Gly Pro Val Gly Pro Lys Gly Glu Asn Gly Ser Ala Gly Glu Pro Gly
                85                  90                  95

Pro Lys Gly Glu Arg Gly Leu Ser Gly Gly Ser Gly Ser Gln Ile Met
            100                 105                 110

Tyr Asn Tyr Pro Ala Met Leu Gly His Ala Gly Asp Met Ala Gly Tyr
        115                 120                 125

Ala Gly Thr Leu Gln Ser Leu Gly Ala Glu Ile Ala Val Glu Gln Ala
    130                 135                 140

Ala Leu Gln Ser Ala Trp Gln Gly Asp Thr Gly Ile Thr Tyr Gln Ala
145                 150                 155                 160

Trp Gln Ala Gln Trp Asn Gln Ala Met Glu Asp Leu Val Arg Ala Tyr
                165                 170                 175

His Ala Met Ser Ser Thr His Glu Ala Asn Thr Met Ala Met Met Ala
            180                 185                 190

Arg Asp Thr Ala Glu Ala Ala Lys Trp Gly Gly Gly Ser Gly Pro Pro
        195                 200                 205

Gly Leu Pro Gly Ile Pro Gly Pro Ala Gly Lys Glu Gly Pro Ser Gly
    210                 215                 220

Lys Gln Gly Asn Ile Gly Pro Gln Gly Lys Pro Gly Pro Lys Gly Glu
225                 230                 235                 240

Ala Gly Pro Lys Gly Glu Val Gly Ala Pro Gly Met Gln Gly Ser Thr
                245                 250                 255

Gly Ala Lys Gly Ser Thr Gly Pro Lys Gly Glu Arg Gly Ala Pro Gly
            260                 265                 270

Val Gln Gly Ala Pro Gly Asn Ala Gly Ala Ala Gly Pro Ala Gly Pro
        275                 280                 285

Ala Gly Pro Gln Gly Ala Pro Gly Ser Arg Gly Pro Pro Gly Leu Lys
    290                 295                 300

Gly Asp Arg Gly Val Pro Gly Asp Arg Gly Ile Lys Gly Glu Ser Gly
305                 310                 315                 320

Leu Pro Asp Ser Ala Ala Leu Arg Gln Gln Met Glu Ala Leu Lys Gly
                325                 330                 335

Lys Leu Gln Arg Leu Glu Val Ala Phe Ser His Tyr Gln Lys Ala Ala
            340                 345                 350

Leu Phe Pro Asp Gly Gly Gly Ser Gly Asp Glu Asp Pro Gln Ile Ala
        355                 360                 365

Ala His Val Val Ser Glu Ala Asn Ser Asn Ala Ala Ser Val Leu Gln
    370                 375                 380

Trp Ala Lys Lys Gly Tyr Tyr Thr Met Lys Ser Asn Leu Val Met Leu
385                 390                 395                 400

Glu Asn Gly Lys Gln Leu Thr Val Lys Arg Glu Gly Leu Tyr Tyr Val
                405                 410                 415

Tyr Thr Gln Val Thr Phe Cys Ser Asn Arg Glu Pro Ser Ser Gln Arg
            420                 425                 430

Pro Phe Ile Val Gly Leu Trp Leu Lys Pro Ser Ser Gly Ser Glu Arg
        435                 440                 445

Ile Leu Leu Lys Ala Ala Asn Thr His Ser Ser Ser Gln Leu Cys Glu
    450                 455                 460

Gln Gln Ser Val His Leu Gly Gly Val Phe Glu Leu Gln Ala Gly Ala
465                 470                 475                 480

Ser Val Phe Val Asn Val Thr Glu Ala Ser Gln Val Ile His Arg Val
                485                 490                 495

Gly Phe Ser Ser Phe Gly Leu Leu Lys Leu
            500                 505

<210>  10
<211>  1521
<212>  DNA
<213>  Artificial Sequence

<220>
<223>  S40-H

<400>  10
atgctgcctt tcctgtctat gctggtcctg ctggtgcagc cactggggaa cctgggagct      60

gagatgaaga gcctgtcaca gagaagtgtg cccaacacct gcacactggt catgtgcagc     120

ccaaccgaga acggcctgcc aggaagggac ggaagagacg gaagggaggg acccagaggc     180

gagaagggcg accctggcct gcctggacca atgggcctgt ccggcctgca gggaccaaca     240

ggacctgtgg gaccaaaggg agagaacggc agcgccggcg agcccggccc taagggagag     300

aggggcctgt ccggaggcag cggctcccag atcatgtaca attatcctgc catgctggga     360

cacgcaggcg atatggcagg atacgcaggc accctgcagt ctctgggagc agagatcgca     420

gtggagcagg ccgccctgca gagcgcctgg cagggcgaca ccggaatcac ataccaggca     480

tggcaggcac agtggaacca ggccatggag gatctggtga gagcctatca cgccatgagc     540

tccacccacg aggccaatac aatggccatg atggccaggg acacagccga ggccgccaag     600

tggggaggag gctctggacc acctggcctg cccggcatcc caggccccgc cggcaaggag     660

ggccctagcg gcaagcaggg caatatcgga ccacagggca agcctggacc aaagggagag     720

gcaggaccaa agggagaagt gggagcaccc ggcatgcagg gctccaccgg agcaaagggc     780

tctacaggcc ctaagggcga gcgcggcgcc cctggcgtgc agggcgcccc aggcaacgca     840

ggagcagcag gaccagcagg acctgccggc ccacagggcg ccccaggcag ccggggccca     900

cccggcctga agggcgaccg cggcgtgcca ggcgatcggg gcatcaaggg agagtctggc     960

ctgccagaca gcgccgccct gaggcagcag atggaggccc tgaagggcaa gctgcagcgg    1020

ctggaggtgg ccttctccca ctaccagaag gccgccctgt ttccagatgg aggaggctct    1080

ggcgacgagg acccccagat cgcagcacac gtggtgtctg aggccaacag caatgccgcc    1140

tccgtgctgc agtgggccaa gaagggctac tataccatga agtccaacct ggtcatgctg    1200

gagaatggca agcagctgac agtgaagaga gagggcctgt actacgtgta cacccaggtg    1260

acattctgct ccaacagaga gccctctagc cagaggcctt ttatcgtggg cctgtggctg 1320

aagccctcct ctggctctga gagaatcctg ctgaaggccg ccaataccca cagctcctct 1380

cagctgtgcg agcagcagag cgtgcacctg ggaggcgtgt tcgagctgca ggcaggagcc 1440

tccgtgtttg tgaacgtgac tgaggcatca caggtcatcc atcgggtggg ctttagctcc 1500

ttcgggctgc tgaaactgtg a 1521

<210> 11
<211> 736
<212> PRT
<213> Artificial Sequence

<220>
<223> S40-HAPE

<400> 11

Met Leu Pro Phe Leu Ser Met Leu Val Leu Leu Val Gln Pro Leu Gly
1               5                   10                  15

Asn Leu Gly Ala Glu Met Lys Ser Leu Ser Gln Arg Ser Val Pro Asn
            20                  25                  30

Thr Cys Thr Leu Val Met Cys Ser Pro Thr Glu Asn Gly Leu Pro Gly
        35                  40                  45

Arg Asp Gly Arg Asp Gly Arg Glu Gly Pro Arg Gly Glu Lys Gly Asp
    50                  55                  60

Pro Gly Leu Pro Gly Pro Met Gly Leu Ser Gly Leu Gln Gly Pro Thr
65                  70                  75                  80

Gly Pro Val Gly Pro Lys Gly Glu Asn Gly Ser Ala Gly Glu Pro Gly
                85                  90                  95

Pro Lys Gly Glu Arg Gly Leu Ser Gly Gly Ser Gly Ser Gln Ile Met
            100                 105                 110

Tyr Asn Tyr Pro Ala Met Leu Gly His Ala Gly Asp Met Ala Gly Tyr
        115                 120                 125

Ala Gly Thr Leu Gln Ser Leu Gly Ala Glu Ile Ala Val Glu Gln Ala
    130                 135                 140

Ala Leu Gln Ser Ala Trp Gln Gly Asp Thr Gly Ile Thr Tyr Gln Ala
145                 150                 155                 160

Trp Gln Ala Gln Trp Asn Gln Ala Met Glu Asp Leu Val Arg Ala Tyr

165                 170                 175

His Ala Met Ser Ser Thr His Glu Ala Asn Thr Met Ala Met Met Ala
            180                 185                 190

Arg Asp Thr Ala Glu Ala Ala Lys Trp Gly Gly Gly Ser Gly Gly Thr
        195                 200                 205

Glu Gln Gln Trp Asn Phe Ala Gly Ile Glu Ala Ala Ala Ser Ala Ile
    210                 215                 220

Gln Gly Asn Val Thr Ser Ile His Ser Leu Leu Asp Glu Gly Lys Gln
225                 230                 235                 240

Ser Leu Thr Lys Leu Ala Ala Ala Trp Gly Gly Ser Gly Ser Glu Ala
                245                 250                 255

Tyr Gln Gly Val Gln Gln Lys Trp Asp Ala Thr Ala Thr Glu Leu Asn
            260                 265                 270

Asn Ala Leu Gln Asn Leu Ala Arg Thr Ile Ser Glu Ala Gly Gln Ala
        275                 280                 285

Met Ala Ser Thr Glu Gly Asn Val Thr Gly Met Phe Ala Gly Gly Gly
    290                 295                 300

Ser Gly Gly Ser Phe Val Thr Thr Gln Pro Glu Ala Leu Ala Ala Ala
305                 310                 315                 320

Ala Ala Asn Leu Gln Gly Ile Gly Thr Thr Met Asn Ala Gln Asn Ala
                325                 330                 335

Ala Ala Ala Ala Pro Thr Thr Gly Val Val Pro Ala Ala Ala Asp Glu
            340                 345                 350

Val Ser Ala Leu Thr Ala Ala Gln Phe Ala Ala His Ala Gln Met Tyr
        355                 360                 365

Gln Thr Val Ser Ala Gln Ala Ala Ala Ile His Glu Met Phe Val Asn
    370                 375                 380

Thr Leu Val Ala Ser Ser Gly Ser Tyr Ala Ala Thr Glu Ala Ala Asn
385                 390                 395                 400

Ala Ala Ala Ala Gly Gly Gly Ser Gly Gly Thr Glu Asn Leu Thr Val
                405                 410                 415

Gln Pro Glu Arg Leu Gly Val Leu Ala Ser His His Asp Asn Ala Ala
            420                 425                 430

Val Asp Ala Ser Ser Gly Val Glu Ala Ala Ala Gly Leu Gly Glu Ser
        435                 440                 445

Val Ala Ile Thr His Gly Pro Tyr Cys Ser Gln Phe Asn Asp Thr Leu
    450                 455                 460

Asn Val Tyr Leu Thr Ala His Asn Ala Leu Gly Ser Ser Leu His Thr
465                 470                 475                 480

Ala Gly Val Asp Leu Ala Lys Ser Leu Arg Ile Ala Ala Lys Ile Tyr
                485                 490                 495

Ser Glu Ala Asp Glu Ala Trp Arg Lys Ala Ile Asp Gly Leu Phe Thr
            500                 505                 510

Gly Ser Gly Gly Ser Gly Gly Leu Arg Gly Leu Gln Gly Pro Pro Gly
        515                 520                 525

Ala Leu Gly Pro Pro Gly Ser Val Gly Ser Pro Gly Ser Pro Gly Pro
    530                 535                 540

Lys Gly Gln Lys Gly Asp His Gly Asp Asn Arg Ala Ile Glu Glu Lys
545                 550                 555                 560

Leu Ala Asn Met Glu Ala Glu Ile Arg Ile Leu Lys Ser Lys Leu Gln
                565                 570                 575

Leu Thr Asn Lys Leu His Ala Phe Ser Met Gly Gly Gly Ser Gly Asp
            580                 585                 590

Glu Asp Pro Gln Ile Ala Ala His Val Val Ser Glu Ala Asn Ser Asn
        595                 600                 605

Ala Ala Ser Val Leu Gln Trp Ala Lys Lys Gly Tyr Tyr Thr Met Lys
    610                 615                 620

Ser Asn Leu Val Met Leu Glu Asn Gly Lys Gln Leu Thr Val Lys Arg
625                 630                 635                 640

Glu Gly Leu Tyr Tyr Val Tyr Thr Gln Val Thr Phe Cys Ser Asn Arg
                645                 650                 655

Glu Pro Ser Ser Gln Arg Pro Phe Ile Val Gly Leu Trp Leu Lys Pro
            660                 665                 670

Ser Ser Gly Ser Glu Arg Ile Leu Leu Lys Ala Ala Asn Thr His Ser
        675                 680                 685

Ser Ser Gln Leu Cys Glu Gln Gln Ser Val His Leu Gly Gly Val Phe
    690                 695                 700

Glu Leu Gln Ala Gly Ala Ser Val Phe Val Asn Val Thr Glu Ala Ser
705                 710                 715                 720

Gln Val Ile His Arg Val Gly Phe Ser Ser Phe Gly Leu Leu Lys Leu
                725                 730                 735

<210>  12
<211>  2211
<212>  DNA
<213>  Artificial Sequence

<220>
<223>  S40-HAPE

<400>  12
atgctgccct tcctgtctat gctggtcctg ctggtccagc cactgggaaa cctgggagcc      60

gaaatgaaaa gcctgagtca gaggtcagtg ccaaacacct gcacactggt catgtgcagc     120

ccaaccgaga acggcctgcc aggaagggac ggaagggacg gaagggaggg acccagaggc     180

gagaagggcg accctggcct gcccggccct atgggcctgt ccggcctgca gggaccaaca     240

ggccccgtgg gccctaaggg cgagaacggc agcgccggag agccaggacc aaagggcgag     300

aggggcctgt ccggcggctc cggctctcag atcatgtaca attatcccgc catgctggga     360

cacgcaggcg atatggcagg atacgcaggc accctgcagt ctctgggagc agagatcgca     420

gtggagcagg ccgccctgca gagcgcctgg cagggcgata ccggcatcac ataccaggct     480

tggcaggcac agtggaacca ggccatggag gacctggtga gagcctatca cgccatgagc     540

tccacccacg aggccaatac aatggccatg atggccaggg acaccgccga ggcagcaaag     600

tggggaggag gctccggcgg cacagagcag cagtggaact ttgcaggaat cgaggcagca     660

gcatctgcca tccagggcaa tgtgacctct atccacagcc tgctggacga gggcaagcag     720

agcctgacaa agctggcagc agcatgggga ggcagcggct ccgaggcata tcagggagtg     780

cagcagaagt gggatgccac cgccacagag ctgaacaatg ccctgcagaa cctggcaagg     840

accatcagcg aggcaggaca ggccatggcc tccaccgagg gcaatgtgac aggcatgttc     900

gcaggcggcg gctccggcgg ctcttttgtg accacacagc ctgaggccct ggcagcagca     960

gcagcaaatc tgcagggcat cggcaccaca atgaacgccc agaatgcagc agcagcagca    1020

cctaccacag gcgtggtgcc agccgccgcc gacgaggtga gcgccctgac cgccgcccag    1080

ttcgccgccc acgcccagat gtaccagaca gtgtccgccc aggcagcagc aatccacgag    1140

atgtttgtga acaccctggt ggcctctagc ggcagctatg cagcaacaga ggcagcaaac 1200

gcagcagcag caggcggggg ctccggcggc accgagaatc tgacagtgca gccagagcgg 1260

ctgggcgtgc tggcaagcca ccacgataat gccgccgtgg acgcctcctc tggcgtggag 1320

gcagcagcag gcctgggaga gtccgtggcc atcacccacg gcccttactg ctctcagttt 1380

aacgacaccc tgaacgtgta cctgacagca cacaacgcac tgggcagctc cctgcacaca 1440

gcaggcgtgg atctggccaa gtccctgaga atcgccgcca aaatctactc tgaggccgat 1500

gaggcctgga ggaaggccat cgacggcctg ttcaccggca gcggcggatc cggaggcctg 1560

aggggcctgc agggaccacc aggcgccctg ggccctccag gctctgtggg cagccccggc 1620

tcccccggac ctaagggaca gaagggcgat cacggcgaca accgcgccat cgaggagaag 1680

ctggccaata tggaggccga gatcagaatc ctgaagagca agctgcagct gaccaacaag 1740

ctgcacgcct tctccatggg aggaggctct ggcgatgagg acccccagat cgcagcacac 1800

gtggtgtctg aggccaacag caatgccgcc tccgtgctgc agtgggccaa gaagggctac 1860

tataccatga agtccaacct ggtcatgctg gagaatggca agcagctgac agtgaagcgc 1920

gagggcctgt actacgtgta cacccaggtg acattctgca gcaacaggga gcccagctcc 1980

cagcggcctt ttatcgtggg cctgtggctg aagccttcta gcggctccga gagaatcctg 2040

ctgaaggccg ccaataccca ctcctctagc cagctgtgcg agcagcagtc cgtgcacctg 2100

ggaggcgtgt tcgagctgca ggcaggagca agcgtgttcg tgaacgtgac tgaggcatcc 2160

caggttatcc acagggtcgg ctttagtagc ttcggactgc tgaaactgtg a 2211

<210> 13
<211> 1004
<212> PRT
<213> Artificial Sequence

<220>
<223> S40-HAPEHR

<400> 13

Met Leu Pro Phe Leu Ser Met Leu Val Leu Leu Val Gln Pro Leu Gly
1               5                   10                  15

Asn Leu Gly Ala Glu Met Lys Ser Leu Ser Gln Arg Ser Val Pro Asn
            20                  25                  30

Thr Cys Thr Leu Val Met Cys Ser Pro Thr Glu Asn Gly Leu Pro Gly
        35                  40                  45

Arg Asp Gly Arg Asp Gly Arg Glu Gly Pro Arg Gly Glu Lys Gly Asp
    50                  55                  60

Pro Gly Leu Pro Gly Pro Met Gly Leu Ser Gly Leu Gln Gly Pro Thr
65                  70                  75                  80

Gly Pro Val Gly Pro Lys Gly Glu Asn Gly Ser Ala Gly Glu Pro Gly
                85                  90                  95

Pro Lys Gly Glu Arg Gly Leu Ser Gly Gly Ser Gly Ser Gln Ile Met
            100                 105                 110

Tyr Asn Tyr Pro Ala Met Leu Gly His Ala Gly Asp Met Ala Gly Tyr
        115                 120                 125

Ala Gly Thr Leu Gln Ser Leu Gly Ala Glu Ile Ala Val Glu Gln Ala
    130                 135                 140

Ala Leu Gln Ser Ala Trp Gln Gly Asp Thr Gly Ile Thr Tyr Gln Ala
145                 150                 155                 160

Trp Gln Ala Gln Trp Asn Gln Ala Met Glu Asp Leu Val Arg Ala Tyr
                165                 170                 175

His Ala Met Ser Ser Thr His Glu Ala Asn Thr Met Ala Met Met Ala
            180                 185                 190

Arg Asp Thr Ala Glu Ala Ala Lys Trp Gly Gly Gly Ser Gly Gly Thr
        195                 200                 205

Glu Gln Gln Trp Asn Phe Ala Gly Ile Glu Ala Ala Ala Ser Ala Ile
    210                 215                 220

Gln Gly Asn Val Thr Ser Ile His Ser Leu Leu Asp Glu Gly Lys Gln
225                 230                 235                 240

Ser Leu Thr Lys Leu Ala Ala Ala Trp Gly Gly Ser Gly Ser Glu Ala
                245                 250                 255

Tyr Gln Gly Val Gln Gln Lys Trp Asp Ala Thr Ala Thr Glu Leu Asn
            260                 265                 270

Asn Ala Leu Gln Asn Leu Ala Arg Thr Ile Ser Glu Ala Gly Gln Ala
        275                 280                 285

Met Ala Ser Thr Glu Gly Asn Val Thr Gly Met Phe Ala Gly Gly Gly
    290                 295                 300

Ser Gly Gly Ser Phe Val Thr Thr Gln Pro Glu Ala Leu Ala Ala Ala
305                 310                 315                 320

Ala Ala Asn Leu Gln Gly Ile Gly Thr Thr Met Asn Ala Gln Asn Ala
                325                 330                 335

Ala Ala Ala Ala Pro Thr Thr Gly Val Val Pro Ala Ala Ala Asp Glu
            340                 345                 350

Val Ser Ala Leu Thr Ala Ala Gln Phe Ala Ala His Ala Gln Met Tyr
        355                 360                 365

Gln Thr Val Ser Ala Gln Ala Ala Ala Ile His Glu Met Phe Val Asn
    370                 375                 380

Thr Leu Val Ala Ser Ser Gly Ser Tyr Ala Ala Thr Glu Ala Ala Asn
385                 390                 395                 400

Ala Ala Ala Ala Gly Gly Gly Ser Gly Gly Thr Glu Asn Leu Thr Val
                405                 410                 415

Gln Pro Glu Arg Leu Gly Val Leu Ala Ser His His Asp Asn Ala Ala
            420                 425                 430

Val Asp Ala Ser Ser Gly Val Glu Ala Ala Ala Gly Leu Gly Glu Ser
        435                 440                 445

Val Ala Ile Thr His Gly Pro Tyr Cys Ser Gln Phe Asn Asp Thr Leu
    450                 455                 460

Asn Val Tyr Leu Thr Ala His Asn Ala Leu Gly Ser Ser Leu His Thr
465                 470                 475                 480

Ala Gly Val Asp Leu Ala Lys Ser Leu Arg Ile Ala Ala Lys Ile Tyr
                485                 490                 495

Ser Glu Ala Asp Glu Ala Trp Arg Lys Ala Ile Asp Gly Leu Phe Thr
            500                 505                 510

Gly Ser Gly Gly Ser Gly Gly Thr Thr Ala Arg Asp Ile Met Asn Ala
        515                 520                 525

Gly Val Thr Cys Val Gly Glu His Glu Thr Leu Thr Ala Ala Ala Gln
    530                 535                 540

Tyr Met Arg Glu His Asp Ile Gly Ala Leu Pro Ile Cys Gly Asp Asp
545                 550                 555                 560

Asp Arg Leu His Gly Met Leu Thr Asp Arg Asp Ile Val Ile Lys Gly

                565                 570                 575

Leu Ala Ala Gly Leu Asp Pro Asn Thr Ala Thr Ala Gly Glu Leu Ala
            580                 585                 590

Arg Asp Ser Ile Tyr Tyr Val Asp Ala Asn Ala Ser Ile Gln Glu Met
        595                 600                 605

Leu Asn Val Met Glu Glu His Gln Val Arg Arg Val Pro Val Ile Ser
    610                 615                 620

Glu His Arg Leu Val Gly Ile Val Thr Glu Ala Asp Ile Ala Arg His
625                 630                 635                 640

Leu Pro Glu His Ala Ile Val Gln Phe Val Lys Ala Ile Cys Ser Pro
                645                 650                 655

Met Ala Leu Ala Ser Gly Gly Gly Ser Gly Ser Gly Gly Leu Ser Thr
            660                 665                 670

Ile Ser Ser Lys Ala Asp Asp Ile Asp Trp Asp Ala Ile Ala Gln Cys
        675                 680                 685

Glu Ser Gly Gly Asn Trp Ala Ala Asn Thr Gly Asn Gly Leu Tyr Gly
    690                 695                 700

Gly Leu Gln Ile Ser Gln Ala Thr Trp Asp Ser Asn Gly Gly Val Gly
705                 710                 715                 720

Ser Pro Ala Ala Ala Ser Pro Gln Gln Gln Ile Glu Val Ala Asp Asn
                725                 730                 735

Ile Met Lys Thr Gln Gly Pro Gly Ala Trp Pro Lys Cys Ser Ser Cys
            740                 745                 750

Ser Gln Gly Asp Ala Pro Leu Gly Ser Leu Thr His Ile Leu Thr Phe
        755                 760                 765

Leu Ala Ala Glu Thr Gly Gly Cys Ser Gly Ser Arg Asp Asp Gly Gly
    770                 775                 780

Ser Gly Gly Leu Arg Gly Leu Gln Gly Pro Pro Gly Ala Leu Gly Pro
785                 790                 795                 800

Pro Gly Ser Val Gly Ser Pro Gly Ser Pro Gly Pro Lys Gly Gln Lys
                805                 810                 815

Gly Asp His Gly Asp Asn Arg Ala Ile Glu Glu Lys Leu Ala Asn Met
            820                 825                 830

Glu Ala Glu Ile Arg Ile Leu Lys Ser Lys Leu Gln Leu Thr Asn Lys
        835                 840                 845

Leu His Ala Phe Ser Met Gly Gly Gly Ser Gly Asp Glu Asp Pro Gln
    850                 855                 860

Ile Ala Ala His Val Val Ser Glu Ala Asn Ser Asn Ala Ala Ser Val
865                 870                 875                 880

Leu Gln Trp Ala Lys Lys Gly Tyr Tyr Thr Met Lys Ser Asn Leu Val
                885                 890                 895

Met Leu Glu Asn Gly Lys Gln Leu Thr Val Lys Arg Glu Gly Leu Tyr
            900                 905                 910

Tyr Val Tyr Thr Gln Val Thr Phe Cys Ser Asn Arg Glu Pro Ser Ser
        915                 920                 925

Gln Arg Pro Phe Ile Val Gly Leu Trp Leu Lys Pro Ser Ser Gly Ser
    930                 935                 940

Glu Arg Ile Leu Leu Lys Ala Ala Asn Thr His Ser Ser Ser Gln Leu
945                 950                 955                 960

Cys Glu Gln Gln Ser Val His Leu Gly Gly Val Phe Glu Leu Gln Ala
                965                 970                 975

Gly Ala Ser Val Phe Val Asn Val Thr Glu Ala Ser Gln Val Ile His
            980                 985                 990

Arg Val Gly Phe Ser Ser Phe Gly  Leu Leu Lys Leu
        995                 1000

<210>  14
<211>  3015
<212>  DNA
<213>  Artificial Sequence

<220>
<223>  S40-HAPEHR

<400>  14
atgctgccct tcctgtctat gctggtcctg ctggtccagc cactgggaaa cctgggagcc      60

gaaatgaaaa gcctgagtca gaggtcagtg ccaaacacct gcacactggt catgtgcagc     120

ccaaccgaga acggcctgcc aggaagggac ggaagggacg gaagggaggg acccagaggc     180

gagaagggcg accctggcct gcccggccct atgggcctgt ccggcctgca gggaccaaca 240

ggccccgtgg gccctaaggg cgagaacggc agcgccggag agccaggacc aaagggcgag 300

aggggcctgt ccggcggctc cggctctcag atcatgtaca attatcccgc catgctggga 360

cacgcaggcg atatggcagg atacgcaggc accctgcagt ctctgggagc agagatcgca 420

gtggagcagg ccgccctgca gagcgcctgg cagggcgata ccggcatcac ataccaggct 480

tggcaggcac agtggaacca ggccatggag gacctggtga gagcctatca cgccatgagc 540

tccacccacg aggccaatac aatggccatg atggccaggg acaccgccga ggcagcaaag 600

tggggaggag gctccggcgg cacagagcag cagtggaact ttgcaggaat cgaggcagca 660

gcatctgcca tccagggcaa tgtgacctct atccacagcc tgctggacga gggcaagcag 720

agcctgacaa agctggcagc agcatgggga ggcagcggct ccgaggcata tcagggagtg 780

cagcagaagt gggatgccac cgccacagag ctgaacaatg ccctgcagaa cctggcaagg 840

accatcagcg aggcaggaca ggccatggcc tccaccgagg gcaatgtgac aggcatgttc 900

gcaggcggcg gctccggcgg ctcttttgtg accacacagc ctgaggccct ggcagcagca 960

gcagcaaatc tgcagggcat cggcaccaca atgaacgccc agaatgcagc agcagcagca 1020

cctaccacag gcgtggtgcc agccgccgcc gacgaggtga gcgccctgac cgccgcccag 1080

ttcgccgccc acgcccagat gtaccagaca gtgtccgccc aggcagcagc aatccacgag 1140

atgtttgtga acaccctggt ggcctctagc ggcagctatg cagcaacaga ggcagcaaac 1200

gcagcagcag caggcggggg ctccggcggc accgagaatc tgacagtgca gccagagcgg 1260

ctgggcgtgc tggcaagcca ccacgataat gccgccgtgg acgcctcctc tggcgtggag 1320

gcagcagcag gcctgggaga gtccgtggcc atcacccacg gcccttactg ctctcagttt 1380

aacgacaccc tgaacgtgta cctgacagca cacaacgcac tgggcagctc cctgcacaca 1440

gcaggcgtgg atctggccaa gtccctgaga atcgccgcca aaatctactc tgaggccgat 1500

gaggcctgga ggaaggccat cgacggcctg ttcaccggca gcggcggatc cggcggaacc 1560

acagcccgcg acatcatgaa tgcaggagtg acatgcgtgg gagagcacga gacactgaca 1620

gcagcagccc agtatatgcg ggagcacgat atcggcgccc tgcctatctg tggcgacgat 1680

gaccgcctgc acggcatgct gaccgatcgg gatatcgtga tcaagggcct ggcagcaggc 1740

ctggacccaa acaccgcaac agcaggagag ctggccagag atagtatcta ctatgtggac 1800

gccaacgcct ccatccagga gatgctgaat gtgatggagg agcaccaggt gcggagagtg 1860

cccgtgatca gcgagcaccg cctggtggga atcgtgaccg aggcagatat cgcaaggcac 1920

ctgccagagc acgcaatcgt gcagtttgtg aaggccatct gcagccccat ggccctggca 1980

tccggcggag gctctggcag cggcggcctg agcacaatct ctagcaaggc cgatgatatc 2040

gattgggacg caatcgcaca gtgtgagtcc ggcggaaact gggcagcaaa caccggcaat 2100

ggcctgtacg gcggcctgca gatcagccag gccacatggg atagcaacgg aggagtgggc 2160

tctcccgcag cagcaagccc tcagcagcag atcgaggtgg ccgacaatat catgaagacc 2220

cagggacctg gagcttggcc aaagtgctcc tcttgttctc agggcgatgc acctctgggc 2280

agcctgaccc acatcctgac attcctggca gcagagacag gcggttgttc cggctctcgg 2340

gacgacggag gatccggagg cctgaggggc ctgcagggac caccaggcgc cctgggccct 2400

ccaggctctg tgggcagccc cggctccccc ggacctaagg gacagaaggg cgatcacggc 2460

gacaaccgcg ccatcgagga gaagctggcc aatatggagg ccgagatcag aatcctgaag 2520

agcaagctgc agctgaccaa caagctgcac gccttctcca tgggaggagg ctctggcgat 2580

gaggaccccc agatcgcagc acacgtggtg tctgaggcca acagcaatgc cgcctccgtg 2640

ctgcagtggg ccaagaaggg ctactatacc atgaagtcca acctggtcat gctggagaat 2700

ggcaagcagc tgacagtgaa gcgcgagggc ctgtactacg tgtacaccca ggtgacattc 2760

tgcagcaaca gggagcccag ctcccagcgg ccttttatcg tgggcctgtg gctgaagcct 2820

tctagcggct ccgagagaat cctgctgaag gccgccaata cccactcctc tagccagctg 2880

tgcgagcagc agtccgtgca cctgggaggc gtgttcgagc tgcaggcagg agcaagcgtg 2940

ttcgtgaacg tgactgaggc atcccaggtt atccacaggg tcggctttag tagcttcgga 3000

ctgctgaaac tgtga 3015

<210> 15
<211> 1128
<212> PRT
<213> Artificial Sequence

<220>
<223> S40-HAPEHR-20

<400> 15

Met Leu Pro Phe Leu Ser Met Leu Val Leu Leu Val Gln Pro Leu Gly
1               5                   10                  15

Asn Leu Gly Ala Glu Met Lys Ser Leu Ser Gln Arg Ser Val Pro Asn
            20                  25                  30

Thr Cys Thr Leu Val Met Cys Ser Pro Thr Glu Asn Gly Leu Pro Gly
        35                  40                  45

Arg Asp Gly Arg Asp Gly Arg Glu Gly Pro Arg Gly Glu Lys Gly Asp
    50                  55                  60

Pro Gly Leu Pro Gly Pro Met Gly Leu Ser Gly Leu Gln Gly Pro Thr
65                  70                  75                  80

Gly Pro Val Gly Pro Lys Gly Glu Asn Gly Ser Ala Gly Glu Pro Gly
                85                  90                  95

Pro Lys Gly Glu Arg Gly Leu Ser Gly Gly Ser Gly Ser Gln Ile Met
            100                 105                 110

Tyr Asn Tyr Pro Ala Met Leu Gly His Ala Gly Asp Met Ala Gly Tyr
        115                 120                 125

Ala Gly Thr Leu Gln Ser Leu Gly Ala Glu Ile Ala Val Glu Gln Ala
    130                 135                 140

Ala Leu Gln Ser Ala Trp Gln Gly Asp Thr Gly Ile Thr Tyr Gln Ala
145                 150                 155                 160

Trp Gln Ala Gln Trp Asn Gln Ala Met Glu Asp Leu Val Arg Ala Tyr
                165                 170                 175

His Ala Met Ser Ser Thr His Glu Ala Asn Thr Met Ala Met Met Ala
            180                 185                 190

Arg Asp Thr Ala Glu Ala Ala Lys Trp Gly Gly Gly Ser Gly Gly Thr
        195                 200                 205

Glu Gln Gln Trp Asn Phe Ala Gly Ile Glu Ala Ala Ala Ser Ala Ile
    210                 215                 220

Gln Gly Asn Val Thr Ser Ile His Ser Leu Leu Asp Glu Gly Lys Gln
225                 230                 235                 240

Ser Leu Thr Lys Leu Ala Ala Ala Trp Gly Gly Ser Gly Ser Glu Ala
                245                 250                 255

Tyr Gln Gly Val Gln Gln Lys Trp Asp Ala Thr Ala Thr Glu Leu Asn
            260                 265                 270

Asn Ala Leu Gln Asn Leu Ala Arg Thr Ile Ser Glu Ala Gly Gln Ala
        275                 280                 285

Met Ala Ser Thr Glu Gly Asn Val Thr Gly Met Phe Ala Gly Gly Gly
    290                 295                 300

Ser Gly Gly Ser Phe Val Thr Thr Gln Pro Glu Ala Leu Ala Ala Ala
305                 310                 315                 320

Ala Ala Asn Leu Gln Gly Ile Gly Thr Thr Met Asn Ala Gln Asn Ala
                325                 330                 335

Ala Ala Ala Ala Pro Thr Thr Gly Val Val Pro Ala Ala Ala Asp Glu
            340                 345                 350

Val Ser Ala Leu Thr Ala Ala Gln Phe Ala Ala His Ala Gln Met Tyr
        355                 360                 365

Gln Thr Val Ser Ala Gln Ala Ala Ala Ile His Glu Met Phe Val Asn
    370                 375                 380

Thr Leu Val Ala Ser Ser Gly Ser Tyr Ala Ala Thr Glu Ala Ala Asn
385                 390                 395                 400

Ala Ala Ala Ala Gly Gly Gly Ser Gly Gly Thr Glu Asn Leu Thr Val
                405                 410                 415

Gln Pro Glu Arg Leu Gly Val Leu Ala Ser His His Asp Asn Ala Ala
            420                 425                 430

Val Asp Ala Ser Ser Gly Val Glu Ala Ala Ala Gly Leu Gly Glu Ser
        435                 440                 445

Val Ala Ile Thr His Gly Pro Tyr Cys Ser Gln Phe Asn Asp Thr Leu
    450                 455                 460

Asn Val Tyr Leu Thr Ala His Asn Ala Leu Gly Ser Ser Leu His Thr
465                 470                 475                 480

Ala Gly Val Asp Leu Ala Lys Ser Leu Arg Ile Ala Ala Lys Ile Tyr
                485                 490                 495

Ser Glu Ala Asp Glu Ala Trp Arg Lys Ala Ile Asp Gly Leu Phe Thr
            500                 505                 510

Gly Ser Gly Gly Ser Gly Gly Thr Thr Ala Arg Asp Ile Met Asn Ala
        515                 520                 525

Gly Val Thr Cys Val Gly Glu His Glu Thr Leu Thr Ala Ala Ala Gln
    530                 535                 540

Tyr Met Arg Glu His Asp Ile Gly Ala Leu Pro Ile Cys Gly Asp Asp
545                 550                 555                 560

Asp Arg Leu His Gly Met Leu Thr Asp Arg Asp Ile Val Ile Lys Gly
                565                 570                 575

Leu Ala Ala Gly Leu Asp Pro Asn Thr Ala Thr Ala Gly Glu Leu Ala

            580                 585                 590

Arg Asp Ser Ile Tyr Tyr Val Asp Ala Asn Ala Ser Ile Gln Glu Met
        595                 600                 605

Leu Asn Val Met Glu Glu His Gln Val Arg Arg Val Pro Val Ile Ser
    610                 615                 620

Glu His Arg Leu Val Gly Ile Val Thr Glu Ala Asp Ile Ala Arg His
625                 630                 635                 640

Leu Pro Glu His Ala Ile Val Gln Phe Val Lys Ala Ile Cys Ser Pro
                645                 650                 655

Met Ala Leu Ala Ser Gly Gly Gly Ser Gly Ser Gly Gly Leu Ser Thr
            660                 665                 670

Ile Ser Ser Lys Ala Asp Asp Ile Asp Trp Asp Ala Ile Ala Gln Cys
        675                 680                 685

Glu Ser Gly Gly Asn Trp Ala Ala Asn Thr Gly Asn Gly Leu Tyr Gly
    690                 695                 700

Gly Leu Gln Ile Ser Gln Ala Thr Trp Asp Ser Asn Gly Gly Val Gly
705                 710                 715                 720

Ser Pro Ala Ala Ala Ser Pro Gln Gln Gln Ile Glu Val Ala Asp Asn
                725                 730                 735

Ile Met Lys Thr Gln Gly Pro Gly Ala Trp Pro Lys Cys Ser Ser Cys
            740                 745                 750

Ser Gln Gly Asp Ala Pro Leu Gly Ser Leu Thr His Ile Leu Thr Phe
        755                 760                 765

Leu Ala Ala Glu Thr Gly Gly Cys Ser Gly Ser Arg Asp Asp Gly Gly
    770                 775                 780

Ser Gly Gly Gly Phe Pro Gly Pro Pro Gly Pro Lys Gly Glu Pro Gly
785                 790                 795                 800

Ser Pro Ala Gly Arg Gly Glu Arg Gly Phe Gln Gly Ser Pro Gly Lys
                805                 810                 815

Met Gly Pro Ala Gly Ser Lys Gly Glu Pro Gly Gly Ser Gly Ser Gly
            820                 825                 830

Gly Ala Ser Asn Tyr Asp Cys Cys Leu Ser Tyr Ile Gln Thr Pro Leu
        835                 840                 845

Pro Ser Arg Ala Ile Val Gly Phe Thr Arg Gln Met Ala Asp Glu Ala
    850                 855                 860

Cys Asp Ile Asn Ala Ile Ile Phe His Thr Lys Lys Arg Lys Ser Val
865                 870                 875                 880

Cys Ala Asp Pro Lys Gln Asn Trp Val Lys Arg Ala Val Asn Leu Leu
                885                 890                 895

Ser Leu Arg Val Lys Lys Met Gly Ser Gly Ser Gly Ser Gly Gly Leu
            900                 905                 910

Arg Gly Leu Gln Gly Pro Pro Gly Ala Leu Gly Pro Pro Gly Ser Val
        915                 920                 925

Gly Ser Pro Gly Ser Pro Gly Pro Lys Gly Gln Lys Gly Asp His Gly
    930                 935                 940

Asp Asn Arg Ala Ile Glu Glu Lys Leu Ala Asn Met Glu Ala Glu Ile
945                 950                 955                 960

Arg Ile Leu Lys Ser Lys Leu Gln Leu Thr Asn Lys Leu His Ala Phe
                965                 970                 975

Ser Met Gly Gly Gly Ser Gly Asp Glu Asp Pro Gln Ile Ala Ala His
            980                 985                 990

Val Val Ser Glu Ala Asn Ser Asn Ala Ala Ser Val Leu Gln Trp Ala
        995                 1000                1005

Lys Lys Gly Tyr Tyr Thr Met Lys Ser Asn Leu Val Met Leu Glu
    1010                1015                1020

Asn Gly Lys Gln Leu Thr Val Lys Arg Glu Gly Leu Tyr Tyr Val
    1025                1030                1035

Tyr Thr Gln Val Thr Phe Cys Ser Asn Arg Glu Pro Ser Ser Gln
    1040                1045                1050

Arg Pro Phe Ile Val Gly Leu Trp Leu Lys Pro Ser Ser Gly Ser
    1055                1060                1065

Glu Arg Ile Leu Leu Lys Ala Ala Asn Thr His Ser Ser Ser Gln
    1070                1075                1080

Leu Cys Glu Gln Gln Ser Val His Leu Gly Gly Val Phe Glu Leu
    1085                1090                1095

Gln Ala Gly Ala Ser Val Phe Val Asn Val Thr Glu Ala Ser Gln
    1100                1105                1110

Val Ile His Arg Val Gly Phe Ser Ser Phe Gly Leu Leu Lys Leu
    1115                1120                1125

<210> 16
<211> 3387
<212> DNA
<213> Artificial Sequence

<220>
<223> S40-HAPEHR-20

<400> 16
atgctgccct tcctgtctat gctggtcctg ctggtccagc cactgggaaa cctgggagcc      60

gaaatgaaaa gcctgagtca gaggtcagtg ccaaacacct gcacactggt catgtgcagc     120

ccaaccgaga acggcctgcc aggaagggac ggaagggacg gaagggaggg acccagaggc     180

gagaagggcg accctggcct gcccggccct atgggcctgt ccggcctgca gggaccaaca     240

ggccccgtgg gccctaaggg cgagaacggc agcgccggag agccaggacc aaagggcgag     300

aggggcctgt ccggcggctc cggctctcag atcatgtaca attatcccgc catgctggga     360

cacgcaggcg atatggcagg atacgcaggc accctgcagt ctctgggagc agagatcgca     420

gtggagcagg ccgccctgca gagcgcctgg cagggcgata ccggcatcac ataccaggct     480

tggcaggcac agtggaacca ggccatggag gacctggtga gagcctatca cgccatgagc     540

tccacccacg aggccaatac aatggccatg atggccaggg acaccgccga ggcagcaaag     600

tggggaggag gctccggcgg cacagagcag cagtggaact ttgcaggaat cgaggcagca     660

gcatctgcca tccagggcaa tgtgacctct atccacagcc tgctggacga gggcaagcag     720

agcctgacaa agctggcagc agcatgggga ggcagcggct ccgaggcata tcagggagtg     780

cagcagaagt gggatgccac cgccacagag ctgaacaatg ccctgcagaa cctggcaagg     840

accatcagcg aggcaggaca ggccatggcc tccaccgagg gcaatgtgac aggcatgttc     900

gcaggcggcg gctccggcgg ctcttttgtg accacacagc ctgaggccct ggcagcagca     960

gcagcaaatc tgcagggcat cggcaccaca atgaacgccc agaatgcagc agcagcagca    1020

cctaccacag gcgtggtgcc agccgccgcc gacgaggtga gcgccctgac cgccgcccag    1080

ttcgccgccc acgcccagat gtaccagaca gtgtccgccc aggcagcagc aatccacgag    1140

atgtttgtga acaccctggt ggcctctagc ggcagctatg cagcaacaga ggcagcaaac    1200

gcagcagcag caggcggggg ctccggcggc accgagaatc tgacagtgca gccagagcgg    1260

ctgggcgtgc tggcaagcca ccacgataat gccgccgtgg acgcctcctc tggcgtggag 1320

gcagcagcag gcctgggaga gtccgtggcc atcacccacg gcccttactg ctctcagttt 1380

aacgacaccc tgaacgtgta cctgacagca cacaacgcac tgggcagctc cctgcacaca 1440

gcaggcgtgg atctggccaa gtccctgaga atcgccgcca aaatctactc tgaggccgat 1500

gaggcctgga ggaaggccat cgacggcctg ttcaccggca gcggcggatc cggcggaacc 1560

acagcccgcg acatcatgaa tgcaggagtg acatgcgtgg gagagcacga gacactgaca 1620

gcagcagccc agtatatgcg ggagcacgat atcggcgccc tgcctatctg tggcgacgat 1680

gaccgcctgc acggcatgct gaccgatcgg gatatcgtga tcaaggccct ggcagcaggc 1740

ctggacccaa acaccgcaac agcaggagag ctggccagag atagtatcta ctatgtggac 1800

gccaacgcct ccatccagga gatgctgaat gtgatggagg agcaccaggt gcggagagtg 1860

cccgtgatca gcgagcaccg cctggtggga atcgtgaccg aggcagatat cgcaaggcac 1920

ctgccagagc acgcaatcgt gcagtttgtg aaggccatct gcagccccat ggccctggca 1980

tccggcggag gctctggcag cggcggcctg agcacaatct ctagcaaggc cgatgatatc 2040

gattgggacg caatcgcaca gtgtgagtcc ggcggaaact gggcagcaaa caccggcaat 2100

ggcctgtacg gcggcctgca gatcagccag gccacatggg atagcaacgg aggagtgggc 2160

tctcccgcag cagcaagccc tcagcagcag atcgaggtgg ccgacaatat catgaagacc 2220

cagggacctg gagcttggcc aaagtgctcc tcttgttctc agggcgatgc acctctgggc 2280

agcctgaccc acatcctgac attcctggca gcagagacag gcggttgttc cggctctcgg 2340

gacgacggag gatccggagg aggatttcca ggaccacctg gaccaaaggg cgagccaggc 2400

agccccgccg gcagaggaga gaggggattc caggctctcc ctggcaagat gggaccagca 2460

ggcagcaagg gagagccagg aggcagcggc tccggcggcg cctccaatta cgactgctgt 2520

ctgtcttata tccagacccc tctgccaagc agagccatcg tgggcttcac aaggcagatg 2580

gccgatgagg cctgcgacat caacgccatc atctttcaca ccaagaagag aaagagcgtg 2640

tgcgccgatc caaagcagaa ctgggtgaag agagccgtga atctgctgtc cctgagggtg 2700

aagaagatgg gctctggcag cggctccgga ggcctgaggg gcctgcaggg accaccaggc 2760

gccctgggcc ctccaggctc tgtgggcagc cccggctccc ccggacctaa gggacagaag 2820

ggcgatcacg gcgacaaccg cgccatcgag gagaagctgg ccaatatgga ggccgagatc 2880

agaatcctga agagcaagct gcagctgacc aacaagctgc acgccttctc catgggagga 2940

ggctctggcg atgaggaccc ccagatcgca gcacacgtgg tgtctgaggc caacagcaat 3000

gccgcctccg tgctgcagtg ggccaagaag ggctactata ccatgaagtc caacctggtc 3060

atgctggaga atggcaagca gctgacagtg aagcgcgagg gcctgtacta cgtgtacacc 3120

caggtgacat tctgcagcaa cagggagccc agctcccagc ggccttttat cgtgggcctg 3180

tggctgaagc cttctagcgg ctccgagaga atcctgctga aggccgccaa tacccactcc 3240

tctagccagc tgtgcgagca gcagtccgtg cacctgggag gcgtgttcga gctgcaggca 3300

ggagcaagcg tgttcgtgaa cgtgactgag gcatcccagg ttatccacag ggtcggcttt 3360

agtagcttcg gactgctgaa actgtga 3387

<210> 17
<211> 248
<212> PRT
<213> Homo sapiens

<400> 17

Met Ser Leu Phe Pro Ser Leu Pro Leu Leu Leu Leu Ser Met Val Ala
1               5                   10                  15

Ala Ser Tyr Ser Glu Thr Val Thr Cys Glu Asp Ala Gln Lys Thr Cys
            20                  25                  30

Pro Ala Val Ile Ala Cys Ser Ser Pro Gly Ile Asn Gly Phe Pro Gly
        35                  40                  45

Lys Asp Gly Arg Asp Gly Thr Lys Gly Glu Lys Gly Glu Pro Gly Gln
    50                  55                  60

Gly Leu Arg Gly Leu Gln Gly Pro Pro Gly Lys Leu Gly Pro Pro Gly
65                  70                  75                  80

Asn Pro Gly Pro Ser Gly Ser Pro Gly Pro Lys Gly Gln Lys Gly Asp
                85                  90                  95

Pro Gly Lys Ser Pro Asp Gly Asp Ser Ser Leu Ala Ala Ser Glu Arg
            100                 105                 110

Lys Ala Leu Gln Thr Glu Met Ala Arg Ile Lys Lys Trp Leu Thr Phe
        115                 120                 125

Ser Leu Gly Lys Gln Val Gly Asn Lys Phe Phe Leu Thr Asn Gly Glu
    130                 135                 140

Ile Met Thr Phe Glu Lys Val Lys Ala Leu Cys Val Lys Phe Gln Ala
145                 150                 155                 160

Ser Val Ala Thr Pro Arg Asn Ala Ala Glu Asn Gly Ala Ile Gln Asn
                165                 170                 175

Leu Ile Lys Glu Glu Ala Phe Leu Gly Ile Thr Asp Glu Lys Thr Glu

180                 185                 190

Gly Gln Phe Val Asp Leu Thr Gly Asn Arg Leu Thr Tyr Thr Asn Trp
        195                 200                 205

Asn Glu Gly Glu Pro Asn Asn Ala Gly Ser Asp Glu Asp Cys Val Leu
    210                 215                 220

Leu Leu Lys Asn Gly Gln Trp Asn Asp Val Pro Cys Ser Thr Ser His
225                 230                 235                 240

Leu Ala Val Cys Glu Phe Pro Ile
                245

<210>  18
<211>  375
<212>  PRT
<213>  Homo sapiens

<400>  18

Met Leu Leu Phe Leu Leu Ser Ala Leu Val Leu Leu Thr Gln Pro Leu
1               5                   10                  15

Gly Tyr Leu Glu Ala Glu Met Lys Thr Tyr Ser His Arg Thr Met Pro
            20                  25                  30

Ser Ala Cys Thr Leu Val Met Cys Ser Ser Val Glu Ser Gly Leu Pro
        35                  40                  45

Gly Arg Asp Gly Arg Asp Gly Arg Glu Gly Pro Arg Gly Glu Lys Gly
    50                  55                  60

Asp Pro Gly Leu Pro Gly Ala Ala Gly Gln Ala Gly Met Pro Gly Gln
65                  70                  75                  80

Ala Gly Pro Val Gly Pro Lys Gly Asp Asn Gly Ser Val Gly Glu Pro
                85                  90                  95

Gly Pro Lys Gly Asp Thr Gly Pro Ser Gly Pro Pro Gly Pro Pro Gly
            100                 105                 110

Val Pro Gly Pro Ala Gly Arg Glu Gly Pro Leu Gly Lys Gln Gly Asn
        115                 120                 125

Ile Gly Pro Gln Gly Lys Pro Gly Pro Lys Gly Glu Ala Gly Pro Lys
    130                 135                 140

Gly Glu Val Gly Ala Pro Gly Met Gln Gly Ser Ala Gly Ala Arg Gly

145                 150                 155                 160

Leu Ala Gly Pro Lys Gly Glu Arg Gly Val Pro Gly Glu Arg Gly Val
                165                 170                 175

Pro Gly Asn Thr Gly Ala Ala Gly Ser Ala Gly Ala Met Gly Pro Gln
            180                 185                 190

Gly Ser Pro Gly Ala Arg Gly Pro Pro Gly Leu Lys Gly Asp Lys Gly
        195                 200                 205

Ile Pro Gly Asp Lys Gly Ala Lys Gly Glu Ser Gly Leu Pro Asp Val
    210                 215                 220

Ala Ser Leu Arg Gln Gln Val Glu Ala Leu Gln Gly Gln Val Gln His
225                 230                 235                 240

Leu Gln Ala Ala Phe Ser Gln Tyr Lys Lys Val Glu Leu Phe Pro Asn
                245                 250                 255

Gly Gln Ser Val Gly Glu Lys Ile Phe Lys Thr Ala Gly Phe Val Lys
            260                 265                 270

Pro Phe Thr Glu Ala Gln Leu Leu Cys Thr Gln Ala Gly Gly Gln Leu
        275                 280                 285

Ala Ser Pro Arg Ser Ala Ala Glu Asn Ala Ala Leu Gln Gln Leu Val
    290                 295                 300

Val Ala Lys Asn Glu Ala Ala Phe Leu Ser Met Thr Asp Ser Lys Thr
305                 310                 315                 320

Glu Gly Lys Phe Thr Tyr Pro Thr Gly Glu Ser Leu Val Tyr Ser Asn
                325                 330                 335

Trp Ala Pro Gly Glu Pro Asn Asp Asp Gly Gly Ser Glu Asp Cys Val
            340                 345                 350

Glu Ile Phe Thr Asn Gly Lys Trp Asn Asp Arg Ala Cys Gly Glu Lys
        355                 360                 365

Arg Leu Val Val Cys Glu Phe
    370                 375

<210>  19
<211>  145
<212>  PRT
<213>  Homo sapiens

<400>  19

Asp Gln Asn Pro Gln Ile Ala Ala His Val Ile Ser Glu Ala Ser Ser
1               5                   10                  15

Lys Thr Thr Ser Val Leu Gln Trp Ala Glu Lys Gly Tyr Tyr Thr Met
            20                  25                  30

Ser Asn Asn Leu Val Thr Leu Glu Asn Gly Lys Gln Leu Thr Val Lys
        35                  40                  45

Arg Gln Gly Leu Tyr Tyr Ile Tyr Ala Gln Val Thr Phe Cys Ser Asn
    50                  55                  60

Arg Glu Ala Ser Ser Gln Ala Pro Phe Ile Ala Ser Leu Cys Leu Lys
65                  70                  75                  80

Ser Pro Gly Arg Phe Glu Arg Ile Leu Leu Arg Ala Ala Asn Thr His
                85                  90                  95

Ser Ser Ala Lys Pro Cys Gly Gln Gln Ser Ile His Leu Gly Gly Val
            100                 105                 110

Phe Glu Leu Gln Pro Gly Ala Ser Val Phe Val Asn Val Thr Asp Pro
        115                 120                 125

Ser Gln Val Ser His Gly Thr Gly Phe Thr Ser Phe Gly Leu Leu Lys
    130                 135                 140

Leu
145

<210>  20
<211>  70
<212>  PRT
<213>  Homo sapiens

<400>  20

Ala Ser Asn Phe Asp Cys Cys Leu Gly Tyr Thr Asp Arg Ile Leu His
1               5                   10                  15

Pro Lys Phe Ile Val Gly Phe Thr Arg Gln Leu Ala Asn Glu Gly Cys
            20                  25                  30

Asp Ile Asn Ala Ile Ile Phe His Thr Lys Lys Lys Leu Ser Val Cys
        35                  40                  45

Ala Asn Pro Lys Gln Thr Trp Val Lys Tyr Ile Val Arg Leu Leu Ser

50                  55                  60

Lys Lys Val Lys Asn Met
65                  70

<210>  21
<211>  5006
<212>  DNA
<213>  Artificial Sequence

<220>
<223>  pTRIP vector

<400>  21
ctttaagacc aatgacttac aaggcagctg tagatcttag ccacttttta aaagaaaagg      60

ggggactgga agggctaatt cactcccaac gaagacaaga tcgtcgagag atgctgcata     120

taagcagctg ctttttgctt gtactgggtc tctctggtta gaccagatct gagcctggga     180

gctctctggc taactaggga acccactgct taagcctcaa taaagcttgc cttgagtgct     240

tcaagtagtg tgtgcccgtc tgttgtgtga ctctggtaac tagagatccc tcagaccctt     300

ttagtcagtg tggaaaatct ctagcagttc tagagggccc gtttaaaccc gctgatcagc     360

ctcgactgtg ccttctagtt gccagccatc tgttgtttgc ccctcccccg tgccttcctt     420

gaccctggaa ggtgccactc ccactgtcct ttcctaataa aatgaggaaa ttgcatcgca     480

ttgtctgagt aggtgtcatt ctattctggg gggtggggtg gggcaggaca gcaaggggga     540

ggattgggaa gacaatagca ggcatgctgg ggatgcggtg ggctctatgg cttctactgg     600

gcggttttat ggacagcaag cgaaccggaa ttgccagctg gggcgccctc tggtaaggtt     660

gggaagccct gcaaagtaaa ctggatggct ttctcgccgc caaggatctg atggcgcagg     720

ggatcaagct ctgatcaaga gacaggatga ggatcgtttc gcatgattga acaagatgga     780

ttgcacgcag gttctccggc cgcttgggtg gagaggctat tcggctatga ctgggcacaa     840

cagacaatcg gctgctctga tgccgccgtg ttccggctgt cagcgcaggg gcgcccggtt     900

ctttttgtca agaccgacct gtccggtgcc ctgaatgaac tgcaagacga ggcagcgcgg     960

ctatcgtggc tggccacgac gggcgttcct tgcgcagctg tgctcgacgt tgtcactgaa    1020

gcgggaaggg actggctgct attgggcgaa gtgccggggc aggatctcct gtcatctcac    1080

cttgctcctg ccgagaaagt atccatcatg gctgatgcaa tgcggcggct gcatacgctt    1140

gatccggcta cctgcccatt cgaccaccaa gcgaaacatc gcatcgagcg agcacgtact    1200

cggatggaag ccggtcttgt cgatcaggat gatctggacg aagagcatca ggggctcgcg    1260

ccagccgaac tgttcgccag gctcaaggcg agcatgcccg acggcgagga tctcgtcgtg    1320

acccatggcg atgcctgctt gccgaatatc atggtggaaa atggccgctt ttctggattc    1380

atcgactgtg gccggctggg tgtggcggac cgctatcagg acatagcgtt ggctacccgt    1440

gatattgctg aagagcttgg cggcgaatgg gctgaccgct tcctcgtgct ttacggtatc 1500

gccgctcccg attcgcagcg catcgccttc tatcgccttc ttgacgagtt cttctgaatt 1560

attaacgctt acaatttcct gatgcggtat tttctcctta cgcatctgtg cggtatttca 1620

caccgcatac aggtggcact tttcggggaa atgtgcgcgg aacccctatt tgtttatttt 1680

tctaaataca ttcaaatatg tatccgctca tgagacaata accctgataa atgcttcaat 1740

aatagcacgt gctaaaactt catttttaat ttaaaaggat ctaggtgaag atcctttttg 1800

ataatctcat gaccaaaatc ccttaacgtg agttttcgtt ccactgagcg tcagaccccg 1860

tagaaaagat caaaggatct tcttgagatc ctttttttct gcgcgtaatc tgctgcttgc 1920

aaacaaaaaa accaccgcta ccagcggtgg tttgtttgcc ggatcaagag ctaccaactc 1980

tttttccgaa ggtaactggc ttcagcagag cgcagatacc aaatactgtc cttctagtgt 2040

agccgtagtt aggccaccac ttcaagaact ctgtagcacc gcctacatac ctcgctctgc 2100

taatcctgtt accagtggct gctgccagtg gcgataagtc gtgtcttacc gggttggact 2160

caagacgata gttaccggat aaggcgcagc ggtcgggctg aacggggggt tcgtgcacac 2220

agcccagctt ggagcgaacg acctacaccg aactgagata cctacagcgt gagctatgag 2280

aaagcgccac gcttcccgaa gggagaaagg cggacaggta tccggtaagc ggcagggtcg 2340

gaacaggaga gcgcacgagg gagcttccag ggggaaacgc ctggtatctt tatagtcctg 2400

tcgggtttcg ccacctctga cttgagcgtc gatttttgtg atgctcgtca ggggggcgga 2460

gcctatggaa aaacgccagc aacgcggcct ttttacggtt cctggccttt tgctggcctt 2520

ttgctcacat gttcttgact cttcgcgatg tacgggccag atatacgcgc gcgttgggag 2580

ctttttgcaa aagcctaggc ctccaaaaaa gcctcctcac tacttctgga atagctcaga 2640

ggcagaggcg gcctcggcct ctgcataaat aaaaaaaatt agtcagccat ggggcggaga 2700

atgggcggaa ctgggcggag ttaggggcgg gatgggcgga gttaggggcg ggactatggt 2760

tgctgactaa ttgagatgcc cgacattgat tattgactag ttggaagggc taattcactc 2820

ccaacgaaga caagatatcc ttgatctgtg gatctaccac acacaaggct acttccctga 2880

ttagcagaac tacacaccag ggccagggat cagatatcca ctgacctttg gatggtgcta 2940

caagctagta ccagttgagc cagagaagtt agaagaagcc aacaaaggag agaacaccag 3000

cttgttacaa cctgtgagcc tgcatgggat ggatgacccg gagagagaag tgttagagtg 3060

gaggtttgac agccgcctag catttcatca cggtggcccg agagctgcat ccggagtact 3120

tcaagaactg ctgatatcga gcttgctaca agggactttc cgctggggga ctttccaggg 3180

aggcgtggcc tgggcgggac tggggagtgg cgagccctca gatcctgcat ataagcagct 3240

gctttttgcc tgtactgggt ctctctggtt agaccagatc tgagcctggg agctctctgg 3300

ctaactaggg aacccactgc ttaagcctca ataaagcttg ccttgagtgc ttcaagtagt 3360

gtgtgcccgt ctgttgtgtg actctggtaa ctagagatcc ctcagaccct tttagtcagt 3420

gtggaaaatc tctagcagtg gcgcccgaac agggacttga aagcgaaagg gaaaccagag 3480

gagctctctc gacgcaggac tcggcttgct gaagcgcgca cggcaagagg cgaggggcgg 3540

cgactggtga gtacgccaaa aattttgact agcggaggct agaaggagag agatgggtgc 3600

gagagcgtca gtattaagcg ggggagaatt agatcgcgat gggaaaaaat tcggttaagg 3660

ccagggggaa agaaaaaata taaattaaaa catatagtat gggcaagcag ggagctagaa 3720

cgattcgcag ttaatcctgg cctgttagaa acatcagaag gctgtagaca aatactggga 3780

cagctacaac catcccttca gacaggatca gaagaactta gatcattata taatacagta 3840

gcaaccctct attgtgtgca tcaaaggata gagataaaag acaccaagga agctttagac 3900

aagatagagg aagagcaaaa caaaagtaag accaccgcac agcaagcggc cgctgatctt 3960

cagacctgga ggaggagata tgagggacaa ttggagaagt gaattatata aatataaagt 4020

agtaaaaatt gaaccattag gagtagcacc caccaaggca aagagaagag tggtgcagag 4080

agaaaaaaga gcagtgggaa taggagcttt gttccttggg ttcttgggag cagcaggaag 4140

cactatgggc gcagcgtcaa tgacgctgac ggtacaggcc agacaattat tgtctggtat 4200

agtgcagcag cagaacaatt tgctgagggc tattgaggcg caacagcatc tgttgcaact 4260

cacagtctgg ggcatcaagc agctccaggc aagaatcctg gctgtggaaa gatacctaaa 4320

ggatcaacag ctcctgggga tttggggttg ctctggaaaa ctcatttgca ccactgctgt 4380

gccttggaat gctagttgga gtaataaatc tctggaacag atttggaatc acacgacctg 4440

gatggagtgg gacagagaaa ttaacaatta cacaagctta atacactcct taattgaaga 4500

atcgcaaaac cagcaagaaa agaatgaaca agaattattg gaattagata aatgggcaag 4560

tttgtggaat tggtttaaca taacaaattg gctgtggtat ataaaattat tcataatgat 4620

agtaggaggc ttggtaggtt taagaatagt ttttgctgta ctttctatag tgaatagagt 4680

taggcaggga tattcaccat tatcgtttca gacccacctc ccaaccccga ggggacccga 4740

caggcccgaa ggaatagaag aagaaggtgg agagagagac agagacagat ccattcgatt 4800

agtgaacgga tctcgacggt atcgccgaat tcacaaatgg cagtattcat ccacaatttt 4860

aaaagaaaag gggggattgg ggggtacagt gcaggggaaa gaatagtaga cataatagca 4920

acagacatac aaactaaaga attacaaaaa caaattacaa aaattcaaaa ttttcgggtt 4980

tattacaggg acagcagaga tccagg 5006

<210> 22
<211> 471
<212> DNA
<213> Artificial Sequence

<220>
<223>  BCuag promoter

<400>  22
gatctgttaa cttccccgaa gtagcaatgt atttcccaga aaaggaataa tccttctggg      60

aattctggcg aggtttccgg gaaagcagca ccgcccttgg ccgtcctgcc aatttcactt     120

tctagtttca ctttcccttt tgtaactaaa atgtaaatga cataggaaaa ctgaaaggga     180

gaagtgaaag tgggaaattc ctctgcatgc cttcttaaac atcacgagac tctaagaaaa     240

ggaaactgaa aacgggaaag tccctctctc taacctggca ctgcgtcgct ggcttggaga     300

caggtgacgg tccctgcggg ccttgtcctg attggctggg cacgcgttta atataagtgg     360

aggcgtcgcg ctggcgggca ttcctgaagc tgacagcatt cgggccgagt aacaactccg     420

ccccattgac gcaaatgggc ggtaggcgtg tacggtggga ggtctatata a              471


<210>  23
<211>  605
<212>  DNA
<213>  Woodchuck hepatitis virus

<400>  23
aattcccgat aatcaacctc tggattacaa aatttgtgaa agattgactg gtattcttaa      60

ctatgttgct ccttttacgc tatgtggata cgctgcttta atgcctttgt atcatgctat     120

tgcttcccgt atggctttca ttttctcctc cttgtataaa tcctggttgc tgtctcttta     180

tgaggagttg tggcccgttg tcaggcaacg tggcgtggtg tgcactgtgt ttgctgacgc     240

aacccccact ggttggggca ttgccaccac ctgtcagctc ctttccggga ctttcgcttt     300

ccccctccct attgccacgg cggaactcat cgccgcctgc cttgcccgct gctggacagg     360

ggctcggctg ttgggcactg acaattccgt ggtgttgtcg gggaagctga cgtcctttcc     420

gcggctgctc gcctgtgttg ccacctggat tctgcgcggg acgtccttct gctacgtccc     480

ttcggccctc aatccagcgg accttccttc ccgcggcctg ctgccggctc tgcggcctct     540

tccgcgtctt cgccttcgcc ctcagacgag tcggatctcc ctttgggccg cctccccgca     600

tcggg                                                                 605


<210>  24
<211>  422
<212>  PRT
<213>  Artificial Sequence

<220>
<223>  hSPD40-ESXH

<400>  24

Met Leu Leu Phe Leu Leu Ser Ala Leu Val Leu Leu Thr Gln Pro Leu
1               5                   10                  15

Gly Tyr Leu Glu Ala Glu Met Lys Thr Tyr Ser His Arg Thr Met Pro
            20                  25                  30

Ser Ala Cys Thr Leu Val Met Cys Ser Ser Val Glu Ser Gly Leu Pro
        35                  40                  45

Gly Arg Asp Gly Arg Asp Gly Arg Glu Gly Pro Arg Gly Glu Lys Gly
    50                  55                  60

Asp Pro Gly Leu Pro Gly Ala Ala Gly Gln Ala Gly Met Pro Gly Gln
65                  70                  75                  80

Ala Gly Pro Val Gly Pro Lys Gly Asp Asn Gly Ser Val Gly Glu Pro
                85                  90                  95

Gly Pro Lys Gly Asp Thr Gly Pro Ser Gly Gly Ser Gly Ser Gln Ile
            100                 105                 110

Met Tyr Asn Tyr Pro Ala Met Leu Gly His Ala Gly Asp Met Ala Gly
        115                 120                 125

Tyr Ala Gly Thr Leu Gln Ser Leu Gly Ala Glu Ile Ala Val Glu Gln
    130                 135                 140

Ala Ala Leu Gln Ser Ala Trp Gln Gly Asp Thr Gly Ile Thr Tyr Gln
145                 150                 155                 160

Ala Trp Gln Ala Gln Trp Asn Gln Ala Met Glu Asp Leu Val Arg Ala
                165                 170                 175

Tyr His Ala Met Ser Ser Thr His Glu Ala Asn Thr Met Ala Met Met
            180                 185                 190

Ala Arg Asp Thr Ala Glu Ala Ala Lys Trp Gly Gly Gly Ser Gly Gly
        195                 200                 205

Leu Arg Gly Leu Gln Gly Pro Pro Gly Lys Leu Gly Pro Pro Gly Asn
    210                 215                 220

Pro Gly Pro Ser Gly Ser Pro Gly Pro Lys Gly Gln Lys Gly Asp Pro
225                 230                 235                 240

Gly Lys Ser Pro Asp Gly Asp Ser Ser Leu Ala Ala Ser Glu Arg Lys
                245                 250                 255

Ala Leu Gln Thr Glu Met Ala Arg Ile Lys Lys Trp Leu Thr Phe Ser

260                 265                 270

Leu Gly Gly Ser Gly Asp Gln Asn Pro Gln Ile Ala Ala His Val Ile
        275                 280                 285

Ser Glu Ala Ser Ser Lys Thr Thr Ser Val Leu Gln Trp Ala Glu Lys
    290                 295                 300

Gly Tyr Tyr Thr Met Ser Asn Asn Leu Val Thr Leu Glu Asn Gly Lys
305                 310                 315                 320

Gln Leu Thr Val Lys Arg Gln Gly Leu Tyr Tyr Ile Tyr Ala Gln Val
                325                 330                 335

Thr Phe Cys Ser Asn Arg Glu Ala Ser Ser Gln Ala Pro Phe Ile Ala
            340                 345                 350

Ser Leu Cys Leu Lys Ser Pro Gly Arg Phe Glu Arg Ile Leu Leu Arg
        355                 360                 365

Ala Ala Asn Thr His Ser Ser Ala Lys Pro Cys Gly Gln Gln Ser Ile
    370                 375                 380

His Leu Gly Gly Val Phe Glu Leu Gln Pro Gly Ala Ser Val Phe Val
385                 390                 395                 400

Asn Val Thr Asp Pro Ser Gln Val Ser His Gly Thr Gly Phe Thr Ser
                405                 410                 415

Phe Gly Leu Leu Lys Leu
            420

## Description

### Field of the invention

The invention relates to lentiviral vectors designed to provide a new generation of multifunctional vectors leveraged to target and activate Dendritic Cells, to route immunogens to MHC-II pathway, and to induce both CD4⁺ and CD8⁺ T-cell responses.

In particular the invention relates to such lentiviral vectors expressing antigen(s) selected for their interest in eliciting an immunological response in a host, in particular a human host in need thereof wherein the immunological response encompassing a CD4+ T cell response. The antigens may be expressed from an insert in the lentiviral backbone of the vector consisting of a polynucleotide encoding a single antigen or from a polynucleotide encoding multiple antigens. The polynucleotide encoding the single or multiple antigens is provided in the vector by means of a scaffold carrier.

The lentiviral vector of the invention is provided for use in the design of immunological compositions, preferably of a vaccine candidate, in particular a vaccine suitable for a human host.

### Background of the invention

Lentiviral Vectors (LV) provide one of the most efficient vaccine platforms, relied on their outstanding potential of gene transfer to the nuclei of the host cells, including notably Antigen Presenting Cells (APC). Such nuclear transfer of genes initiates expression of antigens which readily access the Major Histocompatibility Complex Class-I (MHC-I) presentation machinery, i.e., proteasome, for further triggering of CD8⁺ T cells¹⁻³. In net contrast with their substantial ability at routing the endogenously produced antigens into the MHC-I pathway, viral vectors, including LV, are barely effective or inoperative in delivery of non-secreted antigens to the endosomal MHC-II compartment (MIIC) and unable to trigger CD4⁺ T cells. Although CD8⁺ T cells contribute largely to the immune control of infectious diseases or tumor growth, CD4⁺ T cells are the major immune players. In addition to their long lifespan and their own direct effector functions, CD4⁺ T cells orchestrate the immune system by regulating innate immunity, tailoring B-cell responses and supporting CD8⁺ T-cell effector functions⁴. Therefore, leveraging the potential of LV to induce CD4⁺ T cells will maximize their success rate in vaccine strategies.

### Summary of the invention

In one aspect, the present invention relates to a recombinant lentiviral vector genome comprising a polynucleotide encoding a fusion polypeptide, wherein said fusion polypeptide comprises arranged from N-terminal to C-terminal ends a first recombinant polypeptide and a second polypeptide, wherein:
(i) said first recombinant polypeptide comprises a multimerization scaffold which comprises at least one collectin or a fragment thereof suitable to enable self-assembly of multimers of the first polypeptide, fused with at least one antigenic polypeptide;
(ii) said second polypeptide comprises a CD40L ectodomain or a receptor binding fragment thereof, in particular the CD40L ectodomain of the human CD40L.

The present invention further relates to a DNA plasmid comprising the recombinant vector genome according to the invention.

The present invention also relates to a recombinant lentiviral vector or a recombinant lentiviral vector particle which comprises the recombinant lentiviral vector genome according to the invention.

The present invention also relates to a fusion polypeptide comprising arranged from N-terminal to C-terminal ends a first recombinant polypeptide and a second polypeptide, wherein:
(i) said first recombinant polypeptide comprises a multimerization scaffold which comprises at least one collectin or a fragment thereof suitable to enable self-assembly of multimers of the first polypeptide, fused with at least one antigenic polypeptide;
(ii) said second polypeptide comprises a CD40L ectodomain or a receptor binding fragment thereof, in particular the CD40L ectodomain of the human CD40L.

The invention further relates to a host cell, preferably a mammalian host cell, in particular a human host, transfected with a DNA plasmid according to the invention, in particular wherein said host cell is a HEK-293T cell line or a K562 cell line.

In another aspect, the invention relates to a pharmaceutical composition, in particular a vaccine composition, suitable for administration to a mammalian host, in particular a human host, comprising a recombinant lentiviral vector of the invention, a recombinant lentiviral vector particle of the invention, or a host cell of the invention together with one or more pharmaceutically acceptable excipient(s) suitable for administration to a host in need thereof, in particular a human host.

In particular, the invention relates to the pharmaceutical composition for use in the elicitation of a protective, preferentially prophylactic, immune response by the elicitation of T-cell responses directed against epitopes contained in the antigenic polypeptide or immunogenic fragments thereof, and/or cellular and/or humoral response in a host in need thereof, in particular a human host.

Another aspect of the invention relates to a method for the preparation of recombinant lentiviral vector particles suitable for the preparation of a pharmaceutical composition, in particular a vaccine, comprising the following steps:
a) transfecting the recombinant lentiviral transfer vector carrying the lentiviral vector genome according to the invention, or the DNA plasmid according to the invention in a host cell, for example a HEK-293T cell line or a K562 cell line;
b) co-transfecting the cell of step a) with: (i) a plasmid vector encoding envelope proteins and with a plasmid vector encoding the lentiviral GAG and POL or mutated POL protein as packaging construct; and (ii) a plasmid encoding VSV-G Indiana or New Jersey envelope ;
c) culturing the host cell under conditions suitable for the production of recombinant lentiviral vector particles expressing the antigenic polypeptide, or an immunogenic fragment thereof;
d) recovering the recombinant lentiviral particles expressing the antigenic polypeptide, or an immunogenic fragment thereof.

### Detailed description of the invention

The inventors have designed and prepared a platform of lentiviral vector encoding a recombinant secreted protein monomer based on soluble collagen-containing C-type lectins (collectins), such as Mannan-Binding Lectin (MBL) or Surfactant-associated Protein D (SPD), as scaffold protein carriers. The inventors have discovered that these scaffold protein carriers, when fused with the CD40L ectodomain, could elicit an MHC-II antigen presentation, and a strong CD4-mediated immune response. This was unexpected, since the T-cell immunogenicity of the existing lentiviral platforms were mostly restricted to a CD8⁺ T-cell-mediated immune response.
The invention hence discloses a recombinant lentiviral vector genome comprising a polynucleotide encoding a fusion polypeptide expressed as a recombinant carrier protein.
The recombinant carrier protein is encoded by a polynucleotide that is recombined in the backbone of the lentiviral transfer vector in order to enable preparing lentiviral vector particles expressing the protein carrier harboring the antigen(s) for elicitation of an immunological response.

The recombinant protein carrier expressed by the lentiviral vector of the invention is obtained as a fusion polypeptide or as a multimer of such fusion polypeptide carrying single or multiple distinct antigens suitable for the elicitation of an immunogenic response, in particular a protective immunogenic response or advantageously a sterile protection against the pathogen providing the antigen(s).

In one aspect, the invention thus relates to a recombinant lentiviral vector genome comprising a polynucleotide encoding a fusion polypeptide, comprising, arranged from N-terminal to C-terminal ends a first recombinant polypeptide and a second polypeptide, wherein:
(i) said first recombinant polypeptide comprises a multimerization scaffold which comprises at least one collectin or a fragment thereof suitable to enable self-assembly of multimers of the first polypeptide, fused with at least one antigenic polypeptide;
(ii) said second polypeptide comprises a CD40L ectodomain or a receptor binding fragment thereof.

As used herein, a multimerization scaffold refers to a polypeptide chain that is capable of self-assembling, thereby inducing the formation of multimeric proteins.
According to the invention, when a polypeptide is fused with another polypeptide, the nucleotide sequences encoding the two polypeptides are ligated to each other in-frame to create a chimeric gene encoding a fusion protein. In the invention, the nucleotide sequence of the antigenic polypeptide may be ligated in 5' or 3' position with respect to the nucleotide sequence of the collectin or fragment thereof. Preferably, it is inserted, i.e. fused in-frame within the nucleotide sequence of the collectin or fragment thereof, such that the collectin or its fragment harbors the antigenic polypeptide within its polypeptide chain. Preferably, the antigenic polypeptide is harbored within the collagen-like domain of the collectin. The fusion between two polypeptides may be direct or indirect. In particular, a linker or spacer peptide or polypeptide chain may be present between the two fused polypeptides.
According to the invention, the second polypeptide comprises CD40 ligand (CD40L) or a receptor binding fragment thereof capable of binding to the CD40 receptor when contained in the fusion polypeptide of the invention, in particular a CD40L ectodomain or a receptor binding fragment thereof. CD40L is preferably human CD40L.
The CD40L ectodomain is preferably the ectodomain of human CD40L. In particular, the CD40L ectodomain has the sequence set forth in SEQ ID No. 19. The second polypeptide may also comprise any receptor binding fragment or region of CD40L or its ectodomain, i.e. a fragment or region capable of binding CD40.
In this fusion protein, the collectin or its fragment acts as a carrier for the antigenic polypeptide. The collectin also acts as a multimerization scaffold which promotes the formation of collectin-CD40L multimers, which are competent to bind CD40 receptor at the surface of the Antigen Presenting Cells (APCs) which will become activated.

The invention also relates to the fusion polypeptide, in a monomer or multimer form. Preferably, the collectin or its fragment is able to induce a trimerization of the fusion polypeptide. Collectin trimers are also able to further multimerize, inducing the formation of dodecamers (tetramers of trimers) or octodecamers (hexamers of trimers). The fusion polypeptide expressed as a multimer is in particular a soluble macromolecule carrier that is able to circulate in the blood of the host or able to be taken up by APCs.

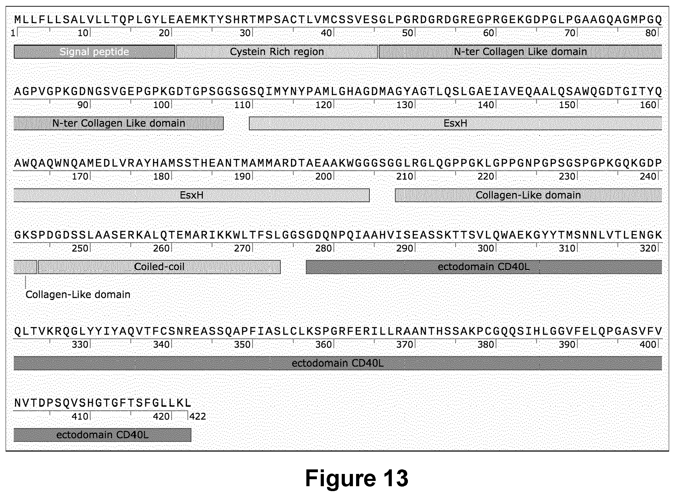

Collectins are typically composed of four distinct regions: (i) a crosslinking region, (ii) a collagen-like region, (iii) a neck region, and (iv) a Carbohydrate-Recognition Domain (CRD). Preferably, the collectin or collectin fragment comprises the following regions of a collectin, from N-terminal to C-terminal ends: at least one crosslinking region; at least one collagen-like region of a collectin; and at least one neck region of a collectin. In one embodiment, the carbohydrate recognition domain (CRD) of the collectin is absent from the fusion protein. In this embodiment, the second polypeptide comprising the ectodomain of CD40L or a receptor binding fragment thereof, may replace the CRD of the collectin, i.e. the second polypeptide may be fused to the neck region of the collectin, optionally with a linker or a spacer. In particular, the CD40L ectodomain or its fragment may be fused with the neck region via a rigid spacer such as a coiled coil region which prevents interaction between CD40L ectodomain and the antigenic polypeptide to be expressed.
The collectin may be selected from mannan-binding lectin (MBL), surfactant protein D (SP-D), surfactant protein A (SP-A), collectin liver 1 (CL-L1), collectin placenta 1 (CL-P1), conglutinin, collectin of 43 kDa (CL-43), collectin of 46 kDa (CL-46), and collectin kidney 1 (CL-K1). Preferably, the collectin is a human collectin selected from human MBL (UniProtKB - P11226), SP-D (UniProtKB - P35247), CL-L1 (UniProtKB - Q9Y6Z7), SP-A1 (UniProtKB - Q8IWL2), SP-A2 (UniProtKB - Q8IWL1), CL-P1 (UniProtKB - Q5KU26) and CL-K1 (UniProtKB - Q9BWP8). Preferably, the collectin is MBL or SP-D, especially human MBL or human SP-D, such as human MBL of SEQ ID no. 17 or human SP-D of SEQ ID No. 18.
In one embodiment, the collectin or collectin fragment in the fusion polypeptide of the invention is a chimeric collectin or a fragment thereof. A chimeric collectin comprises two or more collectin fragments originating from different collectins. In one embodiment, the chimeric collectin comprises a fragment of human Pulmonary surfactant-associated protein D (SP-D, UniProtKB - P35247) in particular amino acids 1-106 comprising a signal peptide ensuring secretion, a cysteine-rich region and a collagen-like domain, and a fragment of human Mannose-binding protein C (MBL, UniProtKB - P11226), in particular amino acids 65-130 comprising a collagen-like domain and a coiled-coil region. The antigenic polypeptide is preferably fused to the chimeric collectin between the SP-D fragment and the MBL fragment. An exemplary structure of a fusion protein comprising a chimeric cellectin is shown in Figure 13.

Preferably, the polynucleotide(s) encoding the antigenic polypeptide(s) is or are inserted within the nucleotide sequence of the collagen-like region of said collectin, in particular it is fused in frame within this sequence.
The antigenic polypeptide may be linked within the collagen-like region of said collectin via linkers, in particular flexible peptide linkers, such as "GS" linkers made of stretches of glycine and serine, in particular the (Gly-Gly-Gly-Gly-Ser)ₙ linker. Suitable linkers are also shown in the Examples, in particular in Tables S2 and S3.

According to the invention the fusion polypeptide carries one or several antigens or antigenic polypeptides. In a particular embodiment the fusion polypeptide provides at least 2, in particular at least 3 or at least 4 or at least 5 and in particular are especially 2, 3, 4 or 5, and accordingly encompass at least 2, at least 3 or at least 4 antigens (and/or antigenic fragments or mutated antigens with respect to a native or wild type determined antigen of a pathogen). In a particular embodiment the antigenic polypeptide contained in the fusion polypeptide comprises or consists of a fusion of up to 6 antigens or antigenic fragments or mutated fragments thereof.
In one embodiment, antigen or immunogenic fragment thereof is selected from a bacterial, parasite or viral pathogen, or is a tumoral antigen or immunogenic fragment thereof, in particular wherein the at least two antigens or immunogenic fragments thereof are selected from distinct pathogens. In one embodiment, the pathogen is selected from *Mycobacterium tuberculosis* (Mtb), an influenza virus in particular a type A, type B or type C influenza virus, more specifically an H1N1, H2N2 or H3N2 influenza virus, or a coronavirus, in particular SARS-CoV-2.
In particular, the antigenic polypeptide may comprise one or more *Mycobacterium tuberculosis* (Mtb) antigens, in particular selected from EsxA (UniProtKB - P9WNK7), EspC (UniProtKB - P9WJD7), EsxH (UniProtKB - P9WNK3), PE19 (UniProtKB - Q79FK4), Hypoxic response protein 1 (Hrp1) (UniProtKB - P9WJA3) and Resuscitation promoting factor D (RpfD) (UniProtKB - P9WG27), or immunogenic fragments thereof, e.g. a fragment lacking the initial methionine. Preferably, the immunogenic fragment of RpfD is the RpfD₄₂₋₁₅₄ ectodomain. In one embodiment, the antigenic polypeptide may comprise one of the following Mtb antigenic combinations:
(a) EsxH;
(b) EsxH and EsxA;
(c) EsxH, EsxA and PE19;
(d) EsxH, EsxA, PE19 and EspC;
(e) EsxH, EsxA, PE19, EspC, Hrp1 and RpfD;
or immunogenic fragments thereof.

In one embodiment, the fusion polypeptide further comprises polypeptides, in particular via 2A self-cleaving peptides, ensuring other immuno-modulatory functions like that of CXCL9, CXCL10, CCL3, CCL4 and/or CCL5 as pro-inflammatory Th1-related chemokines, or CXCL20 as Th17-promoting chemokine.
In one embodiment, the fusion polypeptide further comprises a polypeptide comprising CCL20, or a receptor binding domain thereof. CCL20, or the receptor binding domain thereof, may be in particular inserted within the collagen-like domain of the collectin.
In one embodiment, the polypeptide comprises human CCL20 or a receptor binding domain thereof. In particular, the polypeptide comprises the human CCL20 sequence set forth in SEQ ID No. 20.
In one embodiment, the fusion polypeptide has the sequence set forth in SEQ ID NO: 24 and in Figure 13, wherein the sequence of the antigenic polypeptide EsxH may be replaced by another antigenic polypeptide of interest.
The invention accordingly also relates to a nucleic acid molecule encoding the fusion polypeptide defined herein. The nucleic acid may be DNA, in particular cDNA or may be RNA, in particular stabilized RNA. The RNA sequences are deducted from the DNA sequences wherein the Thymine (T) nucleobase is replaced by an Uracile (U) nucleobase. RNA polynucleotides may be obtained by transcription of DNA or cDNA or may be synthesized.
The nucleic acid molecule may further comprise control nucleotide sequences for the transcription or for the expression of the fusion polypeptide comprising the antigen(s). It may also be modified, in order to be operably ligated to a distinct polynucleotide such as a plasmid or a vector genome (transfer plasmid), in particular a lentiviral vector genome. It may also be modified, in particular to be rendered more stable such as for use as RNA. In a further embodiment, the nucleic acid is a mammalian codon-optimized, in particular a human codon-optimized sequence for expression in mammalian, respectively human cells.

The invention also relates to a plasmid vector recombined with a nucleic acid molecule encoding the fusion polypeptide carrying antigen(s) selected for the elicitation of an immune response in a host.

In a further embodiment, the plasmid vector is a transfer vector in particular a lentiviral transfer vector suitable to provide the genome of a lentiviral vector of the invention. The lentiviral vector expresses the selected antigenic polypeptides with their carrier protein as a secreted monomer or multimer when expressed *in vivo* in a host.

An "antigen" or an "antigenic polypeptide" as defined herein as a wild type or native antigen of a pathogenic organism or as a fragment of such wild type a native antigen or as a mutated polypeptide comprising less than 5% of mutated especially substituted amino acid residues with respect to the wild type or native antigen. Mutations are in particular point mutations of 1, 2, 3 or 4 amino acid residues of the amino acid sequence of the wild type or native antigen. A fragment of the wild type or the native antigen advantageously keeps the immunogenic properties of the polypeptide from which it derives or shows improved immunogenic properties when it is expressed by the lentiviral vector of the invention and advantageously shows immune protective properties when expressed in a host. A fragment of an antigen has an amino acid sequence which is sufficient to provide one or several epitope(s) in particular T cell epitopes and more particularly CD4+ or CD8+ T cell epitopes or both and which keeps the immunogenic, especially the protective properties leading to the protective activity of the antigenic polypeptide from which it derives and/or exhibits such protective properties when expressed by the lentiviral vector of the invention.
The expression *"T-cell epitope"* refers to antigenic determinants that are involved in the adaptive immune response driven by T cells. In particular said T-cell epitopes elicit T cells, when delivered to the host in suitable conditions. According to a particular embodiment the antigenic polypeptides targeted according to the invention and the polypeptide derivatives of these antigenic polypeptides comprise epitope(s) mediating CD4+ T cell response and advantageously also CD8⁺ T cell response.
Polypeptides and antigens described and used in the invention may have at least 50% amino acid identity with the native protein, in particular at least 60%, in particular at least 70%, in particular at least 80%, more particularly at least 90 or 95%, more particularly at least 99% identity.

In a particular embodiment, the nucleic acid molecule containing the genome of the transfer vector is provided as a plasmid comprising the lentiviral backbone vector recombined with a polynucleotide encoding the selected antigen(s) of the pathogen, for their expression as a monomeric fusion polypeptide or as a multimeric, preferably soluble carrier protein when said vector genome is provided in a lentiviral vector particle that is used for administration to a host.

Additionally, the nucleic acid molecule may contain sequences for the control of transcription and/or for the control of expression, and/or may contain sequences for ligation to a distinct nucleic acid such as for ligation to a plasmid or a vector genome. Hence the nucleic acid may contain one or more of sequences for restriction site(s), Kozak sequence, promoter or other sequences as disclosed herein and illustrated in the examples.

The expression "vector" relates to biological or chemical entities suitable for the delivery of the polynucleotides encoding the antigenic polypeptides of the combination of compounds to the cells of the host administered with such vectors. Vectors are well known in the art and may be viral vectors as those described herein such as lentiviruses which infect human. The invention relates in particular to the use of HIV vectors, especially HIV-1 vectors which are illustrated in the Examples. Details for the construction for HIV-1 vectors are known in the art and provided in the examples.

In accordance with the invention, lentiviral vectors expressing antigenic polypeptides are provided wherein the vectors have or comprise in their genome (vector genome) a recombinant polynucleotide which encodes a fusion polypeptide according to the invention, wherein said fusion polypeptide comprises at least one antigenic polypeptide, in particular of a pathogen.

The lentiviral vectors of the invention, especially the preferred HIV-1 based vectors, replication-incompetent pseudotyped lentiviral vector, in particular a replication-incompetent pseudotyped HIV-1 lentiviral vector, wherein said vector contains a genome comprising a mammal codon-optimized synthetic nucleic acid, in particular a human-codon optimized synthetic nucleic acid, wherein said synthetic nucleic acid encodes a fusion polypeptide according to the invention, comprising an antigenic polypeptide, in particular the antigenic polypeptide(s) of a determined pathogen infecting a mammal, in particular a human host.

Use of codon-optimized sequences in the genome of the vector particles allows in particular strong expression of the antigenic polypeptide in the cells of the host administered with the vector, especially by improving mRNA stability or reducing secondary structures. In addition, the expressed antigenic polypeptide undergoes post translational modifications which are suitable for processing of the antigenic polypeptide in the cells of the host, in particular by modifying translation modification sites (such as glycosylation sites) in the encoded polypeptide. Codon optimization tools are well known in the art, including algorithms and services such as those made available by GeneArt (Life technologies-USA) and DNA2.0 (Menlo Park, California - USA). In a particular embodiment codon-optimization is carried out on the open reading frame (ORF) sequence encoding the antigenic polypeptide and the optimization is carried out prior to the introduction of the sequence encoding the ORF into the plasmid intended for the preparation of the vector genome. In another embodiment additional sequences of the vector genome are also codon-optimized.

The active ingredients consisting of the viral vectors may be integrative pseudotyped lentiviral vectors, especially replication-incompetent integrative pseudotyped lentiviral vectors, in particular a HIV-1 vector. Such lentiviral vectors may in addition contain a genome comprising a mammal-codon optimized synthetic nucleic acid, in particular a human-codon optimized synthetic nucleic acid, wherein said synthetic nucleic acid encodes the antigenic polypeptide(s) of a determined pathogen infecting a mammal such as disclosed herein, in particular a virus or a bacteria or a parasite infecting a human host.

Alternatively, the lentiviral vector and in particular the HIV-1 based vector may be a non-integrative replication-incompetent pseudotyped lentiviral vector.

A particular embodiment of a lentiviral vector suitable to achieve the invention relates to a lentiviral vector whose genome is obtained from the pTRIP vector plasmid, in particular the pTRIP vector plasmid of nucleotide sequence SEQ ID No. 21, wherein the nucleic acid encoding the fusion polypeptide has been cloned under control of a promoter functional in mammalian cells, in particular the CMV promoter, the human beta-2 microglobulin promoter, the composite "BCUAG" promoter as disclosed herein (SEQ ID No. 22) and wherein the vector optionally comprises post-transcriptional regulatory element of the woodchuck hepatitis virus (WPRE), wild type or mutated. In particular, the WPRE is a mutant WPRE as set forth in SEQ ID No. 23.

In a further embodiment of the invention, the lentiviral vector particle expressing the fusion polypeptide according to the features herein described is pseudotyped with the glycoprotein G from a Vesicular Stomatitis Virus (V-SVG) of Indiana or of New-Jersey serotype.
The particular features of such lentiviral vectors will be further discussed in detail below.
The invention also relates to a DNA plasmid comprising the recombinant lentiviral vector genome according to the definitions provided herein, in particular wherein said genome is inserted within the pTRIP vector plasmid of nucleotide sequence SEQ ID No. 21.

The invention further relates to a host cell, preferably a mammalian host cell, transfected with a DNA plasmid according to the invention. In particular, said host cell is a HEK-293T cell line or a K562 cell line. The invention further relates to a culture of said host cells.

The invention also relates to a formulation or pharmaceutical composition, in particular a vaccine composition, suitable for administration to a mammalian host, comprising a recombinant lentiviral vector of the invention together with one or more pharmaceutically acceptable excipient(s) suitable for administration to a host in need thereof, in particular a human host.
The invention also relates to a formulation suitable for administration to a mammalian host, in particular a human host comprising as an active ingredient lentiviral vector particles as defined herein for protection against a pathogen infection or against the pathogen-induced condition or disease, together with excipient(s) suitable for administration to a host in need thereof, in particular a human host. The disease may be tuberculosis, influenza, in particular caused by a type A, type B or type C influenza virus, more specifically an H1N1, H2N2 or H3N2 influenza virus. The disease may also be a coronavirus disease, in particular caused by SARS-CoV-2.

In another aspect of the invention the active ingredient or the composition or the formulation comprising the same is for use in the protective immunization against a pathogenic infection or against pathogen-induced condition or disease, in a mammalian host, especially a human host, optionally in association with an appropriate delivery vehicle and optionally with an adjuvant component and/or with an immunostimulant component.

Accordingly, the active ingredient, or the composition, in particular the lentiviral vector particles of the invention, when administered to a host in needs thereof, especially to a mammalian in particular to a human host, elicits an immune response, encompassing activation of naive lymphocytes and generation of effector T-cell response and generation of immune memory antigen-specific T-cell response against antigen(s) of the pathogen.
The immune response involves the induction of MHC-II restricted presentation of the antigenic polypeptide or immunogenic fragments thereof, by an antigen-presenting cell, in particular a dendritic cell, and the induction of a CD4+-T-cell immune response. The immune response may either prevent the infection by the pathogen or may prevent the onset or the development of a pathological state resulting from infection.

Physiologically acceptable vehicles may be chosen with respect to the administration route of the immunization composition. In a preferred embodiment administration may be carried out by injection, in particular intramuscularly, intradermally, subcutaneously, or, by intranasal administration or topical skin application.

Recombinant lentiviral vector particles of the invention are used for elicitation in a host, in particular a human host, of an immune response against the pathogen providing the antigens expressed by the particles, said use involving an immunization pattern comprising administering an effective amount of the LV vector particles that elicits the cellular immune response of the host, especially as an effective amount of said active ingredients to boost the cellular immune response of the host previously elicited by a priming dose of a selected active ingredient against said pathogen, and optionally repeating (once or several times) said administration step for boosting.

For each step of administration of the lentiviral vector particles, in particular in a regimen that encompasses multiple administration steps, it is preferred that the pseudotyping envelope protein(s) of the vector particles is(are) different from the one used in the other step(s), especially originate from different viruses, in particular different VSVs. In the prime-boost regimen, the administered combination of compounds of each step comprises lentiviral vectors as defined herein.

Priming and boosting steps are separated in time by at least 2 weeks, in particular 6 weeks, in particular by at least 8 weeks.
Details on the administration regimen will be discussed further below.

The LV particles provides a cellular immune response (T-cell immune response), particularly a CD4+T-cell immune response and advantageously a CD8+- T-cell immune response, i.e., an adaptive immune response which is mediated by activated cells harbouring respectively CD4 or CD8 receptors.

In a particularly advantageous embodiment, the immune response conferred by the LV particles, is a long-lasting immune response i.e., said immune response encompasses memory cells response and in particular central memory cells response; in a particular embodiment it can be still detected at least several months.

In accordance with the invention when the lentiviral particles are used in a prime-boost regimen or a multiple steps administration regimen, lentiviral vector particles are provided which are pseudotyped with a first determined pseudotyping envelope G protein obtained from the VSV, strain Indiana or New-Jersey, and later administered lentiviral vector particles are provided which are pseudotyped with a second determined pseudotyping envelope G protein obtained from a VSV, strain New Jersey or Indiana. The order of use in the prime-boost regimen of the first and second compounds thus described may alternatively be inversed. Thus, the lentiviral vector particles contained in the separate active ingredients/compounds of the combinations or compositions of the invention when intended for use in a prime-boost regiment are distinct from each other, at least due to the particular pseudotyping envelope protein(s) used for pseudotyping the vector particles.

Doses of lentiviral vectors intended for elicitation of the cellular immune response which is used in the administration pattern, may comprise from 10⁵ TU to 10¹⁰ TU of recombinant lentiviral particles especially from 10⁵ to 10⁸, when integrative vectors are used. The dose intended for administration to the host may comprise from 10⁸ to 10¹⁰ of each type of recombinant lentiviral vector particles when integrative-incompetent vectors are used.

The invention also concerns a method of providing immunization in a mammalian host, especially in a human host, comprising the step of administering, as a prime or as a boost, the recombinant lentiviral vector particles of the invention to elicit the immune response, and optionally repeating the administration steps one or several times, in particular to boost said response, in accordance with the present disclosure.

Optionally, the recombinant lentiviral vector particles may be used in association with an adjuvant compound suitable for administration to a mammalian, especially a human host, and/or with an immunostimulant compound, together with an appropriate delivery vehicle. In a preferred embodiment, the recombinant lentiviral vector particles of the invention may also be used without adjuvant, since the CD40L ectodomain already acts as a slight adjuvant.

The recombinant lentiviral vector particles can be administered to the host via injection through different routes including subcutaneous (s.c.), intradermal (i.d.), intramuscular (i.m.) or intravenous (i.v.) injection, or may be administered orally to topically trough mucosal or skin administration, especially intranasal administration or inhalation. The quantity to be administered (dosage) depends on the subject to be treated, including considering the condition of the patient, the state of the individual's immune system, the route of administration and the size of the host. Suitable dosages range may be determined with respect to the content in equivalent transducing units of HIV-1-derived lentiviral vector particles.

Other examples and features of the invention will be apparent when reading the examples and the figures which illustrate the preparation and application of the lentiviral vector particles with features that may be individually combined with the definitions given in the present description.

### Detailed description of the lentiviral vectors for use according to the invention

The invention accordingly involves lentiviral vector which are recombinant lentiviral particles (i.e. recombinant vector particles), and which may be replication-incompetent lentiviral vectors, especially replication-incompetent HIV-1 based vectors characterized in that: (i) they are pseudotyped with a determined heterologous viral envelope protein or viral envelope proteins originating from a RNA virus which is not HIV, and (ii) they comprise in their genome at least one recombinant polynucleotide encoding at least one antigenic polypeptide (or polypeptide derivative thereof) carrying epitope(s) of an antigen of a pathogen wherein the pathogen is capable of infecting a mammalian host, and wherein said epitopes encompass T-cell epitope(s), in particular both CD4+ T-cell epitopes and CD8+ T-cell epitopes.

According to a particular embodiment of the invention, the lentiviral vectors are either designed to express proficient (i.e., integrative-competent) or deficient (i.e., integrative-incompetent) particles. According to a particular embodiment of the invention, the recombinant lentiviral vector particles are both integration-incompetent and replication-incompetent.
The preparation of the lentiviral vectors is well known from the skilled person and has been extensively disclosed in the literature (confer for review Sakuma T. et al (Biochem. J. (2012) 443, 603-618). The preparation of such vectors is also illustrated herein in the Examples.
In a particular embodiment of the invention, the polynucleotide(s) encoding the antigenic polypeptides (ORF) of the lentiviral vector has(have) been mammal-codon optimized (CO) in particular human-codon optimized. Optionally the lentiviral sequences of the genome of said particles have also a mammal-codon optimized nucleotide sequence. In a particular aspect of the invention the codon optimization has been carried out for expression in mouse cells. In another embodiment the sequence of the polynucleotide(s) encoding the antigenic polypeptides of the lentiviral vector has(have) been human-codon optimized (CO).

It has been observed that codon optimized nucleotide sequences, especially when optimized for expression in mammalian and in particular in human cells, enable the production of higher yield of particles in such mammalian or human cells. Production cells are illustrated in the examples. Accordingly, when lentiviral vector particles of the invention are administered to a mammalian, especially to a human host, higher amounts of particles are produced in said host which favour the elicitation of a strong immune response.
The recombinant lentiviral vector (i.e., lentiviral vectors particles or lentiviral-based vector particles) defined in the present invention are pseudotyped lentiviral vectors consisting of vector particles bearing envelope protein or envelope proteins which originate from a virus different from the particular lentivirus (especially a virus different from HIV, in particular HIV-1), which provides the vector genome of the lentiviral vector particles. Accordingly, said envelope protein or envelope proteins, are "heterologous" viral envelope protein or viral envelope proteins with respect to the vector genome of the particles. In the following pages, reference will also be made to "envelope protein(s)" to encompass any type of envelope protein or envelope proteins suitable to perform the invention.
When reference is made to "lentiviral" vectors (lentiviral-based vectors) in the application, it relates in particular, to HIV-based vectors and especially HIV-1-based vectors.
The lentiviral vectors suitable to perform the invention are so-called replacement vectors, meaning that the sequences of the original lentivirus encoding the lentiviral proteins are essentially deleted in the genome of the vector or, when present, are modified, and especially mutated, especially truncated, to prevent expression of biologically active lentiviral proteins, in particular, in the case of HIV, to prevent the expression by said transfer vector providing the genome of the recombinant lentiviral vector particles, of functional ENV, GAG, and POL proteins and optionally of further structural and/or accessory and/or regulatory proteins of the lentivirus, especially of HIV.
In a particular embodiment, the lentiviral vector is built from a first-generation vector, in particular a first-generation of a HIV-based vector which is characterized in that it is obtained using separate plasmids to provide (i) the packaging construct, (ii) the envelope and (iii) the transfer vector genome. Alternatively, it may be built from a second-generation vector, in particular a second-generation of a HIV-based vector which in addition, is devoid of viral accessory proteins (such as in the case of HIV-1, Vif, Vpu, Vpr or Nef) and therefore includes only four out of nine HIV full genes: *gag, pol, tat* and *rev.* In another embodiment, the vector is built from a third-generation vector, in particular a third-generation of a HIV-based vector which is furthermore devoid of said viral accessory proteins and also is Tat-independent; these third-generation vectors may be obtained using 4 plasmids to provide the functional elements of the vector, including one plasmid encoding the Rev protein of HIV when the vector is based on HIV-1. Such vector system comprises only three of the nine genes of HIV-1. The structure and design of such generations of HIV-based vectors is well known in the art.

In any of these generations of the vector, modifications are additionally provided according to the invention by insertion in the vector backbone of the collectin scaffold as described herein, fused with the ectodomain of CD40L or a receptor binding fragment thereof, to provide a LV vector leveraged to target and activate APC, in particular dendritic cells to route immunogens to MHC-II pathway and to induce both CD4+ and CD8+ T-cell responses.

The "vector genome" of the vector particles is a recombinant nucleic acid which also comprises as a recombined sequence the polynucleotide or transgene of interest encoding the fusion polypeptide according to the invention comprising one or more antigenic polypeptide(s), in particular of pathogen as disclosed herein. The lentiviral-based sequence and polynucleotide/transgene of the vector genome are borne by a plasmid vector thus giving rise to the "transfer vector" also referred to as "sequence vector". Accordingly, these expressions are used interchangeably in the present description. According to a particular embodiment, a vector genome prepared for the invention comprises a nucleic acid having a sequence selected in the group of SEQ ID No. 21, in which the polynucleotide encoding the fusion polypeptide of the invention is inserted.

The vector genome as defined herein accordingly contains, apart from the so-called recombinant polynucleotide(s) encoding the fusion polypeptide of the invention comprising the antigenic polypeptide(s) placed under control of proper regulatory sequences for its expression, the sequences of the original lentiviral genome which are non-coding regions of said genome, and are necessary to provide recognition signals for DNA or RNA synthesis and processing (mini-viral genome). These sequences are cis-acting sequences necessary for packaging (ψ), reverse transcription (LTRs possibly mutated with respect to the original ones) and transcription and optionally integration (RRE) and furthermore for the particular purpose of the invention, they contain a functional sequence favouring nuclear import in cells and accordingly transgene transfer efficiency in said cells, which element is described as a DNA Flap element that contains or consists of the so-called central cPPT-CTS nucleotidic domain present in lentiviral genome sequences especially in HIV-1 or in some retroelements such as those of yeasts.
The structure and composition of the vector genome used to prepare the lentiviral vectors of the invention are based on the principles described in the art and on examples of such lentiviral vectors primarily disclosed in (Zennou et al, 2000; Firat H. et al, 2002; VandenDriessche T. et al). Constructs of this type have been deposited at the CNCM (Institut Pasteur, France) as will be referred to herein. In this respect reference is also made to the disclosure, including to the deposited biological material, in patent applications WO 99/55892, WO 01/27300 and WO 01/27304.
According to a particular embodiment of the invention, a vector genome may be a replacement vector in which all the viral protein coding sequences between the 2 long terminal repeats (LTRs) have been replaced by the recombinant polynucleotide encoding the fusion polypeptide of the invention comprising the antigenic polypeptide(s) as disclosed herein, and wherein the DNA-Flap element has been reinserted in association with the required cis-acting sequences described herein. Further features relating to the composition of the vector genome are disclosed in relation to the preparation of the particles.

In a particular embodiment, a lentiviral vector of the invention may comprise in its genome one or more than one recombinant polynucleotide encoding a fusion polypeptide according to the invention. In particular, said vector genome comprises two polynucleotides which are consecutive or separated on the genome and which encode different polypeptides of either the same or distinct antigens of the pathogen or of distinct pathogens.

Particular features of the lentiviral vectors used in accordance with the various embodiments of the invention are also disclosed in the Examples, such features being either taken alone or in combination to produce the vectors.
According to the invention, the lentiviral vector particles are pseudotyped with a heterologous viral envelope protein or viral polyprotein of envelope originating from an RNA virus which is not the lentivirus providing the lentiviral sequences of the genome of the lentiviral particles.
As examples of typing envelope proteins for the preparation of the lentiviral vector, the invention relates to viral transmembrane glycosylated (so-called G proteins) envelope protein(s) of a Vesicular Stomatitis Virus (VSV), which is(are) for example chosen in the group of VSV-G protein(s) of the Indiana strain and VSV-G protein(s) of the New Jersey strain.
Other examples of VSV-G proteins that may be used to pseudotype the lentiviral vectors of the invention encompass VSV-G glycoprotein may especially be chosen among species classified in the vesiculovirus genus: Carajas virus (CJSV), Chandipura virus (CHPV), Cocal virus (COCV), Isfahan virus (ISFV), Maraba virus (MARAV), Piry virus (PIRYV), Vesicular stomatitis Alagoas virus (VSAV), Vesicular stomatitis Indiana virus (VSIV) and Vesicular stomatitis New Jersey virus (VSNJV) and/or stains provisionally classified in the vesiculovirus genus as Grass carp rhabdovirus, BeAn 157575 virus (BeAn 157575), Boteke virus (BTKV), Calchaqui virus (CQIV), Eel virus American (EVA), Gray Lodge virus (GLOV), Jurona virus (JURV), Klamath virus (KLAV), Kwatta virus (KWAV), La Joya virus (LJV), Malpais Spring virus (MSPV), Mount Elgon bat virus (MEBV), Perinet virus (PERV), Pike fry rhabdovirus (PFRV), Porton virus (PORV), Radi virus (RADIV), Spring viremia of carp virus (SVCV), Tupaia virus (TUPV), Ulcerative disease rhabdovirus (UDRV) and Yug Bogdanovac virus (YBV).
The envelope glycoprotein of the vesicular stomatitis virus (VSV-G) is a transmembrane protein that functions as the surface coat of the wild type viral particles. It is also a suitable coat protein for engineered lentiviral vectors. Presently, nine virus species are definitively classified in the VSV gender, and nineteen rhabdoviruses are provisionally classified in this gender, all showing various degrees of cross-neutralisation. When sequenced, the protein G genes indicate sequence similarities. The VSV-G protein presents an N-terminal ectodomain, a transmembrane region and a C-terminal cytoplasmic tail. It is exported to the cell surface *via* the trans-Golgi network (endoplasmic reticulum and Golgi apparatus).
Vesicular stomatitis Indiana virus (VSIV) and Vesicular stomatitis New Jersey virus (VSNJV) are preferred strains to pseudotype the lentiviral vectors of the invention, or to design recombinant envelope protein(s) to pseudotype the lentiviral vectors. Their VSV-G proteins are disclosed in GenBank, where several strains are presented. For VSV-G New Jersey strain reference is especially made to the sequence having accession number V01214. For VSV-G of the Indiana strain, reference is made to the sequence having accession number AAA48370.1 in Genbank corresponding to strain JO2428.
Said viral envelope protein(s) are capable of uptake by antigen presenting cells and especially by dendritic cells including by liver dendritic cells by mean of fusion and/or of endocytosis. In a particular embodiment, the efficiency of the uptake may be used as a feature to choose the envelope of a VSV for pseudotyping. In this respect the relative titer of transduction (Titer DC/Titer of other transduced cells e.g. 293T cells) may be considered as a test and envelope having a relatively good ability to fuse with DC would be preferred.
Antigen Presenting Cells (APC) and especially Dentritic cells (DC) are proper target cells for pseudotyped lentiviral vectors which are used as immune compositions accordingly.
The VSV-G envelope protein(s) are expressed from a polynucleotide containing the coding sequence for said protein(s), which polynucleotide is inserted in a plasmid (designated envelope expression plasmid or pseudotyping env plasmid) used for the preparation of the lentiviral vector particles of the invention. The polynucleotide encoding the envelope protein(s) is under the control of regulatory sequences for the transcription and/or expression of the coding sequence (including optionally post-transcriptional regulatory elements (PRE) especially a polynucleotide such as the element of the Woodchuck hepatitis virus, i.e. the WPRE sequence, obtainable from Invitrogen or a mutant sequence of WPRE as set forth in SEQ ID No. 23.
Accordingly, a nucleic acid construct is provided which comprises an internal promoter suitable for the use in mammalian cells, especially in human cells *in vivo* and the nucleic acid encoding the envelope protein under the control of said promoter. A plasmid containing this construct is used for transfection of cells suitable for the preparation of vector particles. Promoters may in particular be selected for their properties as constitutive promoters, tissue-specific promoters, or inducible promoters. Examples of suitable promoters encompass the promoters of the following genes: MHC Class-I promoters, human beta-2 microglobulin gene (β2M promoter), EF1α, human PGK, PPI (preproinsulin), thiodextrin, HLA DR invariant chain (P33), HLA DR alpha chain, Ferritin L chain or Ferritin H chain, Chymosin beta 4, Chymosin beta 10, Cystatin Ribosomal Protein L41, CMVie or chimeric promoters such as GAG(CMV early enhancer / chicken β actin) disclosed in Jones S. et al (Jones S. et al Human Gene Therapy, 20:630-640(June 2009)) or beta-2m-CMV (BCUAG) as disclosed herein.
These promoters may also be used in regulatory expression sequences involved in the expression of *gag-pol* derived proteins from the encapsidation plasm ids, and/or to express the antigenic polypeptides from the transfer vector.
Alternatively, when the envelope expression plasmid is intended for expression in stable packaging cell lines, especially for stable expression as continuously expressed viral particles, the internal promoter to express the envelope protein(s) is advantageously an inducible promoter such as one disclosed in Cockrell A.S. et al. (Mol. Biotechnol. (2007) 36:184-204). As examples of such promoters, reference is made to tetracycline and ecdysone inducible promoters. The packaging cell line may be the STAR packaging cell line (ref Cockrell A.S. et al (2007), Ikedia Y. et al (2003) Nature Biotechnol. 21: 569-572) or a SODk packaging cell line, such as SODk0 derived cell lines, including SODk1 and SODk3 (ref Cockrell A.S. et al (2007), Cockrell A;S.et al (2006) Molecular Therapy, 14: 276-284, Xu K. et al. (2001) ,Kafri T. et al (1999) Journal of Virol. 73:576-584).
According to the invention, the lentiviral vectors are the product recovered from co-transfection of mammalian cells, with:
- a vector plasmid comprising (i) lentiviral, especially HIV-1, cis-active sequences necessary for packaging, reverse transcription, and transcription and further comprising a functional lentiviral, especially derived from HIV-1, DNA flap element and (ii) a polynucleotide encoding the fusion polypeptide of the invention, itself comprising one or more antigenic polypeptide(s) of one or more pathogens against which an immune response is sought under the control of regulatory expression sequences, and optionally comprising sequences for integration into the genome of the host cell;
- an expression plasmid encoding a pseudotyping envelope derived from an RNA virus, said expression plasmid comprising a polynucleotide encoding an envelope protein or proteins for pseudotyping, wherein said envelope pseudotyping protein is advantageously from a VSV and is in particular a VSV-G of the Indianan strain or of the New Jersey strain and,
- an encapsidation plasmid, which either comprises lentiviral, especially HIV-1, *gag-pol* packaging sequences suitable for the production of integration-competent vector particles or modified *gag-pol* packaging sequences suitable for the production of integration-deficient vector particles.
The invention thus also concerns lentiviral vector particles as described above, which are the product recovered from a stable cell line transfected with:
- a vector plasmid comprising (i) lentiviral, especially HIV-1, cis-active sequences necessary for packaging, reverse transcription, and transcription and further comprising a functional lentiviral, especially HIV-1, DNA flap element and optionally comprising cis-active sequences necessary for integration, said vector plasmid further comprising, (ii) a polynucleotide of a codon-optimized sequence for murine or for human of the gene encoding the fusion polypeptide of the invention, comprising one or more antigenic polypeptide(s) of one or more pathogens as disclosed herein, under the control of regulatory expression sequences, especially a promoter;
- a VSV-G envelope expression plasmid comprising a polynucleotide encoding a VSV-G envelope protein in particular VSV-G of the Indiana strain or of the New Jersey strain, wherein said polynucleotide is under the control of regulating expression sequences, in particular regulatory expression sequences comprising a promoter, and;
- an encapsidation plasmid, wherein the encapsidation plasmid either comprises lentiviral, especially HIV-1, *gag-pol* coding sequences suitable for the production of integration-competent vector particles or modified *gag-pol* coding sequences suitable for the production of integration-deficient vector particles, wherein said *gag-pol* sequences are from the same lentivirus sub-family as the DNA flap element, wherein said lentiviral *gag-pol* or modified *gag-pol* sequence is under the control of regulating expression sequences.
The stable cell lines expressing the vector particles of the invention are in particular obtained by transduction of the plasmids.

The polynucleotide encodes the fusion polypeptide according to the invention, which comprises at least one antigenic polypeptide of a pathogen according to any embodiment disclosed in the present specification. In particular, it encodes a polypeptide which is a truncated mammalian, especially human, codon-optimized sequence coding for such antigenic polypeptide of a pathogen.
Accordingly, the vector plasmid may comprise one or several expression cassettes for the expression of the various antigenic polypeptides or may comprise bi-cistronic or multi-cistronic expression cassettes where the polynucleotides encoding the fusion polypeptide comprising the antigenic polypeptide(s) and optionally additional various polypeptides are separated by an IRES sequence of viral origin (Internal Ribosome Entry Site), or it may encode fusion protein(s).
The internal promoter contained in the vector genome and controlling the expression of the polynucleotide encoding an antigenic polypeptide of the pathogen (as a transgene or in an expression cassette) may be selected from the promoters of the following genes: MHC Class I promoters, such as human beta-2 microglobulin gene (β2M promoter), or EF1α, human PGK, PPI (preproinsulin), thiodextrin, HLA DR invariant chain (P33), HLA DR alpha chain, Ferritin L chain or Ferritin H chain, Chymosin beta 4, Chimosin beta 10, or Cystatin Ribosomal Protein L41 CMVie or chimeric promoters such as GAG(CMV early enhancer / chicken β actin) disclosed in Jones S. et al (2009) or BCUAG.
A promoter among the above-cited internal promoters may also be selected for the expression of the envelope protein(s) and packaging (*gag-pol* derived) proteins.

The following particular embodiments may be carried out when preparing the lentiviral vector based on human lentivirus, and especially based on HIV-1 virus.
According to the invention, the genome of the lentiviral vector is derived from a human lentivirus, especially from the HIV lentivirus. In particular, the pseudotyped lentiviral vector is an HIV-based vector, such as an HIV-1, or HIV-2 based vector, in particular is derived from HIV-1M, for example from the BRU or LAI isolates. Alternatively, the lentiviral vector providing the necessary sequences for the vector genome may be originating from lentiviruses such as EIAV, CAEV, VISNA, FIV, BIV, SIV, HIV-2, HIV-O which are capable of transducing mammalian cells.
As stated above, when considering it apart from the recombinant polynucleotide that it finally contains, the vector genome is a replacement vector in which the nucleic acid between the 2 long terminal repeats (LTRs) in the original lentivirus genome have been restricted to cis-acting sequences for DNA or RNA synthesis and processing, including for the efficient delivery of the transgene to the nuclear of cells in the host, or at least are deleted or mutated for essential nucleic acid segments that would enable the expression of lentiviral structure proteins including biological functional GAG polyprotein and possibly POL and ENV proteins.
In a particular embodiment, the 5' LTR and 3' LTR sequences of the lentivirus are used in the vector genome, but the 3'-LTR at least is modified with respect to the 3'LTR of the original lentivirus at least in the U3 region which for example can be deleted or partially deleted for the enhancer (delta U3). The 5'LTR may also be modified, especially in its promoter region where for example a Tat-independent promoter may be substituted for the U3 endogenous promoter.
In a particular embodiment the vector genome comprises one or several of the coding sequences for Vif-, Vpr, Vpu- and Nef-accessory genes (for HIV-1 lentiviral vectors). Alternatively, these sequences can be deleted independently or each other or can be non-functional (second-generation lentiviral vector).
The vector genome of the lentiviral vector particles comprises, as an inserted cis-acting fragment, at least one polynucleotide consisting in the DNA flap element or containing such DNA flap element. In a particular embodiment, the DNA flap is inserted upstream of the polynucleotide encoding the fusion polypeptide of the invention carrying the antigenic polypeptide(s) and is advantageously - although not necessarily - located in an approximate central position in the vector genome. A DNA flap suitable for the invention may be obtained from a retrovirus, especially from a lentivirus, in particular a human lentivirus especially a HIV-1 retrovirus, or from a retrovirus-like organism such as retrotransposon. It may be alternatively obtained from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. The DNA flap may be either prepared synthetically (chemical synthesis) or by amplification of the DNA providing the DNA Flap from the appropriate source as defined above such as by Polymerase chain reaction (PCR). In a more preferred embodiment, the DNA flap is obtained from an HIV retrovirus, for example HIV-1 or HIV-2 virus including any isolate of these two types.

The DNA flap (also designated cPPT/CTS) (defined in Zennou V. et al. ref 27, 2000, Cell vol 101, 173-185 or in WO 99/55892 and WO 01/27304), is a structure which is central in the genome of some lentiviruses especially in HIV, where it gives rise to a 3-stranded DNA structure normally synthesized during especially HIV reverse transcription and which acts as a cis-determinant of HIV genome nuclear import. The DNA flap enables a central strand displacement event controlled in *cis* by the central polypurine tract (cPPT) and the central termination sequence (CTS) during reverse transcription. When inserted in lentiviral-derived vectors, the polynucleotide enabling the DNA flap to be produced during reverse-transcription, stimulates gene transfer efficiency and complements the level of nuclear import to wild-type levels (Zennou et al., Cell, 2000 Cell vol 101, 173-185 or in WO 99/55892 and WO 01/27304).
Sequences of DNA flaps have been disclosed in the prior art, especially in the above cited patent applications. These sequences are also disclosed in the sequence of the pTRIP vector herein described. They are preferably inserted as a fragment, optionally with additional flanking sequences, in the vector genome, in a position which is preferably near the centre of said vector genome. Alternatively they may be inserted immediately upstream from the promoter controlling the expression of the polynucleotide(s) encoding the fusion polypeptide of the invention. Said fragments comprising the DNA flap, inserted in the vector genome may have a sequence of about 80 to about 200 bp, depending on its origin and preparation.
According to a particular embodiment, a DNA flap has a nucleotide sequence of about 90 to about 140 nucleotides.
In HIV-1, the DNA flap is a stable 99-nucleotide-long plus strand overlap. When used in the genome vector of the lentiviral vector of the invention, it may be inserted as a longer sequence, especially when it is prepared as a PCR fragment. A particular appropriate polynucleotide comprising the structure providing the DNA flap is a 124-base pair polymerase chain reaction (PCR) fragment encompassing the cPPT and CTS regions of the HIV-1 DNA.
It is specified that the DNA flap used in the genome vector and the polynucleotides of the encapsidation plasmid encoding the GAG and POL polyproteins should originate from the same lentivirus sub-family or from the same retrovirus-like organism.
Preferably, the other cis-activating sequences of the genome vector also originate from the same lentivirus or retrovirus-like organism, as the one providing the DNA flap.

The vector genome may further comprise one or several unique restriction site(s) for cloning the recombinant polynucleotide.
In a preferred embodiment, in said vector genome, the 3' LTR sequence of the lentiviral vector genome is devoid of at least the activator (enhancer) and possibly the promoter of the U3 region. In another particular embodiment, the 3' LTR region is devoid of the U3 region (delta U3). In this respect, reference is made to the description in WO 01/27300 and WO 01/27304.
In a particular embodiment, in the vector genome, the U3 region of the LTR 5' is replaced by a non lentiviral U3 or by a promoter suitable to drive tat-independent primary transcription. In such a case, the vector is independent of *tat* transactivator (third generation vector).
The vector genome also comprises the psi (ψ) packaging signal. The packaging signal is derived from the N-terminal fragment of the *gag* ORF. In a particular embodiment, its sequence could be modified by frameshift mutation(s) in order to prevent any interference of a possible transcription/translation of gag peptide, with that of the transgene.
The vector genome may optionally also comprise elements selected among a splice donor site (SD), a splice acceptor site (SA) and/or a Rev-responsive element (RRE). According to a particular embodiment, the vector plasmid (or added genome vector) comprises the following cis-acting sequences for a transgenic expression cassette:
1. The LTR sequence (Long-Terminal Repeat), required for reverse transcription, the sequences required for transcription and including optionally sequences for viral DNA integration. The 3' LTR is deleted in the U3 region at least for the promoter to provide SIN vectors (Self-inactivating), without perturbing the functions necessary for gene transfer, for two major reasons: first, to avoid trans-activation of a host gene, once the DNA is integrated in the genome and secondly to allow self-inactivation of the viral *cis*-sequences after retrotranscription. Optionally, the tat-dependent U3 sequence from the 5'-LTR which drives transcription of the genome is replaced by a non endogenous promoter sequence. Thus, in target cells only sequences from the internal promoter will be transcribed (transgene).
2. The ψ region, necessary for viral RNA encapsidation.
3. The RRE sequence (REV Responsive Element) allowing export of viral messenger RNA from the nucleus to the cytosol after binding of the *Rev* protein.
4. The DNA flap element (cPPT/CTS) to facilitate nuclear import.
5. Optionally post-transcriptional regulatory elements, especially elements that improve the expression of fusion polypeptide and/or antigenic polypeptide in dendritic cells, such as the WPRE cis-active sequence (Woodchuck hepatitis B virus Post-Responsive Element) also added to optimize stability of mRNA (Zufferey et al., 1999), the matrix or scaffold attachment regions (SAR and MAR sequences) such as those of the immunoglobulin-kappa gene (Park F. et al Mol Ther 2001; 4: 164-173).
The lentiviral vector of the invention is non replicative (replication-incompetent) *i.e.,* the vector and lentiviral vector genome are regarded as suitable to alleviate concerns regarding replication competent lentiviruses and especially are not able to form new particles budding from the infected host cell after administration. This may be achieved in well-known ways as the result of the absence in the lentiviral genome of the *gag, pol* or *env* genes, or their absence as "functional genes". The *gag* and *pol* genes are thus, only provided in *trans.* This can also be achieved by deleting other viral coding sequence(s) and/or cis-acting genetic elements needed for particles formation.
By *"functional"* it is meant a gene that is correctly transcribed, and/or correctly expressed. Thus, if present in the lentiviral vector genome of the invention in this embodiment contains sequences of the *gag, pol,* or *env* are individually either not transcribed or incompletely transcribed; the expression *"incompletely transcribed"* refers to the alteration in the transcripts gag, gag-pro or gag-pro-pol, one of these or several of these being not transcribed. Other sequences involved in lentiviral replication may also be mutated in the vector genome, in order to achieve this status. The absence of replication of the lentiviral vector should be distinguished from the replication of the lentiviral genome. Indeed, as described before, the lentiviral genome may contain an origin of replication ensuring the replication of the lentiviral vector genome without ensuring necessarily the replication of the vector particles.
In order to obtain lentiviral vectors according to the invention, the vector genome (as a vector plasmid) must be encapsidated in particles or pseudo-particles. Accordingly, lentiviral proteins, except the envelope proteins, have to be provided *in trans* to the vector genome in the producing system, especially in producing cells, together with the vector genome, having recourse to at least one encapsidation plasmid carrying the *gag* gene and either the *pol* lentiviral gene or an integrative-incompetent *pol* gene, and preferably lacking some or all of the coding sequences for *Vif-, Vpr, Vpu-* and *Nef-*accessory genes and optionally lacking *Tat* (for HIV-1 lentiviral vectors).
A further plasmid is used, which carries a polynucleotide encoding the envelope pseudotyping protein(s) selected for pseudotyping lentiviral vector particles.
In a preferred embodiment, the packaging plasmid encodes only the lentiviral proteins essential for viral particle synthesis. Accessory genes whose presence in the plasmid could raise safety concerns are accordingly removed. Accordingly, viral proteins brought in *trans* for packaging are respectively as illustrated for those originating from HIV-1:
1. GAG proteins for building of the matrix (MA, with apparent Molecular Weight p17), the capsid (CA, p24) and nucleocapsid (NC, p6).
2. POL encoded enzymes: integrase, protease and reverse transcriptase.
3. TAT and REV regulatory proteins, when TAT is necessary for the initiation of LTR-mediated transcription; TAT expression may be omitted if the U3 region of 5'LTR is substituted for a promoter driving tat-independent transcription. REV may be modified and accordingly used for example in a recombinant protein which would enable recognition of a domain replacing the RRE sequence in the vector genome, or used as a fragment enabling binding to the RRE sequence through its RBD (RNA Binding Domain).
In order to avoid any packaging of the mRNA generated from the genes contained in the packaging plasmid in the viral particles, the ψ region is removed from the packaging plasmid. A heterologous promoter is inserted in the plasmid to avoid recombination issues and a poly-A tail is added 3' from the sequences encoding the proteins. Appropriate promoters have been disclosed above.
The envelope plasmid encodes the envelope protein(s) for pseudotyping which are disclosed herein, under the control of an internal promoter, as disclosed herein.
Any or all the described plasm ids for the preparation of the lentiviral vector particles of the invention may be codon optimized (CO) in the segment encoding proteins. Codon optimization according to the invention is preferably performed to improve translation of the coding sequences contained in the plasmids, in mammalian cells, murine or especially human cells. According to the invention, codon optimization is especially suited to directly or indirectly improve the preparation of the vector particles or to improve their uptake by the cells of the host to whom they are administered, or to improve the efficiency of the transfer of the polynucleotide encoding the fusion polypeptide comprising the antigenic polypeptide (transgene) in the genome of the transduced cells of the host. Methods for optimizing codons are well known in the art and codon optimization is especially performed using available programs to that effect. Codon optimization is illustrated for the coding sequences used in the examples.
In a particular embodiment of the invention, the pseudotyped lentiviral vector is also, or alternatively, integrative-competent, thus enabling the integration of the vector genome and of the recombinant polynucleotide which it contains into the genome of the transduced cells or in the cells of the host to whom it has been administered.
In another particular embodiment of the invention, the pseudotyped lentiviral vector is also, or alternatively, integrative-incompetent. In such a case, the vector genome and thus the recombinant polynucleotide which it contains do not integrate into the genome of the transduced cells or in the cells of the host to whom it has been administered.
The present invention relates to the use of a lentiviral vector wherein the expressed integrase protein is defective and which further comprises a polynucleotide especially encoding the fusion polypeptide of the invention, in particular comprising at least one antigenic polypeptide carrying epitope(s) of a pathogen, in an immunogenic composition.
By "integration-incompetent", it is meant that the integrase, preferably of lentiviral origin, is devoid of the capacity of integration of the lentiviral genome into the genome of the host cells i.e., an integrase protein mutated to specifically alter its integrase activity.
Integration-incompetent lentiviral vectors are obtained by modifying the *pol* gene encoding the Integrase, resulting in a mutated *pol* gene encoding an integrative deficient integrase, said modified *pol* gene being contained in the encapsidation plasmid. Such integration-incompetent lentiviral vectors have been described in patent application WO 2006/010834. Accordingly the integrase capacity of the protein is altered whereas the correct expression from the encapsidation plasmid of the GAG, PRO and POL proteins and/or the formation of the capsid and hence of the vector particles, as well as other steps of the viral cycle, preceding or subsequent to the integration step, such as the reverse transcription, the nuclear import, stay intact. An integrase is said defective when the integration that it should enable is altered in a way that an integration step takes place less than 1 over 1000, preferably less than 1 over 10000, when compared to a lentiviral vector containing a corresponding wild-type integrase.
In a particular embodiment of the invention, the defective integrase results from a mutation of class 1, preferably amino acid substitutions (one-amino acid substitution) or short deletions fulfilling the requirements of the expression of a defective integrase. The mutation is carried out within the *pol* gene. These vectors may carry a defective integrase with the mutation D64V in the catalytic domain of the enzyme, which specifically blocks the DNA cleaving and joining reactions of the integration step. The D64V mutation decreases integration of pseudotyped HIV-1 up to 1/10,000 of wild type, but keep their ability to transduce non dividing cells, allowing efficient transgene expression.
Other mutations in the *pol* gene which are suitable to affect the integrase capacity of the integrase of HIV-1 are the following: H12N, H12C, H16C, H16V, S81 R, D41A, K42A, H51A, Q53C, D55V, D64E, D64V, E69A, K71A, E85A, E87A, D116N, D116I, D116A, N120G, N120I, N120E, E152G, E152A, D-35-E, K156E, K156A, E157A, K159E, K159A, K160A, R166A, D167A, E170A, H171A, K173A, K186Q, K186T, K188T, E198A, R199C, R199T, R199A, D202A, K211A, Q214L, Q216L, Q221 L, W235F, W235E, K236S, K236A, K246A, G247W, D253A, R262A, R263A and K264H. In a particular embodiment, mutation in the *pol* gene is performed at either of the following positions D64, D116 or E152, or at several of these positions which are in the catalytic site of the protein. Any substitution at these positions is suitable, including those described above.
Another proposed substitution is the replacement of the amino acid residues RRK (positions 262 to 264) by the amino acids residues AAH.
In a particular embodiment of the invention, when the lentiviral vector is integration-incompetent, the lentiviral genome further comprises an origin of replication (ori), whose sequence is dependent on the nature of cells where the lentiviral genome has to be expressed. Said origin of replication may be from eukaryotic origin, preferably of mammalian origin, most preferably of human origin. It may alternatively be of viral origin, especially coming from DNA circular episomic viruses, such as SV40 or RPS. It is an advantageous embodiment of the invention to have an origin or replication inserted in the lentiviral genome of the lentiviral vector of the invention. Indeed, when the lentiviral genome does not integrate into the cell host genome (because of the defective integrase), the lentiviral genome is lost in cells that undergo frequent cell divisions; this is particularly the case in immune cells, such as B or T cells. The presence of an origin of replication ensures that at least one lentiviral genome is present in each cell, even after cell division, accordingly maximizing the efficiency of the immune response.
The lentiviral vector genome of said lentiviral vectors of the invention may especially be derived from HIV-1 plasmid pTRIPΔU3.CMV-GFP deposited at the CNCM (Paris, France) on October 11, 1999 under number I-2330 (also described in WO01/27300) or variants thereof.
When the vector genome is derived from these particular plasmids, a sequence of a recombinant polynucleotide encoding the fusion polypeptide of the invention, in particular comprising an antigenic polypeptide of a pathogen as disclosed in the present application, is inserted therein, in addition or in replacement of the GFP coding fragment. The CMV promoter may also be substituted by another promoter, especially one of the promoters disclosed above, especially in relation to the expression of the transgene.
The WPRE sequence also contained in the particular deposited pTRIP vectors may optionally be deleted.
Vector particles may be produced after transfection of appropriate cells (such as mammalian cells or human cells, such as Human Embryonic Kidney cells illustrated by 293 T cells) by said plasmids, or by other processes. In the cells used for the expression of the lentiviral particles, all or some of the plasmids may be used to stably express their coding polynucleotides, or to transiently or semi-stably express their coding polynucleotides.
The concentration of particles produced can be determined by measuring the P24 (capsid protein for HIV-1) content of cell supernatants.
The lentiviral vector of the invention, once administered into the host, infects cells of the host, possibly specific cells, depending on the envelope proteins it was pseudotyped with. The infection leads to the release of the lentiviral vector genome into the cytoplasm of the host cell where the retro-transcription takes place. Once under a triplex form (via the DNA flap), the lentiviral vector genome is imported into the nucleus, where the polynucleotide(s) encoding polypeptide(s) of antigen(s) of the pathogen is (are) expressed via the cellular machinery. When non-dividing cells are transduced (such as DC), the expression may be stable. When dividing cells are transduced, such as B cells, the expression is temporary in absence of origin of replication in the lentiviral genome, because of nucleic acid dilution and cell division. The expression may be longer by providing an origin of replication ensuring a proper diffusion of the lentiviral vector genome into daughter cells after cell division. The stability and/or expression may also be increased by insertion of MAR (Matrix Associated Region) or SAR (Scaffold Associated Region) elements in the vector genome.
Indeed, these SAR or MAR regions are AT-rich sequences and enable to anchor the lentiviral genome to the matrix of the cell chromosome, thus regulating the transcription of the polynucleotide encoding the fusion polypeptide of the invention comprising at least one antigenic polypeptide, and particularly stimulating gene expression of the transgene and improving chromatin accessibility.
If the lentiviral genome is non integrative, it does not integrate into the host cell genome. Nevertheless, the at least one polypeptide encoded by the transgene is sufficiently expressed and longer enough to be processed, associated with MHC molecules and finally directed towards the cell surface. Depending on the nature of the polynucleotide(s) encoding antigenic polypeptide(s) of a pathogen, the at least one polypeptide epitope associated with the MHC molecule triggers a cellular immune response.
Unless otherwise stated, or unless technically not relevant, the characteristics disclosed in the present application with respect to any of the various features, embodiments or examples of the structure or use of the lentiviral particles, especially regarding their envelope protein(s), or the recombinant polynucleotide, may be combined according to any possible combinations.
The invention further relates to a combination of compounds for separate administration to a mammalian host, which comprises at least:
(i) lentiviral vector particles of the invention which are pseudotyped with a first determined heterologous viral envelope pseudotyping protein or viral envelope pseudotyping proteins; such first pseudotyping protein may be from the New-Jersey strain of VSV;
(ii) provided separately from lentiviral vector particles in (i), lentiviral vector particles of the invention which are pseudotyped with a second determined heterologous viral envelope pseudotyping protein or viral envelope pseudotyping proteins distinct from said first heterologous viral envelope pseudotyping protein(s); such second pseudotyping protein may be from the Indiana strain of VSV.

In another embodiment of the invention, possibly in combination with the above disclosed alternative forms of the nucleic acid, the polynucleotide encoding the fusion polypeptide of the invention, comprising at least one antigenic polypeptide is structurally modified and/or chemically modified. Illustrative thereof a polynucleotide comprises a Kozak consensus sequence in its 5' region. Other nucleic acid sequences that are not of lentiviral origin may be present in the vector genome are IRES sequence(s) (Internal Ribosome entry site) suitable to initiate polypeptide synthesis WPRE sequence as post-transcriptional regulatory element to stabilize the produced RNA.
According to another embodiment of the invention, if multiple heterologous polypeptides are encoded by one vector genome, the coding sequences may optionally be separated by a linker moiety which is either a nucleic acid-based molecule or a non-nucleic acid-based molecule. Such a molecule may be a functionalized linker molecule aimed at recognizing a 3' functionalized nucleic acid to which it shall be linked. A sequence suitable to function as a linker may alternatively be a nucleic acid which encodes a self-cleaving peptide, such as a 2A peptide.

Further features and properties of the present invention, including features to be used in the embodiments described above will be described in the examples and figures which follow and may accordingly be used to characterise the invention.

### SEQUENCE LISTING

SEQ ID No. 1: M40-H amino acid sequence
SEQ ID No. 2: M40-H DNA sequence
SEQ ID No. 3: M40-HA amino acid sequence
SEQ ID No. 4: M40-HA DNA sequence
SEQ ID No. 5: M40-HAP amino acid sequence
SEQ ID No. 6: M40-HAP DNA sequence
SEQ ID No. 7: M40-HAPE amino acid sequence
SEQ ID No. 8: M40-HAPE DNA sequence
SEQ ID No. 9: S40-H amino acid sequence
SEQ ID No. 10: S40-H DNA sequence
SEQ ID No. 11: S40-HAPE amino acid sequence
SEQ ID No. 12: S40-HAPE DNA sequence
SEQ ID No. 13: S40-HAPEHR amino acid sequence
SEQ ID No. 14: S40-HAPEHR DNA sequence
SEQ ID No. 15: S40-HAPEHR-20 amino acid sequence
SEQ ID No. 16: S40-HAPEHR-20 DNA sequence
SEQ ID No. 17: Human Mannose-binding protein (UniProtKB - P11226), amino acid sequence
SEQ ID No. 18: HUMAN Pulmonary surfactant-associated protein (UniProtKB - P35247), amino acid sequence
SEQ ID No. 19: human homolog of ectodomain CD40L115-260 (UniProtKB - P29965), amino acid sequence
SEQ ID No. 20: human homolog of CCL20 segment 28-97 (UniprotKB - P78556), amino acid sequence
SEQ ID No. 21: pTRIP vector used for subcloning the constructs (pFlapDeltaU3 kpnl to AscI (no promoter, no transgene and no WPRE)
SEQ ID No. 22: BCuag promoter
SEQ ID No. 23: mutant WPRE
SEQ ID No. : 24: hSPD40-ESXH amino acid sequence

### LEGENDS OF THE FIGURES

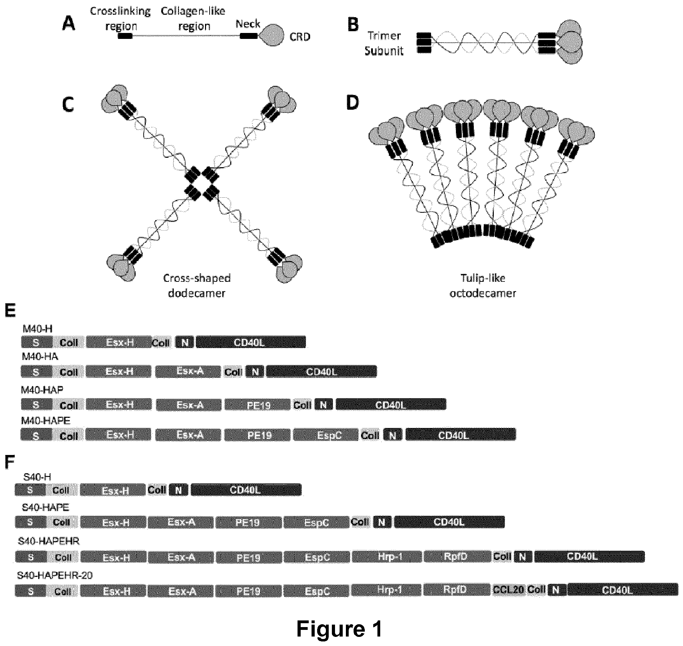

**Figure 1****.** Schematic structure of MBL or SPD collectin polymers. **(A)** Structural domains of MBL or SPD. CRD = Carbohydrate-Recognition Domain. **(B)** MBL or SPD self-assembled, collagen-like triple helixes, formed by interchain cysteine bonds. **(C)** SPD cross-shaped dodecamer. **(D)** SPD or MBL "tulip-bouquet" octodecamer. Adapted from³⁴. **(E-F)** Schematic primary structure of the designed M40 **(E)** or S40 **(F)** monomers, harboring selected Mtb antigens. Crosslinking region (S), Collagen-like region (Coll), Neck region (N).

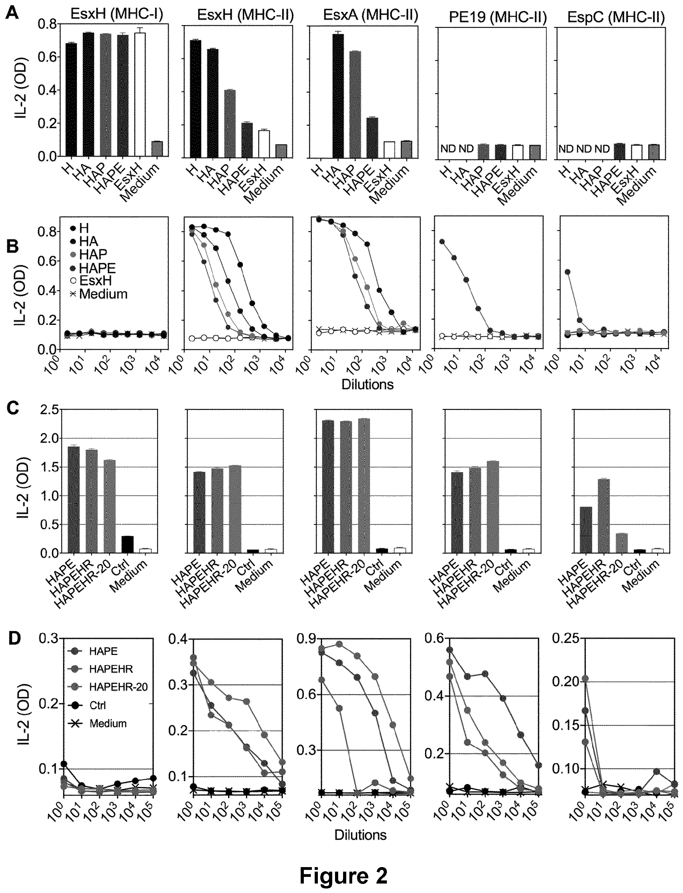

**Figure 2****. Properties of LV::M40 at inducing antigenic presentation by both MHC-I and-II pathways. (A)** BM-DC from BALB/c (H-2^{d}) or C57BL/6 (H-2^{b}) mice were transduced (MOI = 20) with LV::M40-H, -HA, -HAP or -HAPE, under the transcriptional control of BCUAG promoter. Control cells were transduced with LV::EsxH alone. **(B)** BM-DC from BALB/c or C57BL/6 mice were incubated with successive dilutions of supernatants of HEK-293T cells, transduced (MOI = 20) for 48h by each of the indicated LV. **(C)** At day 3 after the addition of LV, or at day 1 after incubation with the HEK-293T cell supernatants or peptides, presentation of MHC-I- or -II-restricted epitopes of the EsxH, EsxA, PE19 or EspC mycobacterial antigens by DC were assessed by their co-culture with T-cell hybridomas specific to EsxH:20-28 (YB8 cell line, restricted by K^{d}), EsxH:74-88 (1G1 cell line, restricted by I-A^{d}), EsxA:1-20 (NB11 cell line, restricted by I-A^{b}), PE:19:1-18 (IF6 cell line, restricted by I-A^{b}), or EspC:45:54 (IF1 cell line, restricted by I-A^{b}). **(D)** BM-DC from BALB/c or C57BL/6 mice were incubated with successive dilutions of supernatants of HEK-293T cells, transduced (MOI = 20) for 48h by each of the indicated LV. Results are concentrations of IL-2 produced by T-cell hybridomas 24h after the beginning of the co-cultures. The amounts of IL-2 produced in the co-culture supernatants are proportional to the efficacy of antigenic presentation by DC and TCR triggering.

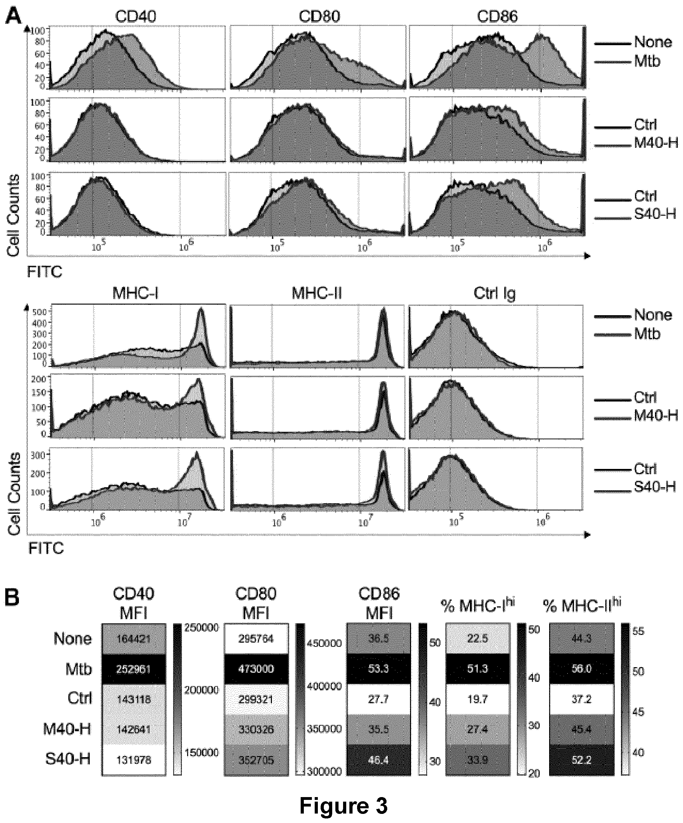

**Figure 3****. Phenotypic Maturation of DC Induced by M40 or S40. (A-B)** Phenotypic maturation of BM-DC from C57BL/6 mice infected at MOI of 5 with Mtb, as positive control, or incubated with supernatants from HEK-293T cells transduced (MOI = 20) with LV::EsxH alone (H = Ctrl), LV::M40-H or LV::S40-H. Expression of co-stimulatory or MHC molecules were assessed by cytometry on CD11b⁺ CD11c⁺ cells at 24 h p.i. **(B)** Heatmaps recapitulating the Mean Fluorescence Intensity (MFI) of CD40 or CD80 surface expression or percentages of CD86^{hi}, MHC-I^{hi}, or MHC-II^{hi} DCs.

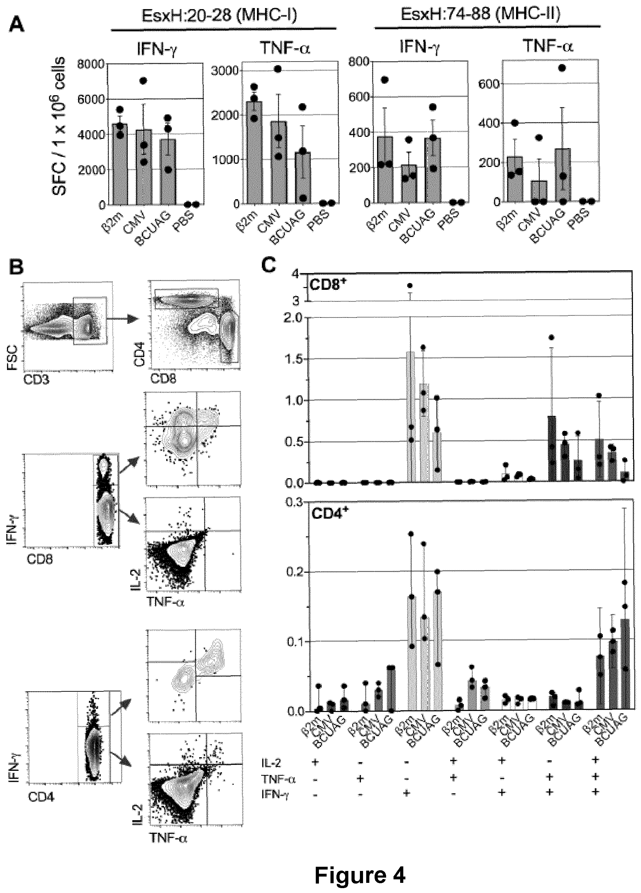

**Figure 4****. T-cell immunogenicity of LV encoding for M40-H. (A)** IFN-γ- or TNF-α-producing CD8⁺ (top) or CD4⁺ (bottom) T-cell responses, as assessed by ELISPOT at day 13 post-immunization, in the spleen of individual BALB/c mice (n = 3), immunized s.c. with 1 × 10⁸ TU/mouse of LV::M40-H, harboring β2m, CMV or BCUAG promoters. Frequencies of Spot Forming Cells (SFC) were determined subsequent to in vitro stimulation of splenocytes with EsxH:20-28 (top) or EsxH:74-88 (bottom) synthetic peptide. Shown are Median with two tailed values and 95% confidence. Quantitative differences between various groups were not statistically significant (non-parametric Mann & Whitney test, *p* < 0.05). **(B)** Cytometric gating strategy of CD4⁺ or CD8⁺ T splenocytes and Representative IFN-γ⁺ or IFN-γ⁻ CD8⁺ or CD4⁺ T cells, expressing TNF-α and/or IL-2. **(C)** Recapitulative percentages of each functional subsets within the CD8⁺ or CD4⁺ T-cell population from the mice immunized with LV::M40-H harboring β2m, CMV or BCUAG promoters. Shown are Mean +/- SD Quantitative differences between various groups were not statistically significant (non-parametric Mann & Whitney test, *p* < 0.05).

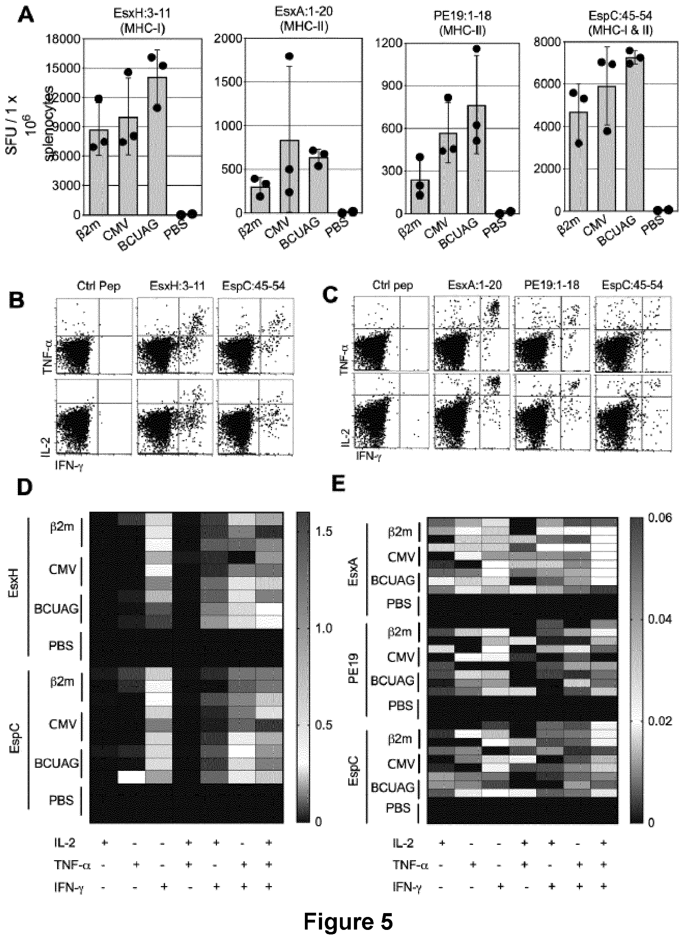

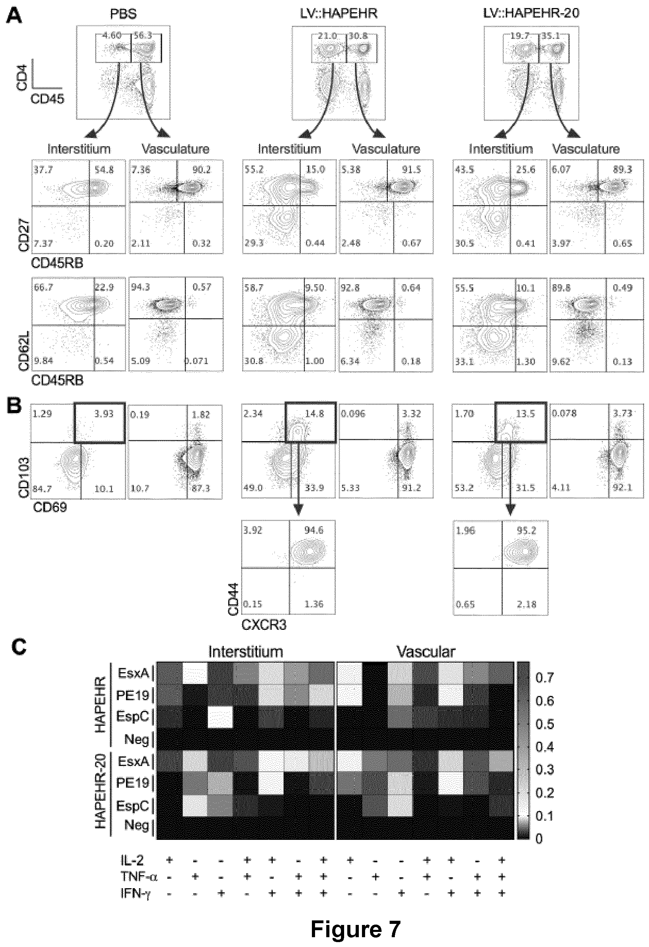

**Figure 5****. Immunogenicity of the poly-antigenic LV::M40-HAPE. (A)** IFN-γ T-cell responses, as assessed by ELISPOT at day 14 post-immunization, in the spleen of individual C57BL/6 mice (*n* = 3), immunized s.c. with 1 × 10⁸ TU/mouse of LV::M40-HAPE harboring β2m, CMV or BCUAG promoters. The frequencies of responding T cells were determined subsequent to in vitro stimulation with EsxH:3-11 (containing MHC-I-restricted epitope) or EsxA:1-20 (containing MHC-II-restricted epitope), PE10:-1-18 (containing MHC-II-restricted epitope), or EspC:45-54 (containing MHC-I and II-restricted epitopes) synthetic peptide. Shown are Median with two tailed values and 95% confidence. Quantitative differences among the groups of mice immunized with LV::M40-HAPE, harboring β2m, CMV or BCUAG promoter, were not statistically significant (non-parametric Mann & Whitney, *p* < 0.05). **(B)** Representative gating strategy **(C)** and dot plots of TNF-α⁺ vs IFN-γ⁺ or IL-2⁺ *vs* IFN-γ⁺ inside the CD8⁺ T splenocyte subset, subsequent to stimulation with a negative control, EsxH:3-11, or EspC:45-54 peptide. **(D-E)** Heatmap CD8⁺ **(D)** recapitulating percentages of each CD4⁺ **(E)** or functional subsets specific to EsxH or EspC antigens in mice immunized with LV::M40-HAPE, harboring β2m, CMV or BCUAG promoters or injected with PBS. Quantitative differences among the groups of mice immunized with LV::M40-HAPE, harboring distinct promoter were not statistically significant (non-parametric Mann & Whitney, *p* < 0.05). The immunized C57BL/6 mice were those detailed in the Figure 7.

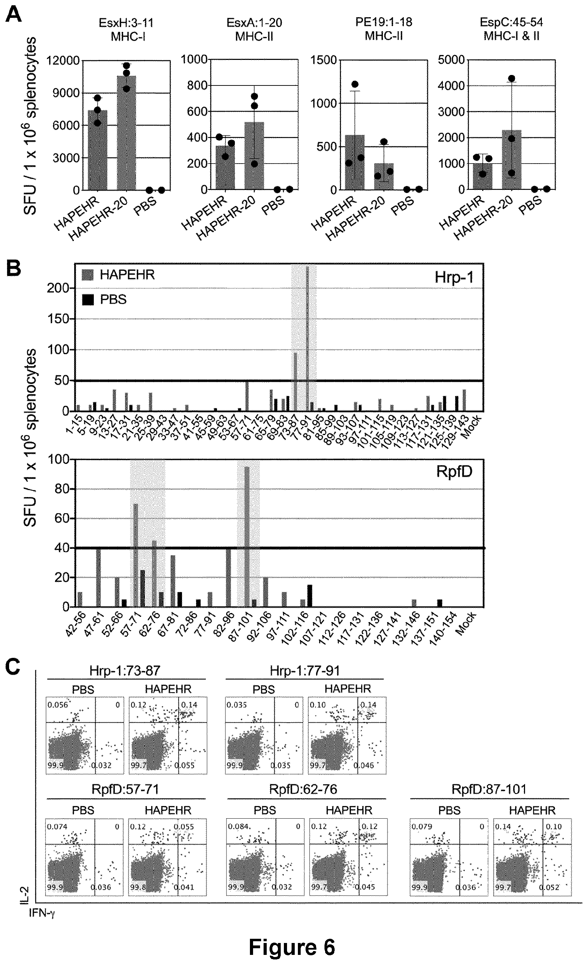

**Figure 6****. Immunogenicity of the Multi-Antigenic LV::S40-HAPEHR or LV::S40-HAPEHR-20. (A)** IFN-γ T-cell responses, as assessed by ELISPOT at day 13 post-immunization, in the spleen of individual C57BL/6 mice (n = 3), immunized s.c. with 1 × 10⁸ TU/mouse of LV::S40-HAPEHR or LV::S40-HAPEHR-20. The frequencies of responding T cells were determined following in vitro stimulation with the indicated synthetic peptides. Shown are Median with two tailed values and 95% confidence. Quantitative differences among the groups of mice immunized with LV::S40-HAPEHR or LV::S40-HAPEHR-20, were not statistically significant (non-parametric Mann & Whitney, *p* < 0.05). **(B)** Epitope mapping of Hrp-1 and RfpD as determined by the pooled splenocytes from C57BL/6 mice, injected with PBS or immunized s.c. with 1 × 10⁸ TU/mouse of LV::S40-HAPEHR, subsequent to stimulation with each of the individual peptides from the Hrp-1- or RfpD-derived overlapping 15-mers offset by 5 a.a.. (C) Cytometric analysis of intracellular IFN-γ versus IL-2 staining of CD4+ T splenocytes after stimulation with 10 µg/ml of the indicated peptides encompassing the immunodominant epitopes, identified in (B).
**Figure 7****. Features of mucosal CD4⁺ T cells triggered by i.n. immunization with LV::S40-HAPEHR or LV::S40-HAPEHR-20.** C57BL/6 mice were immunized i.n. with 1 × 10⁸ TU of LV::S40-HAPEHR or LV::S40-HAPEHR-20. At 14 dpi, lung CD4⁺ T cells were distinguished for their location within the interstitium (CD45_{i.v}⁻) or in the vasculature (CD45_{i.v}⁺) by an i.v. PE-anti-CD45 mAb injection. **(A)** Profile of CD27 versus CD62L or CD45RB, and **(B)** CD103 vs CD69, or CD44 vs CXCR3 of the lung CD4⁺ T cells of the interstitium or vasculature. **(C)** Heatmap recapitulating percentages of (poly)functional CD4⁺ T cells specific to EsxA, PE19 or EspC in the lung interstitium or vasculature, as determined by ICS. Results, representative of 2 independent experiments, are from pooled lungs per group to reach enough cells for accurate cytometric analyses.

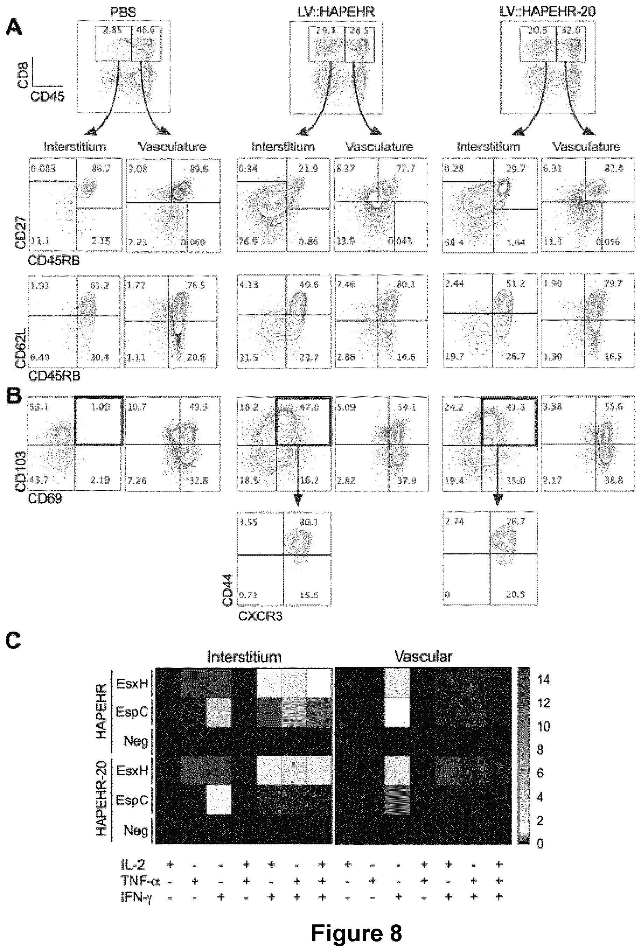

**Figure 8****. Features of mucosal CD8⁺ T cells induced by i.n. immunization with LV::S40-HAPEHR or LV::S40-HAPEHR-20.** The immunized C57BL/6 mice are those studied in the Figure 7. **(A)** Shown are lung CD8⁺ T cells, distinguished for their location within the interstitium (CD45_{i.v}⁻) or in the vasculature (CD45_{i.v}⁺). Profile of CD27 versus CD62L or CD45RB, and **(B)** CD103 vs CD69, or CD44 vs CXCR3 of the lung CD8⁺ T cells from the interstitium or vasculature. **(C)** Heatmap recapitulating percentages of (poly)functional CD8⁺ T cells specific to EsxH or EspC in the lung interstitium or vasculature, as determined by ICS. Results, representative of 2 independent experiments, are from pooled lungs per group to reach enough cells for accurate cytometric analyses.

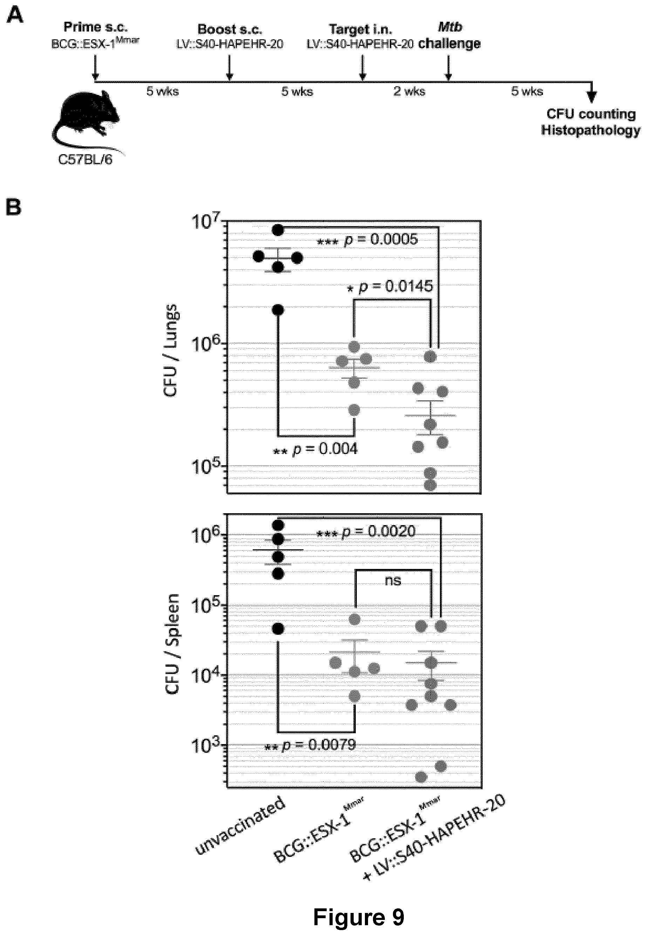

**Figure 9****. Protective potential of an optimized poly-antigenic LV as a booster against Mtb. (A)** Time line of prime-boost-challenge performed in C57BL/6 mice (n = 5-9/group). **(B)** Mtb burdens as quantitated by CFU counting in the lungs and spleen of mice at week 5 post challenge. NS = not significant, *, **, *** = statistically significant, as determined by One Tail Mann Whitney test, *p* = 0.0415, *p* = 0.0040, *p* = 0.00105, respectively.

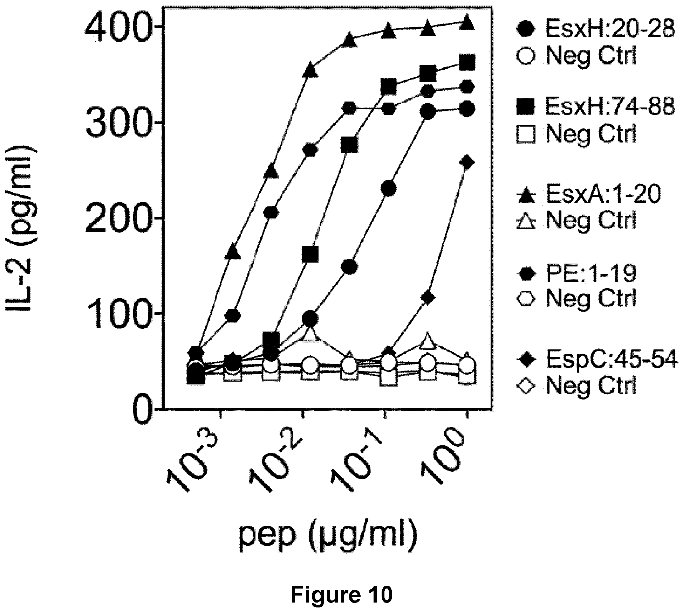

**Figure 10****. Sensitivity of the T-cell hybridomas used in the Mtb antigen presentation assays.** BM-DC from BALB/c (H-2^{d}) or C57BL/6 (H-2^{b}) mice were incubated with various concentrations of homologous or negative control peptides. At day 1, presentation of MHC-I- or -II-restricted epitopes were assessed by use of T-cell hybridomas specific to EsxH:20-28 (YB8, restricted by K^{d}), EsxH:74-88 (1G1, restricted by I-A^{d}), EsxA:1-20 (NB11, restricted by I-A^{b}), PE:19:1-18 (IF6, restricted by I-A^{b}), or EspC:45:54 (IF1, restricted by I-A^{b}). Results are concentrations of IL-2 produced by T-cell hybridomas 24h after the T-cell addition.

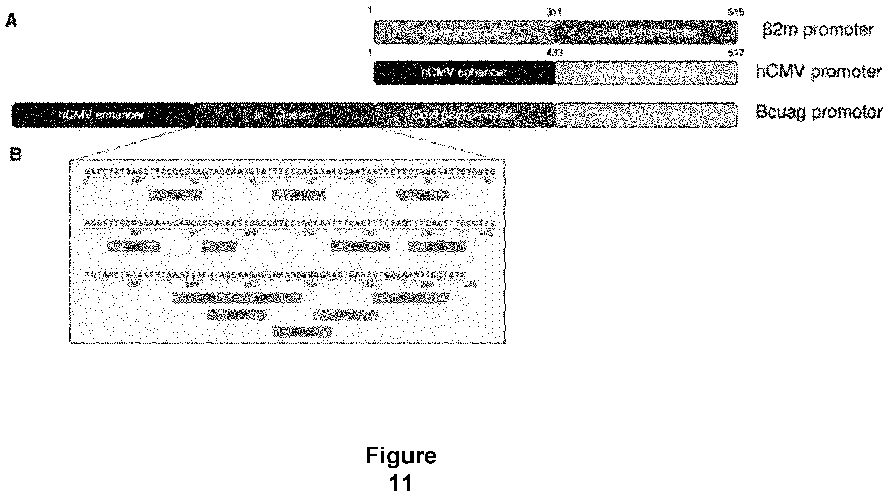

**Figure 11 (A)** **Schematic description of the three used promoters.** β2m promoter (RefSeq: LRG-1215; from 4556 to 5070), hCMV promoter (RefSeq: MN920393.1; from 174188 to 174714), BCUAG is a combination of β2m and hCMV promoters with the addition of "Inf" (Inflammation-related) cluster, a set of cis-regulating motifs associated with inflammation. **(B)** Sequence of Inf Cluster. Cis regulating motifs and associated transcriptional factor are indicated under the sequence.

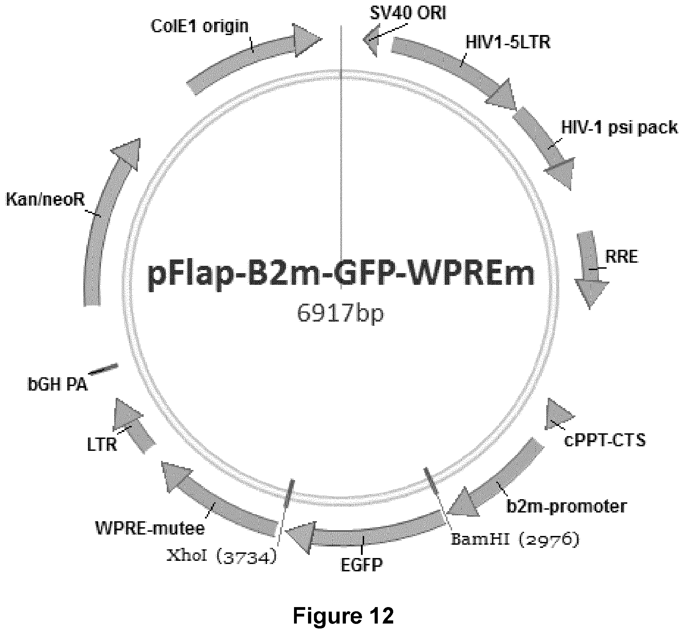

**Figure 12****. Map of the segment of the pFLAP plasmid containing M40 or S40 harboring the selected mycobacterial antigens, under β2m, CMV or BCUAG promoter.** The codon-optimized cDNA sequences, encoding for EsxH variants or poly-antigenic fusion proteins of vaccine interest, were inserted under the SP1-β2m promoter³⁶ in a pFLAP backbone plasmid.
**Figure 13****. Structure of a human SPD-40 polypeptide harboring the antigen EsxH.** P1-106: fragment of Pulmonary surfactant-associated protein D (UniProtKB - P35247 p1-106), ensures secretion via signal peptide (p1-20), enables antigen access to APC surrounding transduced cells and 2 levels of multimerization: collagen-like domain (p46-106), generates a trimerization with hydrogen bounds and cystein-rich region (p21-45), generates a covalent multimerization (n>3) via disulfide bounds. The scaffold is expected to increased bioavailability and CD40L functionality mimicking its natural trimerization. P208-273: fragment from Mannose-binding protein C (UniProtKB - P11226 p65-130). Multimerization via collagen-like domain (p208-243) and coiled-coil region (p244-273). Coiled-coil region is also a rigid spacer between transgene and CD40L ectodomain preventing deleterious interactions. P277-422: fragment of CD40L ectodomain (UniProtKB - P29965 p116-261). Ensures APC targeting and maturation through interaction with its native receptor CD40. Peptide sequence starts after EMQK motif preventing the natural methionine cleavage that occurs in wild type CD40L full-length.

### EXAMPLES

### Introduction

Lentiviral Vectors (LV) provide one of the most efficient vaccine platforms, relied on their outstanding potential of gene transfer to the nuclei of the host cells, including notably Antigen Presenting Cells (APC). Such nuclear transfer of genes initiates expression of antigens which readily access the Major Histocompatibility Complex Class-I (MHC-I) presentation machinery, i.e., proteasome, for further triggering of CD8⁺ T cells¹⁻³. In net contrast with their substantial ability at routing the endogenously produced antigens into the MHC-I pathway, viral vectors, including LV, are barely effective or inoperative in delivery of non-secreted antigens to the endosomal MHC-II compartment (MIIC) and unable to trigger CD4⁺ T cells. Although CD8⁺ T cells contribute largely to the immune control of infectious diseases or tumor growth, CD4⁺ T cells are the major immune players. In addition to their long lifespan and their own direct effector functions, CD4⁺ T cells orchestrate the immune system by regulating innate immunity, tailoring B-cell responses and supporting CD8⁺ T-cell effector functions⁴. Therefore, leveraging the potential of LV to induce CD4⁺ T cells will maximize their success rate in vaccine strategies.

Implication of CD4⁺ T cells is notably of utmost importance in the immune protection against the leading cause of death from a single infectious agent, *Mycobacterium tuberculosis* (Mtb), the etiologic agent of human pulmonary tuberculosis (TB)⁵. During the chronic infection, this intracellular bacillus is lodged inside the phagosomes of infected phagocytes, which results in the presentation of its antigens essentially by MHC-II molecules. Consequently, it is via MHC-II that the adaptive immune effector cells can recognize the infected cells to eradicate them or to strengthen their intracellular microbicidal arsenal^{6, 7}. In order to develop a poly-antigenic and multistage anti-TB subunit vaccine, we engineered a new generation of LV able to induce MHC-II antigen presentation, resulting in CD4⁺ T-cell initiation. In our rational design, we also took into the account the fact that direct delivery of antigens to APC, by their addressing to appropriate co-stimulatory cell surface receptors, substantially increases the immunogenicity by lowering the threshold of required antigen amounts and by providing a slight and local adjuvant effect^{8, 9}.

We generated a platform of LV encoding for secreted monomers of collagen-containing C-type lectins (collectins), i.e., Mannan-Binding Lectin (MBL) or *Surfactant-associated* Protein D (SPD)¹⁰, as scaffold protein carriers. The latter are engineered to harbor multiple Mtb immunogens and the ectodomain of the Tumor Necrosis Factor (TNF) family member CD40 ligand (CD40L, CD154), with the perspective of targeting and activating APC via the co-stimulatory receptor CD40¹¹. Transduction of host cells with such LV results in secretion of antigen-bearing MBL-CD40L ("M40") or SPD-CD40L ("S40") monomers. Such monomers can spontaneously self-assemble into helicoidal trimers, as the first structural units. This leads potentially to a CD40L homo-trimeric configuration, required to cluster CD40. In their turn, the trimers can further tetra- or hexamerize to form soluble macromolecule carriers, able to circulate in biological fluids or be locally taken up by bystander APC. Therefore, such macromolecule carriers can be more efficiently delivered to CD40⁺ APC and notably dendritic cells (DC), in which they gain access to MIIC. Moreover, the C-ter CD40L trimeric motifs will mimic the trivalent membrane CD40L on CD4⁺ T cells to activate DC, similar to an adjuvant. Our results provided proof-of-concept evidences that, in contrast to conventional LV, this new generation of LV encoding for M40 or S40 carriers which bear multistage Mtb immunogens: (i) induces MHC-II-restricted antigen presentation, (ii) triggers CD4⁺ - and CD8⁺ - T cells when used in systemic or intranasal (i.n.) immunization, and (iii) displays a significant booster protective effect in the TB mouse model. This innovating approach can be largely extended to LV-based vaccine candidates against numerous other bacterial or viral infectious diseases or cancers, with the critical advantage of inducing robust CD4⁺ T-cell responses, a rare property for a viral vector vaccine.

### Results

### Rational selection of antigens

To generate an LV-based vaccine vector encoding for M40 or S40 antigen carriers and potentially usable in an infectious disease controllable by CD4⁺ T cells, we selected EsxA, EspC (ESX-1 secretion-associated protein C), EsxH, PE19, Hypoxic response protein 1 (Hrp1), and Resuscitation promoting factor D (RpfD) immunogens from Mtb **(Table 1).** EsxA and EspC virulence factors are strongly immunogenic, due to their small size and their active secretion through the ESX-1 Type VII Secretion System (T7SS), which favor their access to MHC presentation machineries of the host phagocytes^{12, 13}. The highly immunogenic EsxH¹⁴⁻¹⁶, secreted through the ESX-3 T7SS, has shown protective potential in several subunit vaccine candidates. EsxH, and its close relative EsxR, are present in all the Mtb clinical isolates so far studied¹⁷. Inclusion of PE19 from the large family of PE/PPE Mtb proteins, secreted through ESX-5 T7SS^{13, 18-23}, was based on its contents in T-cell epitopes, shared by numerous homologous members of the large PE multigenic family, with various expression profiles at the distinct stages of infection, which may generate a constant display of such shared T-cell epitopes during the course of infection^{20, 23-25}.

As Mtb evolves from acute to persistence phase, its adaptation to starvation, hypoxia, nitrogen stress or host immune pressure, is regulated by the *dosRS* two-component regulatory system²⁶, which initiates transcription of 48 genes, including *rv2626c,* among the most strongly induced²⁷. The resulted Hrp1 can be target of the host adaptive immunity when the bacilli are quiescent within the granuloma²⁸. Immunity to Hrp1 may dampen latent TB reactivation. In addition, Mtb possesses five Resuscitation promoting factors (RpfA-E). Rpf are cell-wall associated or secreted enzymes with peptidoglycan hydrolase, trans-glycosylase and lytic activities. These enzymes contribute to biosynthesis of muropeptides, involved in cell wall remodeling during the bacterial division, at both acute or reactivation phases²⁹⁻³¹. We selected RpfD because of its demonstrated immunogenicity in both mice³² and latent TB humans³³. Both Hrp-1 and Rpf lack human homologs.

### Design of an LV encoding for collectin scaffolds harboring Mtb antigens and a DC targeting segment

Collectins are soluble Pattern Recognition Receptors (PRRs), able to bind oligosaccharides or lipids at the surface of microorganisms and thereby contributing to their elimination by opsonization or complement activation³⁴. Among collectins, MBL and SPD are composed of four distinct segments: (i) N-terminal cysteine-rich crosslinking domain, (ii) collagen-like domain, (iii) α-helical neck domain, and (iv) Carbohydrate-Recognition Domain (CRD) **(****Figure 1A****).** A self-assembled collagen-like triple helix forms the first structural MBL or SPD unit **(****Figure 1B****).** SPD triple-subunits themselves can tetramerize to form cross-shaped dodecamer **(****Figure 1C****).** SPD or MBL triple-subunit can also hexamerize to form "tulip-like nano-bouquet" octodecamer **(****Figure 1D****).** The resulted secreted polymers are soluble³⁴. We first engineered the murine MBL to harbor complete sequences of: (i) EsxH alone, (ii) EsxH and EsxA, (iii) EsxH, EsxA, and PE19, or (iv) EsxH, EsxA, PE19 and EspC within the collagen-like region, while replacing its CRD by the murine CD40L₁₁₅₋₂₆₀ ectodomain. The prospective MBL polymers will be referred to as "M40-H", "M40-HA", "M40-HAP", or "M40-HAPE", respectively **(****Figure 1E****, Table S1).**

In parallel, we engineered SPD to harbor: (i) EsxH alone, (ii) EsxH, EsxA, PE19, EspC, or (iii) EsxH, EsxA, PE19, EspC, Hrp1 and RpfD₄₂₋₁₅₄ within the collagen-like region and we substituted its CRD by CD40L₁₁₅₋₂₆₀ **(****Figure 1F****, Table S1).** The expected SPD polymers are referred to as "S40-H", "S40-HAPE", or "S40-HAPEHR", respectively. To confer some chemo-attracting properties to the resultant fusion protein, we also designed an S40-HAPEHR, which harbors the murine CCL20₂₈₋₉₇ segment within the collagen-like domain ("S40-HAPEHR-20"). CCL20 is the CCR6 ligand, largely involved in the migration and recruitment of DC and lymphocytes³⁵.

### Induction of MHC-II-restricted antigen presentation by LV::M40

DCs (H-2^{d} or H-2^{b}) were directly transduced with LV::M40-H, -HA, -HAP or -HAPE, using the BCUAG promoter. Control DC were transduced with a conventional LV encoding for EsxH without being inserted into an engineered scaffold. Three days post-transduction, the DCs were co-cultured with T-cell hybridomas, specific to the immunodominant epitopes of each of the Mtb antigens. The DCs transduced with either LV were largely able to induce presentation of EsxH via MHC-I **(****Figure 2A****).** In contrast to the conventional LV::EsxH, the LV encoding for M40-H, -HA, -HAP or-HAPE induced the presentation of EsxH, EsxA and PE19, when these antigens were included. No MHC-II presentation of EspC was detected in this context, which can be explained by the relatively weak sensitivity of the anti-EspC T-cell hybridoma **(****Figure 10****).** To evaluate whether M40 or S40 carriers secreted by transduced cells induce presentation through MHC-II, DC were incubated with successive dilutions of M40-H-, -HA-, -HAP-, or -HAPE-containing supernatants from transduced HEK-293T cells **(****Figure 2B****).** At day 1 after incubation, co-culture of the DCs with T-cell hybridomas, showed that these DCs were unable to present EsxH via MHC-I, strongly suggesting that endocytosis/micropinocytosis or CD40-mediated cell entry of the M40 carrier does not allow their access to MHC-I machinery, in contrast to observations made by others ³⁸. In net contrast, DCs incubated with M40-H-, -HA-, -HAP-, or -HAPE-containing supernatants were very efficient at inducing presentation of the respective antigens via MHC-II, including EspC. It was noticed that the intensity of antigen presentation had a propensity to decrease with growing number of antigens carried by the M40 scaffold **(****Figure 2A, B****).** In a mutually non-exclusive manner, this may result from: (i) a slight structural instability of the carriers with the insertion of increasing number of antigens, (ii) a competition among the multiple T-cell epitopes for the available MHC presentation sites.

Direct transduction of DCs with LV::S40-HAPE, -HAPEHR or -HAPEHR-20 induced also efficacious MHC-I- or -II-restricted presentation of the selected Mtb antigens **(****Figure 2C****).** Incubation of DC with successive dilutions of supernatants from HEK-293T cells transduced with LV::S40-HAPE, -HAPEHR or -HAPEHR-20 induced MHC-II-restricted presentation of the Mtb antigens **(****Figure 2D****).** In the absence of identified T-cell epitope or T-cell hybridoma specific to Hrp1 or RpfD, the immunogenicity of these antigens in the context of the developed vectors was studied in vivo, as detailed below. Notably, the addition of Hrp1 and RpfD and CCL20 to the S40 scaffold did not impact the efficacy of the presentation of the other antigens. The assay performed with DC incubated with synthetic peptides harboring the homologous T-cell epitopes showed the sensitivity of the T-cell hybridoma-based presentation assay **(****Figure 10****).**

These results showed that, in net opposition to conventional LV, this new generation of LV encoding for secreted scaffolds which can incorporate numerous antigens and immune mediators, possess a strong capacity at inducing MHC-II-restricted antigen presentation and thus provide a valuable platform for both CD4⁺ and CD8⁺ T cell induction.

### M40 and S40 potential at inducing DC maturation

To evaluate the potential of M40 and S40 carriers to induce DC maturation, BM-DCs were incubated with supernatants from HEK-293T cells transduced with LV::M40-H or LV::S40-H. In parallel, DCs were incubated with supernatants from HEK-293T cells transduced with the conventional LV::H, as a negative control or were infected with Mtb, as a positive control. The expression of surface co-stimulatory and MHC molecules was assessed on CD11b⁺ CD11c⁺ cells at day 1 post incubation **(****Figure 3A, B****).** On DCs incubated with M40-H or S40-H, no increase of CD40 surface expression was detected, probably as a consequence of direct interaction of CD40 with M40-H or S40-H **(****Figure 3A, B****).** CD80 upregulation was only detected on DCs incubated with S40-H, while CD86 upregulation and increase in the percentages of MHC-I^{hi} or MHC-II^{hi} cells was detected on DCs incubated with M40-H or S40-H. Therefore, through induction of M40 or S40 secretion, this new generation of LV is able to induce DC maturation, instrumental for appropriate T-cell activation.

### T-cell immunogenicity of LV encoding for M40 or S40 carrying a single or multiple Mtb immunogens

To assess the immunogenicity of this new generation of LV, BALB/c mice (n = 3/group) were immunized s.c. with LV::M40-H harboring human β2-microglobulin (β2m) promoter³⁶, human CytoMegaloVirus (CMV) immediate early enhancer and promoter (CMV)³⁷, or a composite β2m-CMV promoter ("BCUAG") to get insights on possible consequences of distinct antigen carrier transcription profile on the induction of immune responses **(****Figure 11****).** At day 13 post injection (dpi), stimulation of the splenocytes with EsxH:20-28 (MHC-I) or EsxH:74-88 (MHC-II) peptides^{15, 16} detected both CD8⁺ T and CD4⁺ T cells by ELISPOT **(****Figure 4A****).** Intracellular Cytokine Staining (ICS) showed the multifunctional properties of these CD8⁺ or CD4⁺ **(****Figure 4B, C****)** T cells. Functional CD8⁺ T cells effectors were mainly distributed among IFN-γ⁺ (single positive), IFN-γ⁺ TNF-α⁺ (double positive), or IFN-γ⁺ TNF-α⁺ IL-2⁺ (triple positive) subsets, while CD4⁺ T cells were essentially IFN-γ⁺ (single positive), or IFN-γ⁺ TNF-α⁺ IL-2⁺ (triple positive) **(****Figure 4C****).** It is noteworthy that conventional LV encoding EsxH as single do not induce such CD4⁺ T-cell responses ³⁹.

To evaluate the immunogenic potential of the developed poly-antigenic LV::M40-HAPE, C57BL/6 mice (*n* = 3/group) were immunized s.c. with LV::M40-HAPE harboring β2m, CMV or BCUAG promoter. At 14 dpi, CD8⁺ and CD4⁺ T splenocyte responses, specific to EsxH:3-11 (MHC-I), EsxA:1-20 (MHC-II), PE19:1-18 (MHC-II), or EspC:45-54 (MHC-I and -II)³⁹, were detected in all mice, as assessed by ELISPOT **(****Figure 5A****).** ICS analysis of the splenocytes from the same mice showed the multifunctional properties of the induced CD8⁺ **(****Figure 5B****)** or CD4⁺ **(****Figure 5C****)** T cells. Functional CD8⁺ T cell effectors were again mainly distributed among IFN-γ⁺ single positive, IFN-γ⁺ TNF-α⁺ double positive, or IFN-γ⁺ TNF-α⁺ IL-2⁺ triple positive subsets. CD4⁺ T cells specific to EsxA, PE10 or EspC antigen were preferentially distributed among IFN-γ⁺ single positive, IFN-γ⁺ TNF-α⁺ double positive or IFN-γ⁺ TNF-α⁺ IL-2⁺ triple positive subsets **(****Figure 5D, E****).** No consistent quantitative or qualitative differences were detected in the T-cell responses in the mice immunized with LV::M40 harboring each of the distinct promoters. Again, conventional LV encoding for these Mtb proteins as a poly-antigen, is unable to induce CD4⁺ T-cell responses³⁹.

We further established the induction of both CD8⁺ and CD4⁺ T cells specific to EsxH, EsxA, PE19 and EspC in C57BL/6 mice (*n* = 3/group) immunized s.c. with LV::S40-HAPEHR or LV::S40-HAPEHR-20 **(****Figure 6A****).** The immunogenicity of Hrp-1 and RpfD was assessed by their epitope mapping by use of splenocytes from LV::S40-HAPEHR-immunized mice in ELISPOT assay **(****Figure 6B****).** Hrp-1:77-91 (SIYYVDANASIQEML), RpfD:57-71 (IAQCESGGNWAANT) and RpfD:87-101 (SNGGVGSPAAASPQQ) immunogenic regions were identified.

Altogether, these results provide evidence of the induction of robust, polyfunctional CD4⁺ T-cell responses by immunization with the developed new generation of LV.

### Immunogenicity of the poly-antigenic multistage LV::S40 at the mucosal level

We then evaluated the immunogenicity of LV::S40-HAPEHR or LV::S40-HAPEHR20 in C57BL/6 mice immunized (i.n.) with 1 × 10⁸ TU. At 14 dpi, intravenous (i.v.) injection of the immunized mice with PE-anti-CD45 mAb, 3 min before sacrifice, allowed detection of massive T-cell recruitment to the lung interstitium distinct from those in the vasculature⁴⁰. The lung interstitial (CD45_{i.v.-}) CD4⁺ **(****Figure 7A****)** or CD8⁺ **(****Figure 8A****)** T cells of these LV::S40-HAPEHR- or LV::S40-HAPEHR20-vaccinated mice contained increased frequencies of CD27⁻ CD45RB⁻ CD62L⁻ migrant effectors and CD69⁺ CD103⁺ resident cells **(****Figure 7B****,** **Figure 8B****)** compared to their PBS-injected counterparts. Most of the CD69⁺ CD103⁺ CD4⁺ or CD8⁺ T cells were CD44⁺ CXCR3⁺. ICS analysis of these cells indicated the presence of (poly)functional CD4⁺ **(****Figure 7C****)** or CD8⁺ **(****Figure 8C****)** T cells specific to EsxA, EspC, EsxH or PE19, and essentially located in the lung interstitium.

### Booster protective effect of LV::S40-HAPEHR-20 against Mtb infection

Prime-boost strategies using BCG or an improved live-attenuated vaccine for priming, and subunit vaccine candidates for boosting, is a promising approach to improve the incomplete efficacy of BCG. To assess the booster potential of LV::S40-HAPEHR-20, C57BL/6 mice were left unvaccinated or were immunized s.c. at week 0 with 1 × 10⁶ CFU of a genetically improved BCG, i.e., BCG::ESX-1^{Mmar} vaccine candidate⁴¹ **(****Figure 9A****).** The latter provide the opportunity to perform a prime-boost with the developed LV vaccine as this live-attenuated vaccine actively secretes EsxA and EspC. A group of BCG::ESX-1^{Mmar}-primed mice was boosted s.c. with 1 × 10⁸ TU of LV::S40-HAPEHR-20 at week 5, and then again boosted i.n. at week 10 with the same LV to recruit the induced immune effectors to the lung mucosa. At week 12, mice were challenged with ≈ 200 CFU of Mtb H37Rv via aerosol and lung and spleen mycobacterial burdens were determined at week 17 **(****Figure 9B****).** The average lung Mtb load in the primed-boosted mice was decreased by ≈ 2.5 log₁₀ compared to unvaccinated controls (Mann-Whitney test, p value = 0,0005), and by ≈ 1 log₁₀ compared to their BCG::ESX-1^{Mmar}-vaccinated counterparts (Mann-Whitney test, p value = 0,0415). The LV::S40-HAPEHR-20 boost resulted in a tendency to reduce in the spleen Mtb loads, which was however not statistical significance. An explanation for this is the particularly strong protective effect of ESX-1-complemented BCG strains against dissemination to the spleen^{12, 42, 43}.

### Discussion

We developed a new generation of multifunctional LV which, in comparison to the conventional vector, has been leveraged to: (i) facilitate poly-antigen delivery, (ii) target antigens to APC that it activates, (iii) route antigens through MHC-II pathway, and (iv) induce, in addition to CD8⁺ T cells, robust and polyfunctional CD4⁺ T-cell response. Such LV are tailored to induce secretion of multimeric protein carriers formed by truncated collectin-based scaffolds, able to harbor several antigens, as well as protein components with adjuvant or chemo-attracting properties. This is achieved by insertion of potent immunogens within the collagen-rich regions of MBL or SPD, substituted with CD40L ectodomain at their CRD region. Self-assemblage and polymerization of the monomers produced in the LV-transduced cells in vitro or in vivo, results in secreted multimeric carriers able to interact with CD40⁺ cells, including APC and notably DC. It is known that antigen delivery to appropriate surface receptors of DC improves the efficacy of antigenic presentation by *several orders of* magnitude^{8, 44, 45}. The conventional LV per se, as prepared in our conditions, is barely inflammatory and induces almost no DC phenotypic or functional maturation, even used at very high doses. The capacity of LV to induce transitorily minute levels of IFN-I in vivo and IFN-I signaling in DC in vivo is not linked either to its outstanding T-cell immunogenicity ³⁹. Unlike the conventional LV, the new generation of LV described here, induces some degrees of DC maturation, via CD40 clustering by the trimeric extremities of M40 or S40 carriers. Therefore, these vectors assemble: (i) the intrinsic and outstanding CD8⁺ T-cell immunogenicity of the conventional LV and (ii) the properties of slight adjuvantation, antigen delivery to DC surface receptors, antigen routing to MHC-II and CD4⁺ T-cell immunogenicity of the secreted multimeric scaffolds that they encode.

Since TB is a disease primarily controlled by CD4⁺ T responses, as a first application, we investigated these optimized LV for their potential at inducing T-cell responses against selected Mtb antigens with preferential expression at distinct infection phases. We demonstrated in vitro a slight DC activating property of the secreted M40 or S40 carriers and their large efficiency at MHC-II- (and -I)-restricted presentation of the Mtb antigens inserted within their collagen-like domains. We then evidenced in vivo efficient induction of both (poly)functional CD8⁺ and CD4⁺ T-cell effectors, at the both systemic or mucosal levels, following s.c. or i.n. immunization. Notably only one shot of i.n. immunization generates high quality CD8⁺ and CD4⁺ T-cell effectors, with activated/effector/resident memory phenotype and located at the pulmonary interstitium. In future experiments, it will be informative to determine whether such T cells are located in the lung tertiary lymphoid organs⁴⁶.

One of the multimeric carriers, i.e., S40-HAPEHR also harbors a segment of CCL20, a strong chemo-attracting chemokine. The receptor for CCL20, CCR6 is expressed on lymphocytes and DC, thus S40-HAPEHR-20 should reinforce the recruitment of immune cells. It is admitted that prime immunization with improved live-attenuated vaccine candidates and boosting with subunit vaccines is a promising approach. We used LV::S40-HAPEHR-20 as subunit booster in the mouse TB model after prime with BCG::ESX-1^{Mmar} vaccine candidate, with largely improved protective potential compared to the parental BCG⁴¹. We observed that the lung Mtb burdens were statistically reduced by ∼1 log₁₀ after LV::S40-HAPEHR-20 boosting.

A plasmid DNA encoding SPD-CD40L has been already used as an adjuvant when mixed with another plasmid DNA encoding for the HIV-1 Gag protein and led to a significant enhancement of CD8⁺ T cell responses (43). In net contrast to the LV platform developed here, this plasmid adjuvant was unable to induce CD4⁺ T-cell proliferative or cytokine production. Insertion of SPD-Gag-CD40L into an adenoviral vector serotype 5 (Ad5) has been more recently demonstrated to elicit much stronger Gag-specific CD8⁺ T-cell responses and a protection from a Gag-expressing vaccinia virus in the mouse model⁴⁷. Co-immunization with a plasmid DNA encoding for SPD-gp100-CD40L, bearing the tumor gp100 antigen, and plasmids encoding for IL-12p70 and Granulocyte-Macrophage Colony Stimulating Factor (GM-CSF), increased immune control of the melanoma cells in mice⁴⁸. However, induction of MHC-II-restricted antigenic presentation or CD4⁺ T-cell initiation have not been addressed in these studies. Compared to these previous studies, our project used polymers of M40 or S40 to generate, not only CD8⁺ T cells, but also notably (poly)functional CD4⁺ T-cell responses and some constructs harbor the CCL20 chemoattractant component, without need for additional other adjuvant or immune-stimulatory molecules. This new property of LV at inducing CD4⁺ T cells is of utmost importance, as CD4⁺ T cells are major immune players, based on their: (i) long lifespan, (ii) direct effector functions, (iii) capacity at orchestrating the immune system by regulating innate immunity, (iv) helper functions at tailoring B-cell responses, and (v) helper functions at supporting CD8⁺ T-cell effector pathways⁴.

Altogether, we set up a new generation of LV vector leveraged to target and activate DCs, to route immunogens to MHC-II pathway and to induce both CD4⁺ and CD8⁺ T-cell responses. The applications of this innovating strategy are much larger and can be extended to vaccine LV against multiple other bacterial, viral, parasitic infectious diseases or cancers.

### Material and Methods

### Construction of transfer pFLAP plasmids encoding for MBL or SPD collectin scaffolds, harboring selected mycobacterial antigens, CD40L and/or CCL20

Genes encoding for *Mus musculus* Mannan-Binding Lectin (MBL) or *Surfactant-associated* Protein D (SPD), engineered to harbor selected mycobacterial antigens and/or CCL20 within their collagen-like domains, and murine CD40L ectodomain instead of their CRD, were synthetized by GenScript after codon optimisation. Each of these genes were inserted into the sites BamHI and Xhol of the transfer pFLAPΔU3 plasmid⁴⁹. Transcription is under control of the native human CMV, human β2-microglobulin β2m or BCUAG promoters, the two latter replacing CMV promoter after insertion between Mlul and BamHI sites. The human β2-microglobulin promoter has been previously described⁵⁰. The BCUAG promoter is a hybrid promoter comprising CMV enhancer, inflammation-related cis-regulating region and β2m core promoter **(****Figure 11****).** The pFLAPΔU3 plasmid contains also a mutated WPRE (Woodchuck Posttranscriptional Regulatory Element) sequence to improve protein expression.

### Plasmid amplification and purification

Plasmid DNA were amplified in DH5α *Escherichia coli* in Lysogeny Broth (LB) completed with 50 µg/ml of kanamycin. The plasmid DNA was then purified by use of the NucleoBond Xtra Maxi EF Kit (Macherey Nagel). After drying, the DNA pellets were resuspended in Tris-EDTA Endotoxin-Free (TE-EF) buffer overnight, quantitated in a NanoDrop 2000c spectrophotometer (Thermo Scientific), adjusted to 1 µg/µl in TE-EF buffer, aliquoted and stored at -20°C. The quality of the plasmid DNA was controlled: (i) either undigested or subsequent to digestion with a mixture of 2 plasm id-specific appropriate restriction enzymes prior to gel electrophoresis, and (ii) by sequencing the inserts in each pFLAP plasmid.

### Production and titration of LV

Non-replicative integrative LV were produced in Human Embryonic Kidney (HEK)-293T cells, as previously detailed (Zennou et al., 2000). Briefly, 1 × 10⁷ cells/Petri dish were cultured in DMEM and were co-transfected in a tripartite manner with 1 ml of a mixture of: (i) 2.5 µg/ml of the pSD-GP-NDK packaging plasmid, encoding for codon optimized *gag-pol-tat-rre-rev,* (ii) 10 µg/ml of VSV-G Indiana envelop plasmid, and (iii) 10 µg/ml of "transfer" pFLAP plasmid in Hepes 1X containing 125 mM of Ca(ClO₃)₂. Supernatants were harvested at 48h post-transfection, clarified by 6-minute centrifugation at 2500 rpm and concentrated by 1-hour ultracentrifugation at 22,000 rpm at 4°C. LV were then aliquoted in PBS 1X, PIPES 20 mM, sucrose 2.5%, NaCl 75 mM and conserved at -80°C.

To determine the titers of the produced LV, HEK-293T were distributed at 4 × 10⁴ cell/well in flat-bottom 96-well-plates in complete DMEM in the presence of 8 µM aphidicolin (Sigma) to blocks the cell growth. The cells were then transduced with serial dilutions of concentrated LV. The titers, proportional to efficacy of the nuclear gene transfer, were determined as "Transduction Unit" (TU)/ml by quantitative real-time PCR on total lysates at day 3 post-transduction, by use of forward 5'-TGG AGG AGG AGA TAT GAG GG-3' and reverse 5'-CTG CTG CAC TAT ACC AGA CA-3' primers, specific to pFLAP plasmid and forward 5'-TCT CCT CTG ACT TCA ACA GC-3' and reverse 5'-CCC TGC ACT TTT TAA GAG CC-3' primers specific to the host housekeeping gene *gadph,* as described else where⁵¹.

### Mice, immunization

C57BL/6JRj or BALB/cJ mice (Janvier, Le Genest Saint Isle, France) were used between the age of 7 and 10 weeks. Experimentation on mice was performed in accordance with the European and French guidelines (Directive 86/609/CEE and Decree 87-848 of 19 October 1987) subsequent to approval by the Institut Pasteur Safety, Animal Care and Use Committee, under local ethical committee protocol agreement # CETEA 2013-0036, # CETEA DAP180030, and CETEA 2012-0005 (APAFIS#14638-2018041214002048). Mice were immunized subcutaneously (s.c.) at the basis of the tail with the indicated amounts of LV contained in 200 µl. When indicated, mice were immunized intranasally (i.n.) with the indicated amounts of LV contained in 20 µl, as previously detailed⁵². The i.n. administration was realized under anesthesia, obtained by peritoneal injection of a mixture of Xylazine (Rompun, 10 mg/kg) and Ketamine (Imalgene, 100 mg/kg).

### T-cell assay by ELISPOT

At day 11-14 post-immunization, splenocytes from individual mice (n = 3/group) were homogenized and filtered through 100 µm-pore filters and centrifuged at 1300 rpm during 5 minutes. Cells were then treated with Red Blood Cell Lysing Buffer (Sigma), washed twice in PBS and counted in a MACSQuant10 cytometric system (Miltenyi Biotec). Splenocytes were then seeded at 0.5 - 1 × 10⁵ cells /well in 200 µl of RPMI-GlutaMAX, containing 10% heat-inactivated FBS, 100 U/ml penicillin and 100 µg/ml streptomycin, 1 × 10⁻⁴ M non-essential amino-acids, 1% vol/vol HEPES, 1 × 10⁻³ M sodium pyruvate and 5 × 10⁻⁵ M of β-mercapto-ethanol in the wells of IFN-y or TNF-α ELISPOT plates (Mouse ELISPOT^{PLUS}, Mabtech). Cells were left unstimulated or were stimulated with 2 µg/ml of synthetic peptide (Proteogenix, Strasbourg, France), harboring the well-defined MHC-I-, or -II-restricted T-cell epitopes of each mycobacterial antigen. In parallel, splenocytes were stimulated with 2.5 µg/ml of Concanavalin A (Sigma), as a functionality control. For each individual, the assays were run in technical triplicates, following Mabtech's recommendations. Spots were quantified in an ELR04 ELISPOT reader (AID, Strassberg, Germany).

### T-cell assay by intracellular cytokine staining, lung T-cell phenotyping

Splenocytes from immunized mice were obtained by tissue homogenization and passage through 100 µm-pore filter and were cultured during 6h at 8 × 10⁶ cells/well in 24-well plates in the presence of 10 µg/ml of homologous or control peptide, 1 µg/ml of anti-CD28 (clone 37.51) and 1 µg/ml of anti-CD49d (clone 9C10-MFR4.B) mAbs (BD Pharmingen). During the last 3h of incubation, cells were added with a mixture of Golgi Plug and Golgi Stop (BD Pharmingen). Cells were then collected, washed with PBS containing 3% heat-inactivated FBS and 0.1% NaN₃ (FACS buffer) and incubated for 25 minutes at 4°C with a mixture of Fcγll/III receptor blocking anti-CD16/CD32 (clone 2.4G2), APC eF780-anti-CD3ε (clone 17A2), eFluor450-anti-CD4 (RM4-5) and BV711-anti-CD8α (53-6.7), mAbs (BD Pharmingen and eBioscience). Cells were then washed twice in FACS buffer, permeabilized by use of Cytofix/Cytoperm kit (BD Pharmingen). Cells were then washed twice with PermWash 1x buffer from the Cytofix/Cytoperm kit and incubated with a mixture of FITC-anti-IL-2 (clone JES6-5H4, eBioscience), PE-Dazzle-anti-TNF-α (MP6-XT22, Biolegend) and APC-anti-IFN-y (clone XMG1.2, BD Pharmingen) mAbs or a mixture of appropriate control Ig isotypes, during 30 minutes at 4°C. Cells were then washed twice in PermWash and once in FACS buffer and then fixed with Cytofix (BD Pharmingen) overnight at 4°C. The cells were acquired in an Attune NxT cytometer system (Invitrogen). Data were analyzed by FlowJo software (Treestar, OR, USA). Lung T-cell phenotyping was performed as recently described³⁹.

### Antigenic presentation assay

Bone-marrow derived DC were plated at 5 × 10⁵ cells/well in 24-well plates in RPMI 1640 containing 5% FBS. Cells were transduced with LV or were loaded homologous or control synthetic peptides. At 24 h post infection 5 × 10⁵ appropriate T-cell hybridomas⁵³ were added and the culture supernatants were quantitated for IL-2 production at 24h by ELISA. Synthetic peptides were synthesized by Proteogenix (Schiltigheim, France).

### Protection assay

Mtb H37Rv strain or BCG::ESX-1^{Mmar 41}, were cultured in Dubos broth, complemented with Albumine, Dextrose and Catalase (ADC, Difco, Becton Dickinson, Le Pont-de-Claix, France). Experiments with pathogenic mycobacteria were performed in BSL3, following the hygiene and security recommendations of Institut Pasteur. C57BL/6 mice were primed s.c. with 1 × 10⁶ CFU/mouse of BCG::ESX-1^{Mmar 41} at day 0, boosted s.c. with 5 × 10⁸ TU of SPD40-HAPEHR-20 at week 5, and boosted i.n. with 5 × 10⁸ TU of SPD40-HAPEHR-20 at week 10. The mice were challenged 2 weeks after the mucosal boost by use of a homemade nebulizer via aerosol, as previously described⁵². Briefly, 5 ml of a suspension of 1.7 × 10⁶ CFU/ml of Mtb H37Rv strain were aerosolized in order to deliver an inhaled dose of ≈ 200 CFU/mouse. The mice were then placed in isolator. Five weeks later, lungs or spleen of the infected mice were homogenized by using a MillMixer homogenizer (Qiagen, Courtaboeuf, France) and serial 5-fold dilutions prepared in PBS were plated on 7H11 Agar complemented with ADC (Difco, Becton Dickinson). CFU were counted after 3 weeks of incubation at 37°C. Statistical significance of inter-group Mtb load differences was determined by Mann-Whitney t-test by use of Prism v8.01 (GraphPad Software, Inc.).

### Tables

**Table 1. Mtb proteins rationally selected as target antigens to be incorporated in the prospective multistage anti-TB LV.**

| **Mtb immunogen** | **Locus in H37Rv** | **Size a.a** | **Major characteristics** |
|---|---|---|---|
| EsxA | *rv3875* | 95 | Early Secreted Antigenic Target 6 kDa (ESAT-6) secreted by ESX-1 T7SS |
| EspC | *rv3615c* | 103 | ESX-1 secretion-associated proteins C secreted by ESX-1 T7SS |
| EsxH | *rv0288* | 96 | Virulence-related factor (TB10.4) secreted by ESX-3 T7SS |
| PE19 | *rv1791* | 99 | Virulence-related factor, with numerous homologous secreted by ESX-5 T7SS |
| Hrp1 | *rv2626c* | 143 | Dormancy-related Hypoxic response protein 1 |
| RpfD | *rv2389c* | 154 (42-154)* | Reactivation-related Resuscitation promoting factor D (mb) |

| | | | |
|---|---|---|---|
| *Only the RpfD₄₂₋₁₅₄ ectodomain was included to minimize the hydrophobicity of the resulted protein. | | | |

**Supplemental Table 1. Various M40 and S40 scaffolds designed to harbor the selected Mtb antigens and/or CCL20.**

| **Carrier** | **Antigens** | **Chemo-attractant** | **Nomenclature** | **Monomer Lenght (a.a)** |
|---|---|---|---|---|
| MBL40 | EsxH | - | M40-H | 414 |
| | EsxH-EsxA | - | M40-HA | 556 |
| | EsxH-EsxA-PE19 | - | M40-HAP | 721 |
| | EsxH-EsxA-PE19-EspC | - | M40-HAPE | 885 |
| SPD40 | EsxH | - | S40-H | xx |
| | EsxH-EsxA-PE19-EspC | - | S40-HAPE | 736 |
| | EsxH-EsxA-PE19-EspC-Hrp1-RpfD | - | S40-HAPEHR | 1004 |
| | EsxH-EsxA-PE19-EspC-Hrp1-RpfD | CCL20 | S40-HAPEHR-20 | 1128 |

**Table S2. Sequences of MBL fused with selected Mtb antigens and CCL20 as coded by LV**

| **Poly-antigenic LV** | **Inser t lengt h (a.a.)** | **Sequence** |
|---|---|---|
| LV::M40-**EsxH** (LV::M40-H) | 414 | MSIFTSFLLLCVVTVVYAETLTEGVQNSCPVVTCSSPGLNGFPGKDGRDGAK GEKGEPGQGLRGLQGPPGAVGPTGPPGNPGLKGAVGPKGDRGDRGGGSQIM YNYPAMLGHAGDMAGYAGTLQSLGAEIAVEQAALQSAWQGDTGITYQA WQAQWNQAMEDLVRAYHAMSSTHEANTMAMMARDTAEAAKWGGGS GLRGLQGPPGALGPPGSVGSPGSPGPKGQKGDHGDNRAIEEKLANMEAEIRI LKSKLQLTNKLHAFSMGGGSGDEDPQIAAHVVSEANSNAASVLQWAKKGYY TMKSNLVMLENGKQLTVKREGLYYVYTQVTFCSNREPSSQRPFIVGLWLKP SSGSERILLKAANTHSSSQLCEQQSVHLGGVFELQAGASVFVNVTEASQVIHR VGFSSFGLLKL |
| LV::M40-**EsxH-*EsxA*** (LV::M40-HA) | 556 | MSIFTSFLLLCVVTVVYAETLTEGVQNSCPVVTCSSPGLNGFPGKDGRDGAK GEKGEPGQGLRGLQGPPGAVGPTGPPGNPGLKGAVGPKGDRGDRGGGSQIM YNYPAMLGHAGDMAGYAGTLQSLGAEIAVEQAALQSAWQGDTGITYQA WQAQWNQAMEDLVRAYHAMSSTHEANTMAMMARDTAEAAKWGGGS GFPGPPGPKGEPGSPAGRGERGFQGSPGKMGPAGSKGEPGGGSGTEQQWNF AGIEAAASAIQGNVTSIHSLLDEGKQSLTKLAAAWGGSGSEAYQGVQQKWD ATATELNNALQNLARTISEAGQAMASTEGNVTGMFAGGGSGLPGRDGRDG REGPRGEKGDPGLPGPMGLSGLQGPTGPVGPKGENGSAGEPGPKGERGLSG GGGSFVTTQPEALAAAAANLQGIGTTMNAQNAAAAAPTTGVVPAAADEVSALT AAQFAAHAQMYQTVSAQAAAIHEMFVNTLVASSGSYAATEAANAAAAGGCPG LPGAAGPKGEAGAKGDRGESGLPGIPGKEGPTGPKGNQGEKGIRGEKGDSGP SGGGSTENLTVQPERLGVLASHHDNAAVDASSGVEAAAGLGESVAITHGP YCSQFNDTLNVYLTAHNALGSSLHTAGVDLAKSLRIAAKIYSEADEAWR |
| LV::M40-**EsxH-*****EsxA**-PE19* (LV::M40-HAP) | 721 | MSIFTSFLLLCVVTVVYAETLTEGVQNSCPVVTCSSPGLNGFPGKDGRDGAK GEKGEPGQGLRGLQGPPGAVGPTGPPGNPGLKGAVGPKGDRGDRGGGSQIM YNYPAMLGHAGDMAGYAGTLQSLGAEIAVEQAALQSAWQGDTGITYQA WQAQWNQAMEDLVRAYHAMSSTHEANTMAMMARDTAEAAKWGGGS GFPGPPGPKGEPGSPAGRGERGFQGSPGKMGPAGSKGEPGGGSGTEQQWNF AGIEAAASAIQGNVTSIHSLLDEGKQSLTKLAAAWGGSGSEAYQGVQQKWD ATATELNNALQNLARTISEAGQAMASTEGNVTGMFAGGGSGLPGRDGRDG REGPRGEKGDPGLPGPMGLSGLQGPTGPVGPKGENGSAGEPGPKGERGLSG GGGSFVTTQPEALAAAAANLQGIGTTMNAQNAAAAAPTTGVVPAAADEVSALT AAQFAAHAQMYQTVSAQAAAIHEMFVNTLVASSGSYAATEAANAAAAGGGSG LRGLQGPPGALGPPGSVGSPGSPGPKGQKGDHGDNRAIEEKLANMEAEIRIL KSKLQLTNKLHAFSMGGGSGDEDPQIAAHVVSEANSNAASVLQWAKKGYYT MKSNLVMLENGKQLTVKREGLYYVYTQVTFCSNREPSSQRPFIVGLWLKPS SGSERILLKAANTHSSSQLCEQQSVHLGGVFELQAGASVFVNVTEASQVIHRV GFSSFGLLKL |
| LV::M40-**EsxH-*****EsxA**-PE19-***EspC** (LV::M40-HAPE) | 885 | MSIFTSFLLLCVVTVVYAETLTEGVQNSCPVVTCSSPGLNGFPGKDGRDGAK GEKGEPGQGLRGLQGPPGAVGPTGPPGNPGLKGAVGPKGDRGDRGGGSQIM YNYPAMLGHAGDMAGYAGTLQSLGAEIAVEQAALQSAWQGDTGITYQA WQAQWNQAMEDLVRAYHAMSSTHEANTMAMMARDTAEAAKWGGGS GFPGPPGPKGEPGSPAGRGERGFQGSPGKMGPAGSKGEPGGGSGTEQQWNF AGIEAAASAIQGNVTSIHSLLDEGKQSLTKLAAAWGGSGSEAYQGVQQKWD ATATELNNALQNLARTISEAGQAMASTEGNVTGMFAGGGSGLPGRDGRDG REGPRGEKGDPGLPGPMGLSGLQGPTGPVGPKGENGSAGEPGPKGERGLSG GGGSFVTTQPEALAAAAANLQGIGTTMNAQNAAAAAPTTGVVPAAADEVSALT AAQFAAHAQMYQTVSAQAAAIHEMFVNTLVASSGSYAATEAANAAAAGGCPG LPGAAGPKGEAGAKGDRGESGLPGIPGKEGPTGPKGNQGEKGIRGEKGDSGP SGGGSTENLTVQPERLGVLASHHDNAAVDASSGVEAAAGLGESVAITHGP YCSQFNDTLNVYLTAHNALGSSLHTAGVDLAKSLRIAAKIYSEADEAWR |
| | | KAIDGLFTGGGSGLRGLQGPPGALGPPGSVGSPGSPGPKGQKGDHGDNRAIE EKLANMEAEIRILKSKLQLTNKLHAFSMGGGSGDEDPQIAAHVVSEANSNAA SVLQWAKKGYYTMKSNLVMLENGKQLTVKREGLYYVYTQVTFCSNREPSS QRPFIVGLWLKPSSGSERILLKAANTHSSSQLCEQQSVHLGGVFELQAGASVF VNVTEASQVIHRVGFSSFGLLKL |

**Table S3. Sequences of SPD fused with selected Mtb antigens and CCL20 as coded by LV**

| **Polyantigenic LV** | **Insert lengt h (a.a.)** | **Sequence** |
|---|---|---|
| LV::S40-**EsxH** (LV::S40-H) | 506 | MLPFLSMLVLLVQPLGNLGAEMKSLSQRSVPNTCTLVMCSPTENGLPGRDG RDGREGPRGEKGDPGLPGPMGLSGLQGPTGPVGPKGENGSAGEPGPKGERG LSGGSGSQIMYNYPAMLGHAGDMAGYAGTLQSLGAEIAVEQAALQSAW QGDTGITYQAWQAQWNQAMEDLVRAYHAMSSTHEANTMAMMARDT AEAAKWGGGSGPPGLPGIPGPAGKEGPSGKQGNIGPQGKPGPKGEAGPKGE VGAPGMQGSTGAKGSTGPKGERGAPGVQGAPGNAGAAGPAGPAGPQGAPG SRGPPGLKGDRGVPGDRGIKGESGLPDSAALRQQMEALKGKLQRLEVAFSH YQKAALFPDGGGSGDEDPQIAAHVVSEANSNAASVLQWAKKGYYTMKSNL VMLENGKQLTVKREGLYYVYTQVTFCSNREPSSQRPFIVGLWLKPSSGSERI LLKAANTHSSSQLCEQQSVHLGGVFELQAGASVFVNVTEASQVIHRVGFSSF GLLKL |
| LV::S40-**EsxH-*****EsxA**-PE19-***EspC** (LV::S40-HAPE) | 736 | MLPFLSMLVLLVQPLGNLGAEMKSLSQRSVPNTCTLVMCSPTENGLPGRDG RDGREGPRGEKGDPGLPGPMGLSGLQGPTGPVGPKGENGSAGEPGPKGERG LSGGSGSQIMYNYPAMLGHAGDMAGYAGTLQSLGAEIAVEQAALQSAW QGDTGITYQAWQAQWNQAMEDLVRAYHAMSSTHEANTMAMMARDT AEAAKWGGGSGGTEQQWNFAGIEAAASAIQGNVTSIHSLLDEGKQSLTKLA AAWGGSGSEAYQGVQQKWDATATELNNALQNLARTISEAGQAMASTEGN VTGMFAGGGSGGSFVTTQPEALAAAAANLQGIGTTMNAQNAAAAAPTTGVVP AAADEVSALTAAQFAAHAQMYQTVSAQAAAIHEMFVNTLVASSGSYAATEAAN AAAAGGGSGGTENLTVQPERLGVLASHHDNAAVDASSGVEAAAGLGESV AITHGPYCSQFNDTLNVYLTAHNALGSSLHTAGVDLAKSLRIAAKIYSEA DEAWRKAIDGLFTGSGGSGGLRGLQGPPGALGPPGSVGSPGSPGPKGQKGD HGDNRAIEEKLANMEAEIRILKSKLQLTNKLHAFSMGGGSGDEDPQIAAHVV SEANSNAASVLQWAKKGYYTMKSNLVMLENGKQLTVKREGLYYVYTQVTF CSNREPSSQRPFIVGLWLKPSSGSERILLKAANTHSSSQLCEQQSVHLGGVFE LQAGASVFVNVTEASQVIHRVGFSSFGLLKL |
| LV::S40-**EsxH-*****EsxA**-PE19-***EspC** *-Hrp1-**RpfD*** LV::S40-HAPEHR | 1004 | MLPFLSMLVLLVQPLGNLGAEMKSLSQRSVPNTCTLVMCSPTENGLPGRDG RDGREGPRGEKGDPGLPGPMGLSGLQGPTGPVGPKGENGSAGEPGPKGERG LSGGSGSQIMYNYPAMLGHAGDMAGYAGTLQSLGAEIAVEQAALQSAW QGDTGITYQAWQAQWNQAMEDLVRAYHAMSSTHEANTMAMMARDT AEAAKWGGGSGGTEQQWNFAGIEAAASAIQGNVTSIHSLLDEGKQSLTKLA AAWGGSGSEAYQGVQQKWDATATELNNALQNLARTISEAGQAMASTEGN VTGMFAGGGSGGSFVTTQPEALAAAAANLQGIGTTMNAQNAAAAAPTTGVVP AAADEVSALTAAQFAAHAQMYQTVSAQAAAIHEMFVNTLVASSGSYAATEAAN AAAAGGGSGGTENLTVQPERLGVLASHHDNAAVDASSGVEAAAGLGESV AITHGPYCSQFNDTLNVYLTAHNALGSSLHTAGVDLAKSLRIAAKIYSEA DEAWRKAIDGLFTGSGGSGGTTARDIMNAGVTCVGEHETLTAAAQYMREHD IGALPICGDDDRLHGMLTDRDIVIKGLAAGLDPNTATAGELARDSIYYVDANASI QEMLNVMEEHQVRRVPVISEHRLVGIVTEADIARHLPEHAIVQFVKAICSPMAL ASGGGSGSGGLSTISSKADDIDWDAIAQCESGGNWAANTGNGLYGGLQISQA TWDSNGGVGSPAAASPQQQIEVADNIMKTQGPGAWPKCSSCSQGDAPLGS |
| | | LTHILTFLAAETGGCSGSRDDGGSGGLRGLQGPPGALGPPGSVGSPGSPGPKG QKGDHGDNRAIEEKLANMEAEIRILKSKLQLTNKLHAFSMGGGSGDEDPQI AAHVVSEANSNAASVLQWAKKGYYTMKSNLVMLENGKQLTVKREGLYYVY TQVTFCSNREPSSQRPFIVGLWLKPSSGSERILLKAANTHSSSQLCEQQSVHL GGVFELQAGASVFVNVTEASQVIHRVGFSSFGLLKL |
| LV::S40-**EsxH-*****EsxA**-PE19-***EspC** *Hrp1*-***RpfD***-**CCL20** LV::S40 HAPEHR-20 | 1128 | MLPFLSMLVLLVQPLGNLGAEMKSLSQRSVPNTCTLVMCSPTENGLPGRDG RDGREGPRGEKGDPGLPGPMGLSGLQGPTGPVGPKGENGSAGEPGPKGERG LSGGSGSQIMYNYPAMLGHAGDMAGYAGTLQSLGAEIAVEQAALQSAW QGDTGITYQAWQAQWNQAMEDLVRAYHAMSSTHEANTMAMMARDT AEAAKWGGGSGGTEQQWNFAGIEAAASAIQGNVTSIHSLLDEGKQSLTKLA AAWGGSGSEAYQGVQQKWDATATELNNALQNLARTISEAGQAMASTEGN VTGMFAGGGSGGSFVTTQPEALAAAAANLQGIGTTMNAQNAAAAAPTTGVVP AAADEVSALTAAQFAAHAQMYQTVSAQAAAIHEMFVNTLVASSGSYAATEAAN AAAAGGGSGGTENLTVQPERLGVLASHHDNAAVDASSGVEAAAGLGESV AITHGPYCSQFNDTLNVYLTAHNALGSSLHTAGVDLAKSLRIAAKIYSEA DEAWRKAIDGLFTGSGGSGGTTARDIMNAGVTCVGEHETLTAAAQYMREHD IGALPICGDDDRLHGMLTDRDIVIKGLAAGLDPNTATAGELARDSIYYVDANASI QEMLNVMEEHQVRRVPVISEHRLVGIVTEADIARHLPEHAIVQFVKAICSPMAL ASGGGSGSGGLSTISSKADDIDWDAIAQCESGGNWAANTGNGLYGGLQISQA TWDSNGGVGSPAAASPQQQIEVADNIMKTQGPGAWPKCSSCSQGDAPLGS LTHILTFLAAETGGCSGSRDDGGSGGGFPGPPGPKGEPGSPAGRGERGFQG SPGKMGPAGSKGEPGGSGSGGASNYDCCLSYIQTPLPSRAIVGFTRQMAD EACDINAIIFHTKKRKSVCADPKQNWVKRAVNLLSLRVKKMGSGSGSGG LRGLQGPPGALGPPGSVGSPGSPGPKGQKGDHGDNRAIEEKLANMEAEIRIL KSKLQLTNKLHAFSMGGGSGDEDPQIAAHVVSEANSNAASVLQWAKKGYYT MKSNLVMLENGKQLTVKREGLYYVYTQVTFCSNREPSSQRPFIVGLWLKPS SGSERILLKAANTHSSSQLCEQQSVHLGGVFELQAGASVFVNVTEASQVIHRV GFSSFGLLKL |

### References

1. Di Nunzio, F, Felix, T, Arhel, NJ, Nisole, S, Charneau, P, and Beignon, AS (2012). HIV-derived vectors for therapy and vaccination against HIV. Vaccine 30: 2499-2509.
2. Hu, B, Tai, A, and Wang, P (2011). Immunization delivered by lentiviral vectors for cancer and infectious diseases. Immunol Rev 239: 45-61.
3. Rowe, HM, Lopes, L, Ikeda, Y, Bailey, R, Barde, I, Zenke, M, et al. (2006). Immunization with a lentiviral vector stimulates both CD4 and CD8 T cell responses to an ovalbumin transgene. Mol Ther 13: 310-319.
4. Zhu, J, and Paul, WE (2010). Heterogeneity and plasticity of T helper cells. Cell Res 20: 4-12.
5. WHO (2018). https://www.who.int/tb/global-report-2019.
6. Lewinsohn, DA, Lewinsohn, DM, and Scriba, TJ (2017). Polyfunctional CD4(+) T Cells As Targets for Tuberculosis Vaccination. Front Immunol 8: 1262.
7. Mayer-Barber, KD, and Barber, DL (2015). Innate and Adaptive Cellular Immune Responses to Mycobacterium tuberculosis Infection. Cold Spring Harb Perspect Med 5**.**
8. Dong, H, Stanek, O, Salvador, FR, Langer, U, Morillon, E, Ung, C, et al. (2013). Induction of protective immunity against Mycobacterium tuberculosis by delivery of ESX antigens into airway dendritic cells. Mucosal Immunol 6: 522-534.
9. Gornati, L, Zanoni, I, and Granucci, F (2018). Dendritic Cells in the Cross Hair for the Generation of Tailored Vaccines. Front Immunol 9: 1484.
10. Presanis, JS, Kojima, M, and Sim, RB (2003). Biochemistry and genetics of mannan-binding lectin (MBL). Biochem Soc Trans 31: 748-752.
11. Laman, JD, Claassen, E, and Noelle, RJ (2017). Functions of CD40 and Its Ligand, gp39 (CD40L). Crit Rev Immunol 37: 371-420.
12. Groschel, MI, Sayes, F, Simeone, R, Majlessi, L, and Brosch, R (2016). ESX secretion systems: mycobacterial evolution to counter host immunity. Nat Rev Microbiol 14: 677-691.
13. Majlessi, L, Prados-Rosales, R, Casadevall, A, and Brosch, R (2015). Release of mycobacterial antigens. Immunol Rev 264: 25-45.
14. Billeskov, R, Vingsbo-Lundberg, C, Andersen, P, and Dietrich, J (2007). Induction of CD8 T cells against a novel epitope in TB10.4: correlation with mycobacterial virulence and the presence of a functional region of difference-1. J Immunol 179: 3973-3981.
15. Hervas-Stubbs, S, Majlessi, L, Simsova, M, Morova, J, Rojas, MJ, Nouze, C, et al. (2006). High frequency of CD4+ T cells specific for the TB10.4 protein correlates with protection against Mycobacterium tuberculosis infection. Infect Immun 74: 3396-3407.
16. Majlessi, L, Rojas, MJ, Brodin, P, and Leclerc, C (2003). CD8+-T-cell responses of Mycobacterium-infected mice to a newly identified major histocompatibility complex class I-restricted epitope shared by proteins of the ESAT-6 family. Infect Immun 71: 7173-7177.
17. Tufariello, JM, Chapman, JR, Kerantzas, CA, Wong, KW, Vilcheze, C, Jones, CM, et al. (2016). Separable roles for Mycobacterium tuberculosis ESX-3 effectors in iron acquisition and virulence. Proc Natl Acad Sci U S A 113: E348-357.
18. Abdallah, AM, Verboom, T, Weerdenburg, EM, Gey van Pittius, NC, Mahasha, PW, Jimenez, C, et al. (2009). PPE and PE_PGRS proteins of Mycobacterium marinum are transported via the type VII secretion system ESX-5. Molecular Microbiology 73: 329-340.
19. Banu, S, Honore, N, Saint-Joanis, B, Philpott, D, Prevost, MC, and Cole, ST (2002). Are the PE-PGRS proteins of Mycobacterium tuberculosis variable surface antigens? Mol Microbiol 44: 9-19.
20. Bottai, D, Di Luca, M, Majlessi, L, Frigui, W, Simeone, R, Sayes, F, et al. (2012). Disruption of the ESX-5 system of Mycobacterium tuberculosis causes loss of PPE protein secretion, reduction of cell wall integrity and strong attenuation. Mol Microbiol 83: 1195-1209.
21. Cole, ST, Brosch, R, Parkhill, J, Garnier, T, Churcher, C, Harris, D, et al. (1998). Deciphering the biology of Mycobacterium tuberculosis from the complete genome sequence. Nature 393: 537-544.
22. lantomasi, R, Sali, M, Cascioferro, A, Palucci, I, Zumbo, A, Soldini, S, et al. (2011). PE_PGRS30 is required for the full virulence of Mycobacterium tuberculosis. Cell Microbiol: doi: 10.1111/j.1462-5822.
23. Sayes, F, Sun, L, Di Luca, M, Simeone, R, Degaiffier, N, Fiette, L, et al. (2012). Strong immunogenicity and cross-reactivity of Mycobacterium tuberculosis ESX-5 type VII secretion: encoded PE-PPE proteins predicts vaccine potential. Cell Host Microbe 11: 352-363.
24. Fishbein, S, van Wyk, N, Warren, RM, and Sampson, SL (2015). Phylogeny to function: PE/PPE protein evolution and impact on Mycobacterium tuberculosis pathogenicity. Mol Microbiol 96: 901-916.
25. Gey van Pittius, NC, Sampson, SL, Lee, H, Kim, Y, van Helden, PD, and Warren, RM (2006). Evolution and expansion of the Mycobacterium tuberculosis PE and PPE multigene families and their association with the duplication of the ESAT-6 (esx) gene cluster regions. BMC Evol Biol 6: 95.
26. Roupie, V, Romano, M, Zhang, L, Korf, H, Lin, MY, Franken, KL, et al. (2007). Immunogenicity of eight dormancy regulon-encoded proteins of Mycobacterium tuberculosis in DNA-vaccinated and tuberculosis-infected mice. Infect Immun 75: 941-949.
27. Shi, L, Jung, YJ, Tyagi, S, Gennaro, ML, and North, RJ (2003). Expression of Th1-mediated immunity in mouse lungs induces a Mycobacterium tuberculosis transcription pattern characteristic of nonreplicating persistence. Proc Natl Acad Sci U S A 100: 241-246.
28. Cunningham, AF, and Spreadbury, CL (1998). Mycobacterial stationary phase induced by low oxygen tension: cell wall thickening and localization of the 16-kilodalton alpha-crystallin homolog. J Bacteriol 180: 801-808.
29. Nikitushkin, VD, Demina, GR, and Kaprelyants, AS (2016). Rpf Proteins Are the Factors of Reactivation of the Dormant Forms of Actinobacteria. Biochemistry (Mosc) 81: 1719-1734.
30. Romano, M, Aryan, E, Korf, H, Bruffaerts, N, Franken, CL, Ottenhoff, TH, et al. (2012). Potential of Mycobacterium tuberculosis resuscitation-promoting factors as antigens in novel tuberculosis sub-unit vaccines. Microbes Infect 14: 86-95.
31. Rosser, A, Stover, C, Pareek, M, and Mukamolova, GV (2017). Resuscitation-promoting factors are important determinants of the pathophysiology in Mycobacterium tuberculosis infection. Crit Rev Microbiol 43: 621-630.
32. Yeremeev, W, Kondratieva, TK, Rubakova, EI, Petrovskaya, SN, Kazarian, KA, Telkov, MV, et al. (2003). Proteins of the Rpf family: immune cell reactivity and vaccination efficacy against tuberculosis in mice. Infect Immun 71: 4789-4794.
33. Commandeur, S, van Meijgaarden, KE, Lin, MY, Franken, KL, Friggen, AH, Drijfhout, JW, et al. (2011). Identification of human T-cell responses to Mycobacterium tuberculosis resuscitation-promoting factors in long-term latently infected individuals. Clin Vaccine Immunol 18: 676-683.
34. Gupta, G, and Surolia, A (2007). Collectins: sentinels ofinnate immunity. BioEssays 29.5: 452-464.
35. Lee, AY, Eri, R, Lyons, AB, Grimm, MC, and Korner, H (2013). CC Chemokine Ligand 20 and Its Cognate Receptor CCR6 in Mucosal T Cell Immunology and Inflammatory Bowel Disease: Odd Couple or Axis of Evil? Front Immunol 4: 194.
36. Ku, MW, Authié, P, Souque, P, Bourgine, M, Romano, M, Charneau, P, et al. (manuscript in preparation). Lentiviral vector-induced high-quality memory T cells via programmed antigen expression in dendritic cells.
37. Boshart, M, Weber, F, Jahn, G, Dorsch-Hasler, K, Fleckenstein, B, and Schaffner, W (1985). A very strong enhancer is located upstream of an immediate early gene of human cytomegalovirus. Cell 41: 521-530.
38. Chatterjee, B, Smed-Sorensen, A, Cohn, L, Chalouni, C, Vandlen, R, Lee, BC, et al. (2012). Internalization and endosomal degradation of receptor-bound antigens regulate the efficiency of cross presentation by human dendritic cells. Blood 120: 2011-2020.
39. Lopez, J, Anna, F, Authié, P, Pawlik, A, Ku, MW, Blanc, C, et al. (in preparation). An Optimized Poly-antigenic Lentiviral Vector Induces Protective CD4+ T-Cell Immunity and Predicts a Booster Vaccine against Mycobacterium tuberculosis.
40. Anderson, KG, Mayer-Barber, K, Sung, H, Beura, L, James, BR, Taylor, JJ, et al. (2014). Intravascular staining for discrimination of vascular and tissue leukocytes. Nat Protoc 9: 209-222.
41. Groschel, MI, Sayes, F, Shin, SJ, Frigui, W, Pawlik, A, Orgeur, M, et al. (2017). Recombinant BCG Expressing ESX-1 of Mycobacterium marinum Combines Low Virulence with Cytosolic Immune Signaling and Improved TB Protection. Cell Rep 18: 2752-2765.
42. Majlessi, L, Brodin, P, Brosch, R, Rojas, MJ, Khun, H, Huerre, M, et al. (2005). Influence of ESAT-6 secretion system 1 (RD1) of Mycobacterium tuberculosis on the interaction between mycobacteria and the host immune system. J Immunol 174: 3570-3579.
43. Pym, AS, Brodin, P, Majlessi, L, Brosch, R, Demangel, C, Williams, A, et al. (2003). Recombinant BCG exporting ESAT-6 confers enhanced protection against tuberculosis. Nat Med 9: 533-539.
44. Macri, C, Dumont, C, Johnston, AP, and Mintern, JD (2016). Targeting dendritic cells: a promising strategy to improve vaccine effectiveness. Clin Transl Immunology 5: e66.
45. Stanek, O, Linhartova, I, Majlessi, L, Leclerc, C, and Sebo, P (2012). Complexes of streptavidin-fused antigens with biotinylated antibodies targeting receptors on dendritic cell surface: a novel tool for induction of specific T-cell immune responses Mol Biotechnol Jul;51(3): 221-232.
46. Jones, GW, Hill, DG, and Jones, SA (2016). Understanding Immune Cells in Tertiary Lymphoid Organ Development: It Is All Starting to Come Together. Front Immunol 7: 401.
47. Gupta, S, Termini, JM, Raffa, FN, Williams, CA, Kornbluth, RS, and Stone, GW (2014). Vaccination with a fusion protein that introduces HIV-1 gag antigen into a multitrimer CD40L construct results in enhanced CD8+ T cell responses and protection from viral challenge by vaccinia-gag. J Virol 88: 1492-1501.
48. Gupta, S, Termini, JM, Rivas, Y, Otero, M, Raffa, FN, Bhat, V, et al. (2015). A multi-trimeric fusion of CD40L and gp100 tumor antigen activates dendritic cells and enhances survival in a B16-F10 melanoma DNA vaccine model. Vaccine 33: 4798-4806.
49. Zennou, V, Petit, C, Guetard, D, Nerhbass, U, Montagnier, L, and Charneau, P (2000). HIV-1 genome nuclear import is mediated by a central DNA flap. Cell 101: 173-185.
50. Gussow, D, Rein, R, Ginjaar, I, Hochstenbach, F, Seemann, G, Kottman, A, et al. (1987). The human beta 2-microglobulin gene. Primary structure and definition of the transcriptional unit. J Immunol 139: 3132-3138.
51. Iglesias, MC, Frenkiel, MP, Mollier, K, Souque, P, Despres, P, and Charneau, P (2006). A single immunization with a minute dose of a lentiviral vector-based vaccine is highly effective at eliciting protective humoral immunity against West Nile virus. J Gene Med 8: 265-274.
52. Sayes, F, Pawlik, A, Frigui, W, Groschel, MI, Crommelynck, S, Fayolle, C, et al. (2016). CD4+ T Cells Recognizing PE/PPE Antigens Directly or via Cross Reactivity Are Protective against Pulmonary Mycobacterium tuberculosis Infection. PLoS Pathog 12: e1005770.
53. Sayes, F, Blanc, C, Ates, LS, Deboosere, N, Orgeur, M, Le Chevalier, F, et al. (2018). Multiplexed Quantitation of Intraphagocyte Mycobacterium tuberculosis Secreted Protein Effectors. Cell Rep 23: 1072-1084.

## Claims

1. A recombinant lentiviral vector genome comprising a polynucleotide encoding a fusion polypeptide, wherein said fusion polypeptide comprises arranged from N-terminal to C-terminal ends a first recombinant polypeptide and a second polypeptide, wherein:
(i) said first recombinant polypeptide comprises a multimerization scaffold which comprises at least one collectin or a fragment thereof suitable to enable self-assembly of multimers of the first polypeptide, fused with at least one antigenic polypeptide ;
(ii) said second polypeptide comprises a CD40L ectodomain or a receptor binding fragment thereof, in particular the CD40L ectodomain of the human CD40L, preferably the CD40L ectodomain of SEQ ID No. 19.

2. The recombinant lentiviral vector genome according to claim 1, wherein said collectin or fragment thereof comprises arranged from N-terminal to C-terminal ends:
- at least one crosslinking region of a collectin;
- at least one collagen-like region of a collectin; and
- at least one neck region of a collectin.

3. The recombinant lentiviral vector genome according to claim 2, wherein the polynucleotide encoding the said at least one antigenic polypeptide is fused in-frame within the nucleotide sequence of the collagen-like region of said collectin.

4. The recombinant lentiviral vector genome according to any one of claims 1 to 3, wherein said collectin or fragment thereof is a carbohydrate recognition domain (CRD)-truncated form of collectin, wherein said at least one antigenic polypeptide is inserted within the collagen-like region of the collectin.

5. The recombinant lentiviral vector genome according to any one of claims 1 to 4, wherein said fusion polypeptide further comprises a polypeptide comprising a chemokine, in particular selected from CCL20, a pro-inflammatory Th1-related chemokine such as CXCL9, CXCL10, CCL3, CCL4 and/or CCL5, and/or a Th17-promoting chemokine such as CXCL20, or a receptor binding domain thereof, preferably wherein said chemokine is the CCL20 domain of SEQ ID No. 20, in particular wherein said chemokine or fragment thereof is inserted within the collagen-like domain of said collectin.

6. The recombinant lentiviral vector genome according to any one of claims 1 to 5, wherein said collectin is selected from mannan-binding lectin (MBL), surfactant protein D (SP-D), surfactant protein A (SP-A), collectin liver 1 (CL-L1), collectin placenta 1 (CL-P1), conglutinin collectin of 43 kDa (CL-43), collectin of 46 kDa (CL-46),and collectin kidney 1 (CL-K1), preferably wherein said collectin is a human collectin selected from MBL (UniProtKB - P11226), SP-D (UniProtKB - P35247), CL-L1 (UniProtKB - Q9Y6Z7), SP-A1 (UniProtKB - Q8IWL2), SP-A2 (UniProtKB - Q8IWL1), CL-P1 (UniProtKB - Q5KU26) and CL-K1 (UniProtKB - Q9BWP8).

7. The recombinant lentiviral vector genome according to any one of claims 1 to 6, wherein said antigenic polypeptide is a mono-antigenic polypeptide comprising one antigen or immunogenic fragment thereof, or is a poly-antigenic polypeptide comprising at least two antigens or immunogenic fragments thereof.

8. The recombinant lentiviral vector genome according to any one of claims 1 to 7, wherein the at least one antigen or immunogenic fragment thereof is selected from a bacterial, parasite or viral pathogen, in particular from *Mycobacterium tuberculosis,* an influenza virus or a coronavirus such as SARS-CoV-2 or is a tumoral antigen or immunogenic fragment thereof.

9. The recombinant lentiviral vector genome according to any one of claims 1 to 8, wherein said antigenic polypeptide comprises one or more *Mycobacterium tuberculosis* (Mtb) antigens selected from EsxA, EspC, EsxH, PE19, Hypoxic response protein 1 (Hrp1) and Resuscitation promoting factor D (RpfD), or an immunogenic fragment thereof, in particular one of the following Mtb antigenic combinations:
(a) EsxH;
(b) EsxH and EsxA;
(c) EsxH, EsxA and PE19;
(d) EsxH, EsxA, PE19 and EspC;
(e) EsxA, PE19, EspC, HRp1 and RpfD;
or an immunogenic fragment thereof.

10. The recombinant lentiviral vector genome, wherein said genome is obtained from the pTRIP vector plasmid of nucleotide sequence SEQ ID No. 21, wherein the polynucleotide encoding the fusion polypeptide has been cloned under control of a promoter functional in mammalian cells, in particular the CMV promoter, the human beta-2 microglobulin promoter, the composite BCUAG promoter of SEQ ID No. 22 and wherein the vector optionally comprises post-transcriptional regulatory element of the woodchuck hepatitis virus (WPRE).

11. A DNA plasmid comprising the recombinant vector genome according to any one of claims 1 to 10, in particular wherein said genome is inserted within the pTRIP vector plasmid of nucleotide sequence SEQ ID No.21.

12. A recombinant lentiviral vector particle which comprises the recombinant lentiviral vector genome according to any one of claims 1 to 10.

13. The recombinant lentiviral vector particle according to claim 12 which is a recombinant integration-deficient lentiviral vector particle, in particular the recombinant integration-deficient lentiviral vector is a HIV-1 based vector and is integrase deficient as a result of a mutation of the integrase gene encoded in the genome of the lentivirus in such a way that the integrase is not expressed or not functionally expressed, in particular the mutation in the integrase gene leads to the expression of an integrase substituted on its amino acid residue 64, in particular the substitution is D64V in the catalytic domain of the HIV-1 integrase encoded by Pol.

14. The recombinant lentiviral vector particle according to any one of claim 12 or 13, wherein said recombinant lentiviral vector genome is the genome of a replication-incompetent pseudotyped lentiviral vector, in particular a replication-incompetent pseudotyped HIV-1 lentiviral vector, in particular wherein the vector is pseudotyped with the glycoprotein G from a Vesicular Stomatitis Virus (V-SVG) of Indiana or of New-Jersey serotype.

15. A host cell, preferably a mammalian host cell, transfected with a DNA plasmid according to claim 11, in particular wherein said host cell is a HEK-293T cell line or a K562 cell line.

16. A pharmaceutical composition, in particular a vaccine composition, suitable for administration to a mammalian host, comprising a recombinant lentiviral vector particle of any one of claims 12 to 14 together with one or more pharmaceutically acceptable excipient(s) suitable for administration to a host in need thereof, in particular a human host.

17. The pharmaceutical composition of claim 16, for use in the elicitation of a protective, preferentially prophylactic, immune response by the elicitation of antibodies directed against the antigenic polypeptide or immunogenic fragments thereof, and/or cellular and/or humoral response in a host in need thereof, in particular a human host.

18. The pharmaceutical composition of claim 16 or 17, wherein the immune response involves the induction of MHC-II restricted presentation of the antigenic polypeptide or immunogenic fragments thereof, by an antigen-presenting cell, in particular a dendritic cell, and the induction of a CD4-mediated cellular immune response.

19. The pharmaceutical composition of any one of claims 16 to 18, for preventing and/or treating an infection by a pathogen in a mammalian host in need thereof, in particular a human host.

20. A method for the preparation of recombinant lentiviral vector particles suitable for the preparation of a pharmaceutical composition, in particular a vaccine, comprising the following steps:
a) transfecting the recombinant lentiviral transfer vector carrying the lentiviral vector genome according to any one of claims 1 to 10, or the DNA plasmid according to claim 11 in a host cell, for example a HEK-293T cell line or a K562 cell line;
b) co-transfecting the cell of step a) with: (i) a plasmid vector encoding envelope proteins and with a plasmid vector encoding the lentiviral GAG and POL or mutated POL protein as packaging construct; and (ii) a plasmid encoding VSV-G Indiana or New Jersey envelope,
c) culturing the host cell under conditions suitable for the production of recombinant lentiviral vector particles expressing the antigenic polypeptide, or an immunogenic fragment thereof;
d) recovering the recombinant lentiviral particles expressing the antigenic polypeptide, or an immunogenic fragment thereof.
